# EUROPEAN PATENT APPLICATION

(11) **EP 1 488 802 A2**
(43) Date of publication of application: **22.12.2004**
(21) Application number: 04020554.4
(22) Date of filing: 19.06.1997
(51) Int. Cl.: A61K 38/46, C07H 19/00, C07H 21/02, C07H 21/04, C12N 9/14, C12N 1/20, C12N 15/00

(54) **Thermostabile phosphatases**

(30) Priority: 19.06.1996 US 33752 P
(62) Divisional of application: 97933154.3
(71) Applicant: DIVERSA CORPORATION, San Diego, California 92121 (US)
(72) Inventor: Mathur, Eric J., Carlsbad, CA 92009 (US); Lee, Edward, Arcadia, CA 91007 (US); Bylina, Edward, San Diego, CA 92130 (US)
(74) Representative: VOSSIUS & PARTNER

(57) **Abstract**

Thermostable alkaline phosphatase enzymes derived from bacteria from the genus Ammonifex, Aquifex, Archaeoglobus, Desulfurococcus, Methanococcus, Thermotogales, Pyrolobus, Pyrococcus, and Thermococcus organisms are disclosed. The enzymes are produced from native or recombinant host cells and can be utilized in the pharmaceutical, food, detergent, and baking industry.

## Description

This invention relates to newly identified polynucleotides, polypeptides encoded by such polynucleotides, the use of such polynucleotides and polypeptides, as well as the production and isolation of such polynucleotides and polypeptides. More particularly, the polynucleotides and polypeptides of the present invention have been identified as thermostable alkaline phosphatases.

### BACKGROUND OF THE INVENTION

Phosphatases are a group of enzymes that remove phosphate groups from organophosphate ester compounds. There are numerous phosphatases, including alkaline phosphatases, phosphodiesterases and phytases.

Alkaline phosphatases are widely distributed enzymes and are composed of a group of enzymes which hydrolyze organic phosphate ester bonds at alkaline pH.

Phosphodiesterases are capable of hydrolyzing nucleic acids by hydrolyzing the phosphodiester bridges of DNA and RNA. The classification of phosphodiesterases depends upon which side of the phosphodiester bridge is attacked. The 3' enzymes specifically hydrolyze the ester linkage between the 3' carbon and the phosphoric group whereas the 5' enzymes hydrolyze the ester linkage between the phosphoric group and the 5' carbon of the phosphodiester bridge. The best known of the class 3' enzymes is a phosphodiesterase from the venom of the rattlesnake or from a rustle's viper, which hydrolyses all the 3' bonds in either RNA or DNA liberating nearly all the nucleotide units as nucleotide 5' phosphates. This enzyme requires a free 3' hydroxyl group on the terminal nucleotide residue and proceeds stepwise from that end of the polynucleotide chain. This enzyme and all other nucleases which attack only at the ends of the polynucleotide chains are called exonucleases. The 5' enzymes are represented by a phosphodiesterase from bovine spleen, also an exonuclease, which hydrolyses all the 5' linkages of both DNA and RNA and thus liberates only nucleoside 3' phosphates. It begins its attack at the end of the chain having a free 3' hydroxyl group.

Phytases are enzymes which recently have been introduced to commerce. The phytase enzyme removes phosphate from phytic acid (inositol hexaphosphoric acid), a compound found in plants such as corn, wheat and rice. The enzyme has commercial use for the treatment of animal feed, making the inositol of the phytic acid available for animal nutrition. *Aspergillus ficuum* and wheat are sources of phytase. (Business Communications Co., Inc., 25 Van Zant Street, Norwalk, CT 06855).

Phytase is used to improve the utilization of natural phosphorus in animal feed. Use of phytase as a feed additive enables the animal to metabolize a larger degree of its cereal feed's natural mineral content thereby reducing or altogether eliminating the need for synthetic phosphorus additives. More important than the reduced need for phosphorus additives is the corresponding reduction of phosphorus in pig and chicken waste. Many European countries severely limit the amount of manure that can be spread per acre due to concerns regarding phosphorus contamination of ground water. This is highly important in northern Europe, and will eventually be regulated throughout the remainder of the European Continent and the United States as well. (Excerpts from Business Trend Analysts, Inc., January 1994, Frost and Sullivan Report 1995 and USDA on-line information.)

Alkaline phosphatase hydrolyzes monophosphate esters, releasing an organic phosphate and the cognate alcohol compound. It is non-specific with respect to the alcohol moiety and it is this feature which accounts for the many uses of this enzyme. The enzyme has a pH optimum between 9 and 10, however, it can also function at neutral pH, (study of the enzyme industry conducted by Business Communications Company, Inc., 25 Van Zant Street, Norwalk, Connecticut 06855, 1995.).

Thermostable alkaline phosphatases are not irreversibly inactivated even when heated to 60°C or more for brief periods of time, as, for example, in the practice of hydrolyzing monophosphate esters.

Alkaline phosphatases may be obtained from numerous thermophilic organisms, such as *Ammonifex degensii, Aquifex pyrophilus, Archaeoglobus lithotrophicus, Methanococcus igneus, Pyrolobus*(a *Crenarchaeota*), *Pyrococcus* and Thermococcus, which are mostly Eubacteria and Euryarchaeota. Many of these organisms grow at temperatures up to about 103°C and are unable to grow below 70°C. These anaerobes are isolated from extreme environments. For example, *Thermococcus* CL-2 was isolated from a worm residing on a "black smoker" sulfite structure.

Interest in alkaline phosphatases from thermophilic microbes has increased recently due to their value for commercial applications. Two sources of alkaline phosphatases dominate and compete commercially: (i) animal, from bovine and calf intestinal mucosa, and (ii) bacterial, from *E. coli.* Due to the high turnover number of calf intestinal phosphatase, it is often selected as the label in many enzyme immunoassays. The usefulness of calf alkaline phosphatase, however, is limited by its inherently low thermostability, which is even further compromised during the chemical preparation of the enzyme: antibody conjugates. Bacterial alkaline phosphatase is an alternative to calf alkaline phosphatase due to bacterial alkaline phosphatase's extreme thermotolerance at temperatures as high as 95°C (Tomazic-Allen, S.J., Recombinant Bacterial Phosphatase as an Immunodiagnostic Enzyme, Annals D Biology Clinique, 49(5):287-90 (1991), however, the enzyme has a very low turnover number.

There is a need for novel phosphatase enzymes having enhanced thermostability. This includes a need for thermostable alkaline phosphatases whose enhanced thermostability is beneficial in enzyme labeling processes and certain recombinant DNA techniques, such as in the dephosphorylation of vector DNA prior to insert DNA ligation. Recombinant phosphatase enzymes provide the proteins in a format amenable to efficient production of pure enzyme, which can be utilized in a variety of applications as described herein. Accordingly, there is a need for the characterization, amino acid sequencing, DNA sequencing, and heterologous expression of thermostable phosphatase enzymes. The present invention meets these need by providing DNA and amino acid sequence information and exprssion and purification protocol for thermostable phosphatase derived from several organisms.

### SUMMARY OF THE INVENTION

The present invention provides thermostable phosphatases from several organisms. In accordance with one aspect of the present invention, there are provided novel enzymes, as well as active fragments, analogs and derivatives thereof.

In accordance with another aspect of the present invention, there are provided isolated nucleic acid molecules encoding the enzymes of the present invention, including mRNAs, cDNAs, genomic DNAs, as well as active analogs and fragments of such nucleic acids.

In accordance with another aspect of the present invention, there are provided isolated nucleic acid molecules encoding mature enzymes expressed by the DNA contained in the plasmid DNA vector deposited with the ATCC as Deposit No. 97536 on May 10, 1996.

In accordance with a further aspect of the present invention, there is provided a process for producing such polypeptides by recombinant techniques comprising culturing recombinant prokaryotic and/or eukaryotic host cells, containing a nucleic acid sequence of the present invention, under conditions promoting expression of said enzymes and subsequent recovery of said enzymes.

In accordance with yet a further aspect of the present invention, there is provided a process for utilizing such enzymes for hydrolyzing monophosphate ester bonds, as an enzyme label in immunoassays, for removing 5' phosphate prior to end-labeling, and for dephosphorylating vectors prior to insert ligation.

In accordance with yet a further aspect of the present invention, there are also provided nucleic acid probes comprising nucleic acid molecules of sufficient length to specifically hybridize to a nucleic acid sequence of the present invention.

In accordance with yet a further aspect of the present invention, there is provided a process for utilizing such enzymes, or polynucleotides encoding such enzymes, for in vitro purposes related to scientific research, for example, to generate probes for identifying similar sequences which might encode similar enzymes from other organisms by using certain regions, *i.e.*, conserved sequence regions of the nucleotide sequence.

These and other aspects of the present invention will be apparent to those of skill in the art from the teachings herein.

### BRIEF DESCRIPTION OF THE DRAWINGS

The following drawings are illustrative of embodiments of the invention and are not meant to limit the scope of the invention as encompassed by the claims.

Figure 1 is an illustration of the full-length-DNA and corresponding deduced amino acid sequence of *Ammonifex degensii* KC4 of the present invention. Sequencing was performed using a 378 automated DNA sequence for all sequences of the present.invention (Applied Biosystems, Inc., Foster City, California).

Figure 2 is an illustration of the full-length DNA and corresponding deduced amino acid sequence of *Methanococcus igneus* Kol5.

Figure 3 is an illustration of the full-length DNA and corresponding deduced amino acid sequence of *Thermococcus alcaliphilus* AEDII12RA.

Figure 4 is an illustration of the full-length DNA and corresponding deduced amino acid sequence of *Thermococcus celer.*

Figure 5 is an illustration of the full-length DNA and corresponding deduced amino acid sequence of *Thermococcus* GU5L5.

Figure 6 is an illustration of the full-length DNA and corresponding deduced amino acid sequence of OC9a.

Figure 7 is an illustration of the full-length DNA and corresponding deduced amino acid sequence of M11TL.

Figure 8 is an illustration of the full-length DNA and corresponding deduced amino acid sequence of *Thermococcus* CL-2.

Figure 9 is an illustration of the full-length DNA and corresponding deduced amino acid sequence of *Aquifex* VF-5.

### DETAILED DESCRIPTION OF THE INVENTION

To facilitate understanding of the invention, a number of terms are defined below.

The term "isolated" means altered "by the hand of man" from its natural state; i.e., if it occurs in nature, it has been changed or removed from its original environment, or both. For example, a naturally occurring polynucleotide or a polypeptide naturally present in a living animal in its natural state is not "isolated", but the same polynucleotide or polypeptide separated from the coexisting materials of its natural state is "isolated", as the term is employed herein. For example, with respect to polynucleotides, the term isolated means that it is separated from the nucleic acid and cell in which it naturally occurs.

As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such polynucleotides still would be isolated, as the term is used herein, because they would not be in their naturally occurring form or environment. Similarly, the polynucleotides and polypeptides may occur in a composition, such as a media formulation (solutions for introduction of polynucleotides or polypeptides, for example, into cells or compositions or solutions for chemical or enzymatic reactions which are not naturally occurring compositions) and, therein- remain isolated polynucleotides or polypeptides within the meaning of that term as it is employed herein.

The term "ligation" refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook et al., MOLECULAR CLONING, A LABORATORY MANUAL, 2nd Ed.; Cold Spring Harbor Laboratory Press, Cold Spring Harbor, New York (1989).

The term "oligonucleotide" as used herein is defined as a molecule comprised of two or more deoxyribonucleotides or ribonucleotides, preferably more than three, and usually more than ten. The exact size of an oligonucleotide will depend on many factors, including the ultimate function or use of the oligonucleotide. Oligonucleotides can be prepared by any suitable method, including, for example, cloning and restriction of appropriate sequences and direct chemical synthesis by a method such as the phosphotriester method of Narang et al., 1979, Meth. Enzymol., 68:90-99; the phosphodiester method of Brown *et al.*, 1979, Method Enzymol., 68:109-151, the diethylphosphoramidite method of Beaucage *et al.*, 1981, Tetrahedron Lett., 22:1859-1862; the triester method of Matteucci *et al.*, 1981, J. Am. Chem. Soc., 103:3185-3191, or automated synthesis methods; and the solid support method of U.S. Patent No. 4,458,066.

The term "plasmids" generally is designated herein by a lower case p preceded and/or followed by capital letters and/or numbers, in accordance with standard naming conventions that are familiar to those of skill in the art.
Plasmids disclosed herein are either commercially available, publicly available on an unrestricted basis, or can be constructed from available plasmids by routine application of well known, published procedures. Many plasmids and other cloning and expression vectors that can be used in accordance with the present invention are well known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

The term "polynucleotide(s)" generally refers to any polyribonucleotide or polydeoxyribonucleotide, which may be unmodified RNA or DNA or modified RNA or DNA. Thus, for instance, polynucleotides as used herein refers to, among others, single-and double-stranded DNA, DNA that is a mixture of single-and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.
In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide.

As used herein, the term polynucleotide includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein.

It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including simple and complex cells, *inter alia*.

The term "primer" as used herein refers to an oligonucleotide, whether natural or synthetic, which is capable of acting as a point of initiation of synthesis when placed under conditions in which primer extension is initiated or possible. Synthesis of a primer extension product which is complementary to a nucleic acid strand is initiated in the presence of nucleoside triphosphates and a polymerase in an appropriate buffer at a suitable temperature.

The term "primer" may refer to more than one primer, particularly in the case where there is some ambiguity in the information regarding one or both ends of the target region to be synthesized. For instance, if a nucleic acid sequence is inferred from a protein sequence, a "primer" generated to synthesize nucleic acid encoding said protein sequence is actually a collection of primer oligonucleotides containing sequences representing all possible codon variations based on the degeneracy of the genetic code. One or more of the primers in this collection will be homologous with the end of the target sequence. Likewise, if a "conserved" region shows significant levels of polymorphism in a population, mixtures of primers can be prepared that will amplify adjacent sequences.

The term "restriction endonucleases" and "restriction enzymes" refers to bacterial enzymes which cut double-stranded DNA at or near a specific nucleotide sequence.

The term "gene" means the segment of DNA involved in producing a polypeptide chain;
it includes regions preceding and following the coding region (leader and trailer) as well as intervening sequences (introns) between individual coding segments (exons).

A coding sequence is "operably linked" to another coding sequence when RNA polymerase will transcribe the two coding sequences into a single mRNA, which is then translated into a single polypeptide having amino acids derived from both coding sequences. The coding sequences need not be contiguous to one another so long as the expressed sequences ultimately process to produce the desired protein.

"Recombinant" enzymes refer to enzymes produced by recombinant DNA techniques; *i.e.*, produced from cells transformed by an exogenous DNA construct encoding the desired enzyme. "Synthetic" enzymes are those prepared by chemical synthesis.

A DNA "coding sequence of" or a "nucleotide sequence encoding" a particular enzyme, is a DNA sequence which is transcribed and translated into an enzyme when placed under the control of appropriate regulatory sequences.

The term "thermostable phosphatase" refers to an enzyme which is stable to heat and heat-resistant and catalyzes the removal of phosphate groups from organophosphate ester compounds. Reference to "thermostable phosphatases" includes alkaline phosphatases, phosphodiesterases and phytases.

The phosphatase enzymes of the present invention cannot become irreversibly denatured (inactivated) when subjected to the elevated temperatures for the time necessary to effect the hydrolysis of a phosphate group from an organophosphate ester compound. Irreversible denaturation for purposes herein refers to permanent and complete loss of enzymatic activity. The phosphatase enzymes do not become irreversibly denatured from exposure to temperatures of a range from about 60°C to about 113°C or more. The extreme thermostability of the phosphatase enzymes provides additional advantages over previously characterized thermostable enzymes. Prior to the present invention, efficient hydrolysis of phosphate groups at temperatures as high as 100°C has not been demonstrated. No thermostable phosphatase has been described for this purpose.

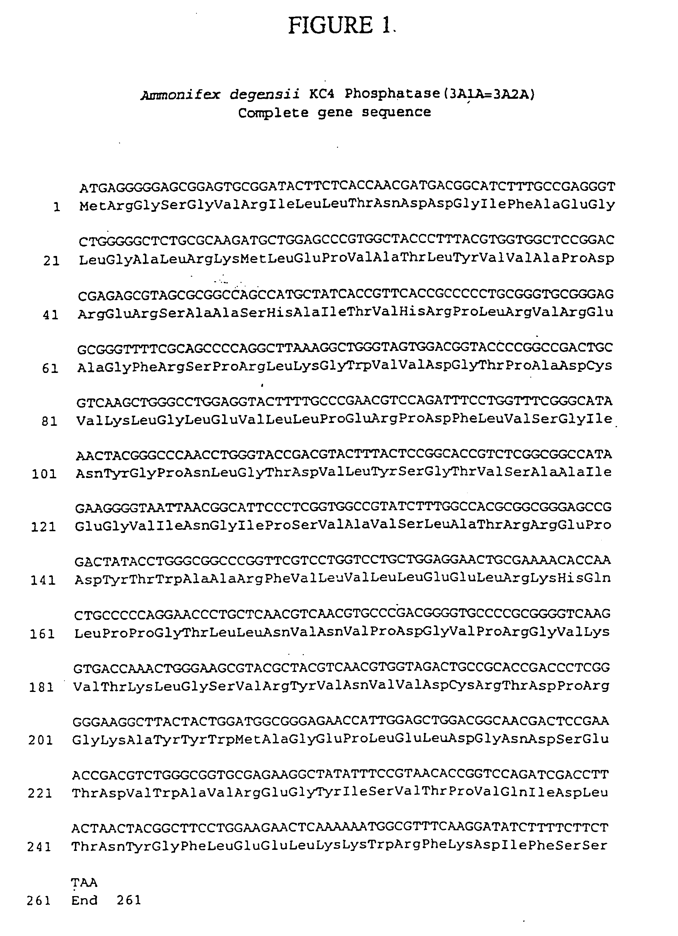

In accordance with an aspect of the present invention, there are provided isolated nucleic acids (polynucleotides) which encode for the mature enzymes having the deduced amino acid sequences of Figures 1-9 (SEQ ID NOS:28-36).

In accordance with another aspect of the present invention, there are provided isolated polynucleotides encoding the enzymes of the present invention. The deposited material is a mixture of genomic clones comprising DNA encoding an enzyme of the present invention. Each genomic clone comprising the respective DNA has been inserted into a pBluescript vector (Stratagene, La Jolla, CA). The deposit has been deposited with the American Type Culture Collection, 12301 Parklawn Drive, Rockville, Maryland 20852, USA, on May 10, 1996 and assigned ATCC Deposit No. 97536.

The deposit(s) have been made under the terms of the Budapest Treaty on the International Recognition of the deposit of micro-organisms for purposes of patent procedure. The strains will be irrevocably and without restriction or condition released to the public upon the issuance of a patent. These deposits are provided merely as convenience to those of skill in the art and are not an admission that a deposit be required under 35 U.S.C. §112. The sequences of the polynucleotides contained in the deposited materials, as well as the amino acid sequences of the polypeptides encoded thereby, are controlling in the event of any conflict with any description of sequences herein. A license may be required to make, use or sell the deposited materials, and no such license is hereby granted.

The polynucleotides of this invention were originally recovered from genomic gene libraries derived from the following organisms:
*Ammonifex degensii* KC4 is a eubacteria from the genus *Ammonifex*. It was isolated in Java, Indonesia. It is a gram-negative, chemolithoautotroph. It grows optimally at 70°C in a low-salt culture medium at pH 7 with 0.2% nitrate as a substrate and H₂/CO₂ in gas phase.

*Methanococcus igneus* KOL5 is a *Euryarchaeota* isolated from Kolbeinsey Ridge in the north of Iceland. It grows optimally at 85°C and pH 7.0 in a high-salt marine medium with H₂/CO₂ in a gas phase. *Aquifex pyrophilus* KOL 5A is a marine bacteria isolated from th Kolbeinsey Ridge in the north of Iceland. It is a gram-negative, rod-shaped, strictly chemolithoautotrophic, knall gas bacterium, and a denitrifier. It grows optimally at 85°C in high-salt marine medium at pH 6.8 with O₂ as a substrate and H₂/CO₂ + 0.5% O₂ in gas phase.

*Thermococcus alcaliphilus* AEDII12RA is from the genus *Thermococcus*. AEDII12RA grows optimally at 85°C, pH 9.5 in a high salt medium (marine) containing polysulfides and yeast extract as substrates and N₂ in gas phase.

*Thermococcus celer* is an *Euryarchaeota.* It grows optimally at 85°C and pH 6.0 in a high-salt marine medium containing elemental sulfur, yeast extract, and peptone as substrates and N₂ in gas phase.

*Thermococcus* GU5L5 is an *Euryarchaeota* isolated from the Guaymas Basin in Mexico. It grows optimally at 85°C and pH 6.0 in a high-salt marine medium containing 1% elemental sulfur, 0.4% yeast extract, and 0.5% peptone as substrates with N₂ in gas phase.

*OC9a*-27A3A is a bacteria of unknown etilogy obtained from Yellowstone National Park and maintained as a pure culture. It grows well on a TK6 medium and has cellulose degrader activity. Further, it codes for an alkaline phosphatase having greater than 50% polypeptide identity and greater than 32% polynucleotide identity to each of Bombyx *mori* and *Escherichia coli C* alkaline phosphatase precursors, which is significant homoloygy. Thus, it is expectged that *OC9a*-27A3A can be cloned and expressed readily in *Escherichi Coli C* in place of its native alkaline phosphatase precursor.

M11 TL is a new species of Desulfurococcus isolated from Diamond Pool in Yellowstone National Park. M11TL grows heterotrophically by fermentation of different organic materials (sulfur is not necessary) and forms grape-like aggregates. The organism grows optimally at 85°C to 88°C and pH 7.0 in a low salt medium containing yeast extract, peptone, and gelatin as substrates with an N₂/CO₂ gas phase.

*Thermococcus* CL-2 is an *Euryarchaeota* isolated from the North Cleft Segment in the Juan de Fuca Ridge. It grows optimally at 88°C in a salt medium with an argon atmosphere.

*Aquifex* VF-5 is a marine bacteria isolated from a beach in Vulcano, Italy. It is a gram-negative, rod-shaped, strictly chemolithoautotrophic, knall gas bacterium. It grows optimally from 85-90°C in high-salt marine medium at pH 6.8, with O₂ as a substrate and H₂/CO₂ + 0.5% O₂ in gas phase.

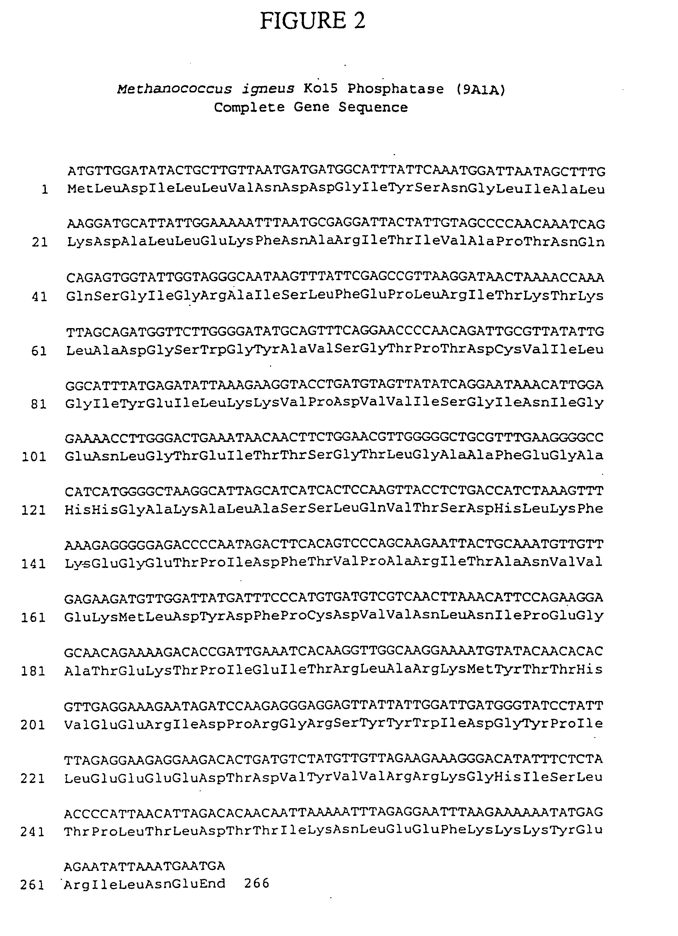

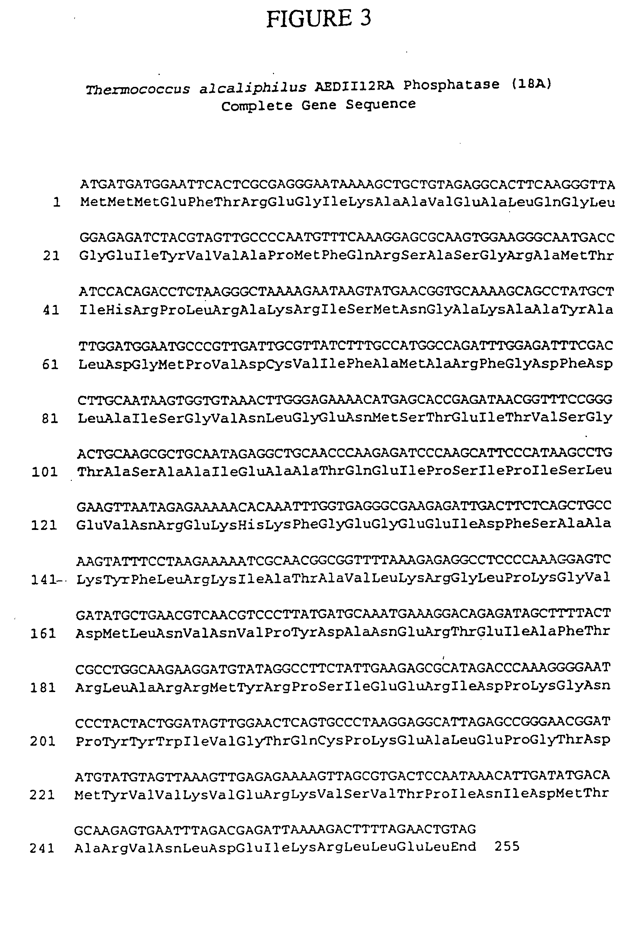

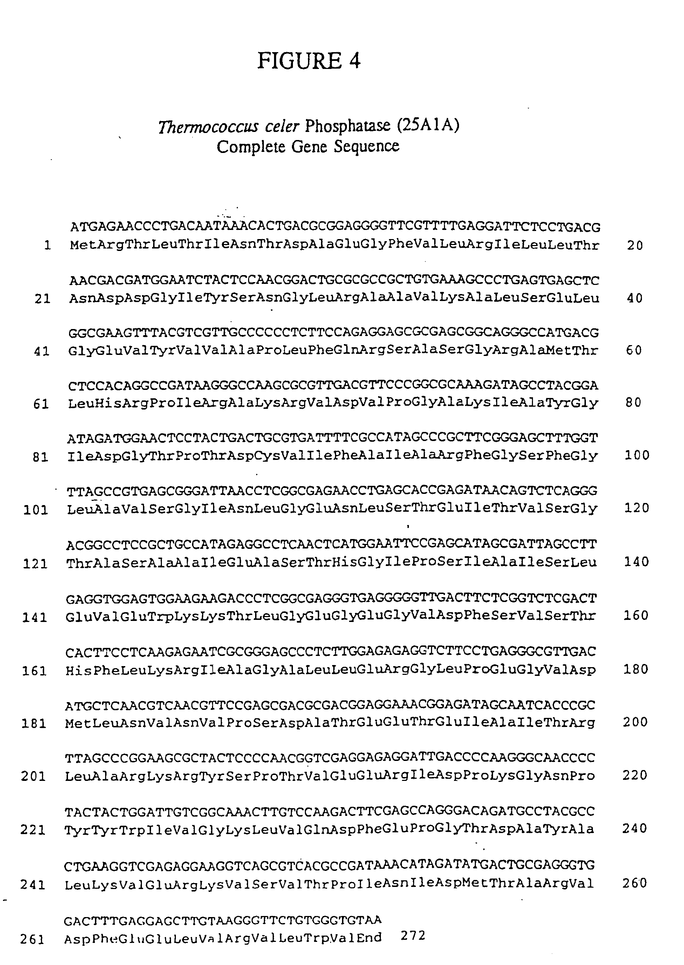

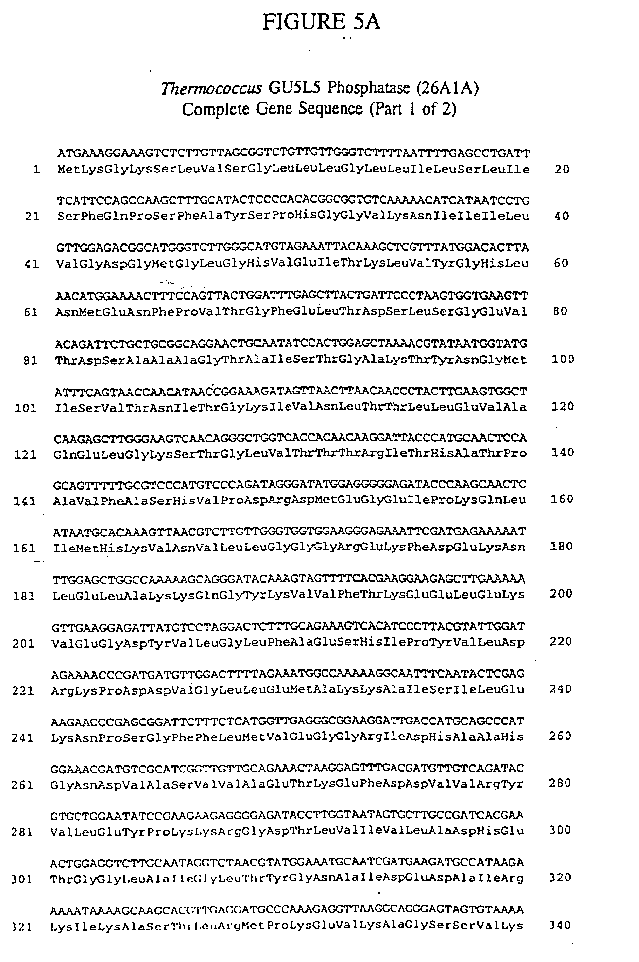

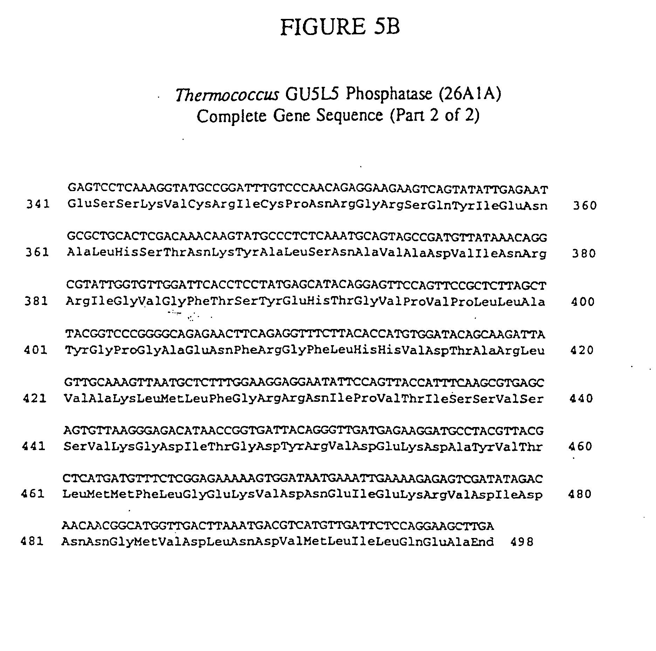

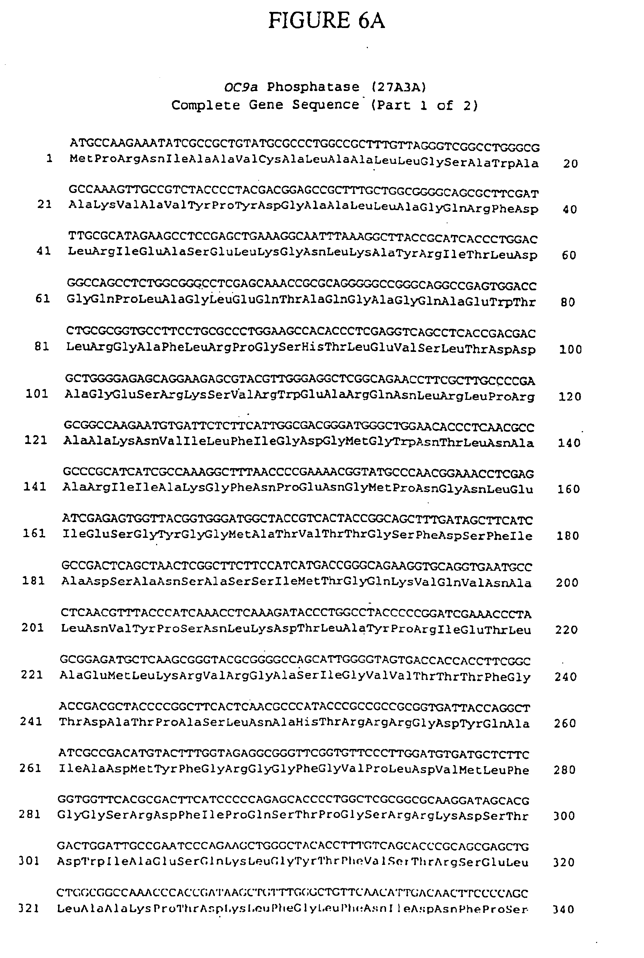

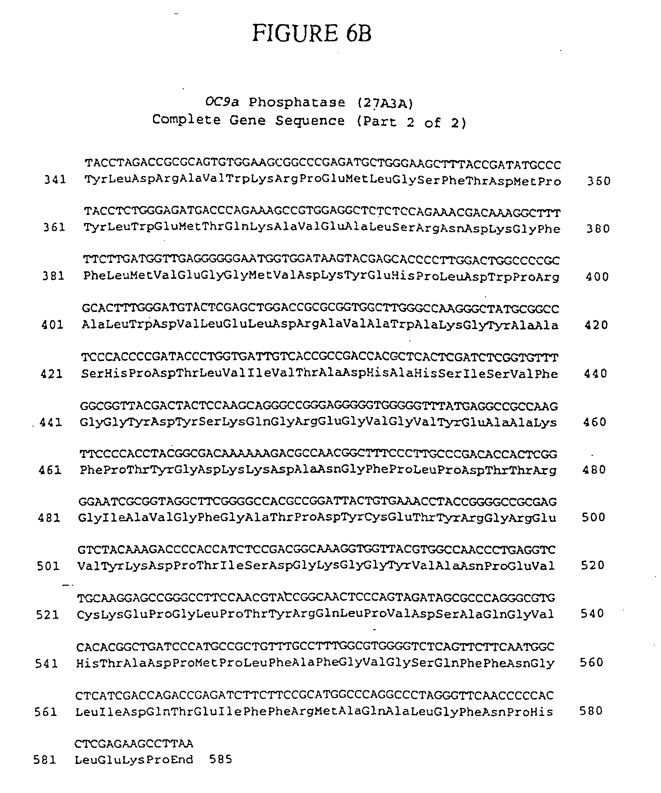

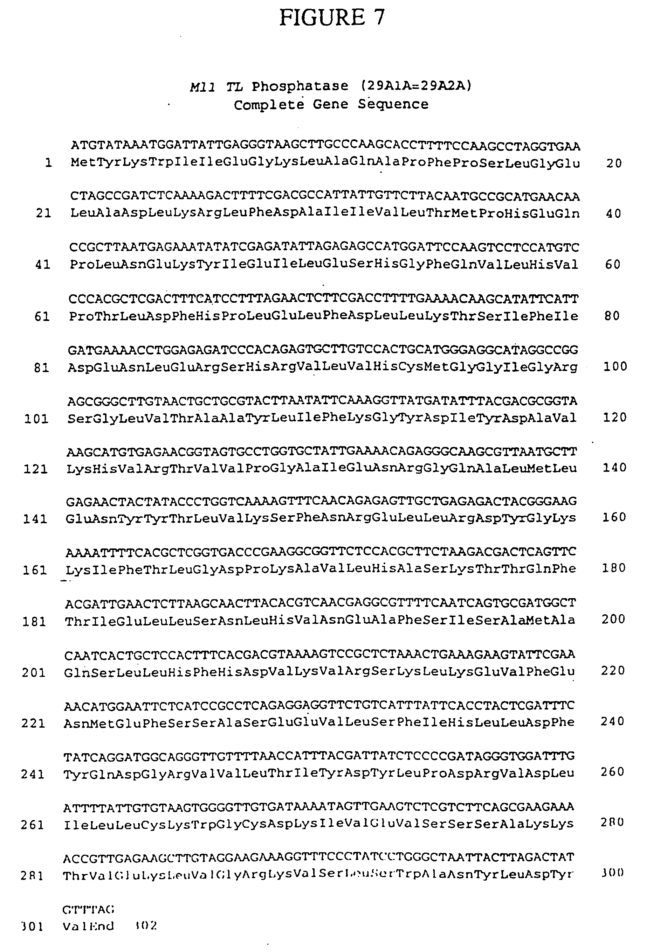

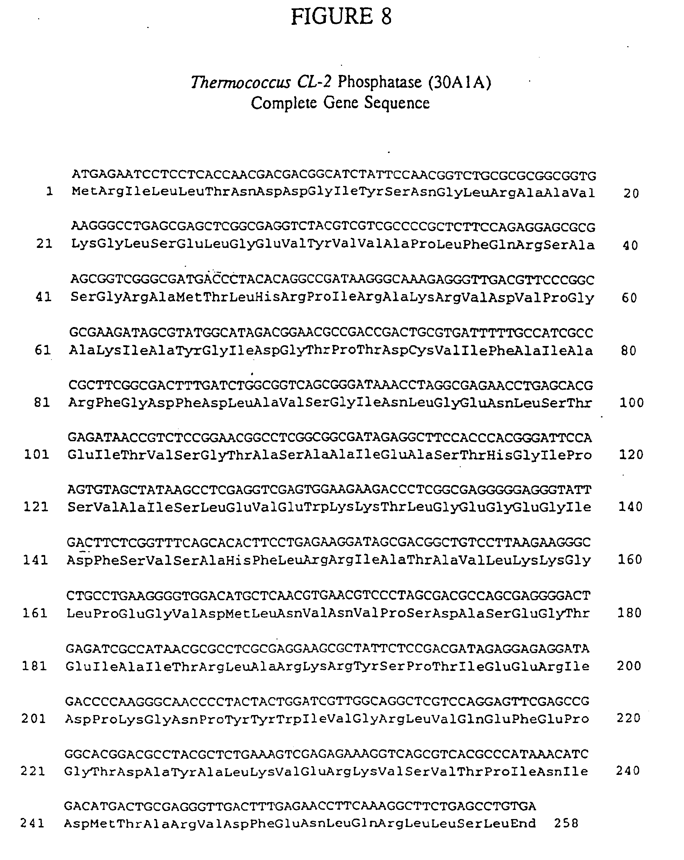

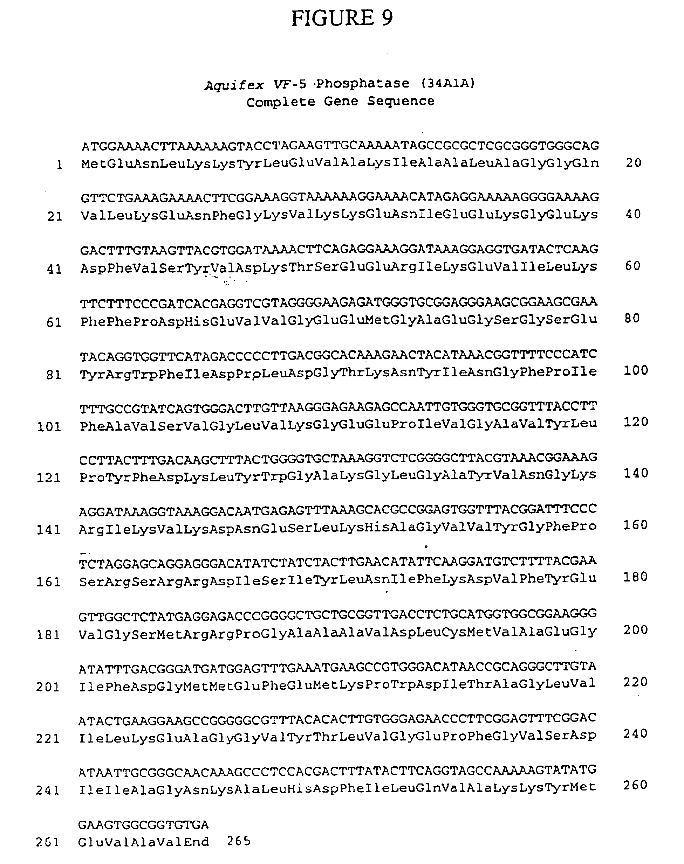

Accordingly, the polynucleotides and enzymes encoded thereby are identified by the organism from which they were isolated, and are sometimes hereinafter referred to as "KC4" (Figure 1 and SEQ ID NOS:19 and 28), "Ko15" (Figure 2 and SEQ ID NOS:20 and 29), "AEDII12RA" (Figure 3 and SEQ ID NOS:21 and 30), "Celer" (Figure 4 and SEQ ID NOS:22 and 31), "GU5L5" (Figure 5 and SEQ ID NOS:23 and 32), "OC9a" (Figure 6 and SEQ ID NOS:24 and 33), "M11TL" (Figure 7 and SEQ ID NOS:25 and 34), "CL-2" (Figure 8 and SEQ ID NOS:26 and 35) and "VF-5" (Figure 9 and SEQ ID NOS:27 and 36).

The polynucleotides and polypeptides of the present invention show identity of the nucleotide and protein level to known genes and proteins encoded thereby as shown in Table 1.

**Table I**

| Clone | Gene/Protein with Closest Homology | Protein Identity | Nucleic Acid Identity |
|---|---|---|---|
| *Ammonifex degensiii* KC4-3A1A | *Yarrowia lipolytica*, *Candida lipolytica,* acid phosphatase | 47% | 24% |
| *Ammonifex degensii* KC4-3A1A | *Saccharomyces cerevisiae*, hypothetical protein YBR094w | 54% | 26% |
| *Methanococcus igeneus* Kol5-9A1A | *Yarrowia lipolytica*, *Candida lipotytica*, acid phosphatase | 45% | 25% |
| *Methanococcus igeneus* Kol5-9A1A | *Saccharomyces cerevisiae*, hypothetical protein YBR094w, hypothetical protein YBR0821 | 52% | 25% |
| *Thermococcus alcaliphilus* AEDII12RA-18A | No homology found | -- | -- |
| *Thermococus celer* 25A1A | No homology found | -- | -- |
| *Thermncoccus* GU5L5-26A1A | *Bacillius subtilis*, alkaline phosphatase IV precursor, alkaline phosphomonoesterase, glycerophosphatase, and phosphomonoesterase | 58% | 38% |
| *Thermococcus* GU5L5-26A1A | *Bacillius subtilis*, alkaline phosphatase III precursor | 58% | 37% |
| OC9a-27A3A | *Bombyx mori* (silkworm), alkaline phosphatase precursor | 54% | 33% |
| OC9a - 27A3A | *Escherichia coli C,* alkaline phosphatase precursor | 53% | 34% |
| M11 TL - 29A1A | *Rhodobacter capsularus*, hypothetical protein B | 43% | 24% |
| *Thermococcus* Cl2-30A1A | *Yarrowia lipolytica*, *Candida lipolytica*, acid phosphatase | 49% | 27% |
| *Thermococcus* CL2-30A1A | *Saccharomyces cerevisiae*, hypothetical protein YBR094w hypothetical protein YBR0821 | 50% | 25% |
| *Aquifex* VF5-34A1A | *Escherichia coli*, suppressor protein suhB | 57% | 34% |

All of the clones identified in Table 1 encode polypeptides which have phosphatase activity.

One means for isolating the nucleic acid molecules encoding the enzymes of the present invention is to probe a gene library with a natural or artificially designed probe using art recognized procedures (see, for example: Current Protocols in Molecular Biology, Ausubel F.M. *et al*. (EDS.) Green Publishing Company Assoc. and John Wiley Interscience, New York, 1989, 1992). It is appreciated by one skilled in the art that the polynucleotides of SEQ ID NOS: 1-18, or fragments thereof (comprising at least 12 contiguous nucleotides), are particularly useful probes. Other particularly useful probes for this purpose are hybridizable fragments of the sequences of SEQ ID NOS: 19-27 (*i.e.*, comprising at least 12 contiguous nucleotides).

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at (Tm less 10°C) for the oligonucleotide probe. The membrane is then exposed to autoradiographic film for detection of hybridization signals.

Stringent conditions means hybridization will occur only if there is at least 90% identity, preferably at least 95% identity and most preferably at least 97% identity between the sequences. Further, it is understood that a section of a 100 bps sequence that is 95 bps in length has 95% identity with the 1090 bps sequence from which it is obtained. *See* J. Sambrook *et al., Molecular Cloning, A Laboratory Manual, 2d Ed*., Cold Spring Harbor Laboratory (1989) which is hereby incorporated by reference in its entirety. Also, it is understood that a fragment of a 100 bps sequence that is 95 bps in length has 95% identity with the 100 bps sequence from which it is obtained.

As used herein, a first DNA (RNA) sequence is at least 70% and preferably at least 80% identical to another DNA (RNA) sequence if there is at least 70% and preferably at least a 80% or 90% identity, respectively, between the bases of the first sequence and the bases of the another sequence, when properly aligned with each other, for example when aligned by BLASTN.

The present invention relates to polynucleotides which differ from the reference polynucleotide such that the differences are silent, for example, the amino acid sequence encoded by the polynucleotides is the same. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

The polynucleotides of this invention were recovered from genomic gene libraries from the organisms listed in Table 1. Gene libraries were generated from either of a Lambda ZAP II or a pBluscript] cloning vector (Stratagene Cloning Systems). Mass excisions were performed on these libraries to generate libraries in the pBluescript phagemid. Libraries were generated and excisions were performed according to the protocols/methods hereinafter described.

The polynucleotides of the present invention may be in the form of RNA or DNA which DNA includes cDNA, genomic DNA, and synthetic DNA. The DNA may be double-stranded or single-stranded, and if single stranded may be the coding strand or non-coding (anti-sense) strand. The coding sequences which encodes the mature enzymes may be identical to the coding sequences shown in Figures 1-9 (SEQ ID NOS: 19-27) or may be a different coding sequence which coding sequence, as a result of the redundancy or degeneracy of the genetic code, encodes the same mature enzymes as the DNA of Figures 1-9 (SEQ ID NOS: 19-27).

The polynucleotide which encodes for the mature enzyme of Figures 1-9 (SEQ ID NOS: 28-36) may include, but is not limited to: only the coding sequence for the mature enzyme; the coding sequence for the mature enzyme and additional coding sequence such as a leader sequence or a proprotein sequence; the coding sequence for the mature enzyme (and optionally additional coding sequence) and non-coding sequence, such as introns or non-coding sequence 5' and/or 3' of the coding sequence for the mature enzyme.

Thus, the term "polynucleotide encoding an enzyme (protein)" encompasses a polynucleotide which includes only coding sequence for the enzyme as well as a polynucleotide which includes additional coding and/or non-coding sequence.

The present invention further relates to variants of the hereinabove described polynucleotides which encode for fragments, analogs and derivatives of the enzymes having the deduced amino acid sequences of Figures 1-9 (SEQ ID NOS: 28-36). The variant of the polynucleotide may be a naturally occurring allelic variant of the polynucleotide or a non-naturally occurring variant of the polynucleotide.

Thus, the present invention includes polynucleotides encoding the same mature enzymes as shown in Figures 1-9 (SEQ ID NOS: 19-27) as well as variants of such polynucleotides which variants encode for a fragment, derivative or analog of the enzymes of Figures 1-9 (SEQ ID NOS: 19-27). Such nucleotide variants include deletion variants, substitution variants and addition or insertion variants.

As hereinabove indicated, the polynucleotides may have a coding sequence which is a naturally occurring-allelic variant of the coding sequences shown in Figures 1-9 (SEQ ID NOS: 19-27). As known in the art, an allelic variant is an alternate form of a polynucleotide sequence which may have a substitution, deletion or addition of one or more nucleotides, which does not substantially alter the function of the encoded enzyme. Also, using directed and other evolution strategies, one may make very minor changes in DNA sequence which can result in major changes in function.

Fragments of the full length gene of the present invention may be used as hybridization probes for a cDNA or a genomic library to isolate the full length DNA and to isolate other DNAs which have a high sequence similarity to the gene or similar biological activity. Probes of this type preferably have at least 10, preferably at least 15, and even more preferably at least 30 bases and may contain, for example, at least 50 or more bases. In fact, probes of this type having at least up to 150 bases or greater may be preferably utilized. The probe may also be used to identify a DNA clone corresponding to a full length transcript and a genomic clone or clones that contain the complete gene including regulatory and promotor regions, exons and introns. An example of a screen comprises isolating the coding region of the gene by using the known DNA sequence to synthesize an oligonucleotide probe. Labeled oligonucleotides having a sequence complementary or identical to that of the gene or portion of the gene sequences of the present invention are used to screen a library of genomic DNA to determine which members of the library the probe hybridizes to.

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

The present invention further relates to polynucleotides which hybridize to the hereinabove-described sequences if there is at least 70%, preferably at least 90%, and more preferably at least 95% identity between the sequences. (As indicated above, 70% identity would include within such definition a 70 bps fragment taken from a 100 bp polynucleotide, for example.) The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode enzymes which either retain substantially the same biological function or activity as the mature enzyme encoded by the DNA of Figures 1-9 (SEQ ID NOS: 19-27). In referring to identity in the case of hybridization, as known in the art, such identity refers to the complementarity of two polynucleotide segments.

Alternatively, the polynucleotide may have at least 15 bases, preferably at least 30 bases, and more preferably at least 50 bases which hybridize to any part of a polynucleotide of the present invention and which has an identity thereto, as hereinabove described, and which may or may not retain activity. For example, such polynucleotides may be employed as probes for the polynucleotides of SEQ ID NOS: 19-27, for example, for recovery of the polynucleotide or as a diagnostic probe or as a PCR primer.

Thus, the present invention is directed to polynucleotides having at least a 70% identity, preferably at least 90% identity and more preferably at least a 95% identity to a polynucleotide which encodes the enzymes of SEQ ID NOS: 28-36 as well as fragments thereof, which fragments have at least 15 bases, preferably at least 30 bases, more preferably at least 50 bases and most preferably fragments having up to at least 150 bases or greater, which fragments are at least 90% identical, preferably at least 95% identical and most preferably at least 97% identical to any portion of a polynucleotide of the present invention.

The present invention further relates to enzymes which have the deduced amino acid sequences of Figures 1-9 (SEQ ID NOS: 28-36) as well as fragments, analogs and derivatives of such enzyme.

The terms "fragment," "derivative" and "analog" when referring to the enzymes of Figures 1-9 (SEQ ID NOS. 28-36) means enzymes which retain essentially the same biological function or activity as such enzymes. Thus, an analog includes a proprotein which can be activated by cleavage of the proprotein portion to produce an active mature enzyme.

The enzymes of the present invention may be a recombinant enzyme, a natural enzyme or a synthetic enzyme, preferably a recombinant enzyme.

The fragment, derivative or analog of the enzymes of Figures 1-9 (SEQ ID NOS.28-36) may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature enzyme is fused with another compound, such as a compound to increase the half-life of the enzyme (for example, polyethylene glycol), or (iv) one in which the additional amino acids are fused to the mature enzyme, such as a leader or secretory sequence or a sequence which is employed for purification of the mature enzyme or a proprotein sequence. Such fragments, derivatives and analogs are deemed to be within the scope of those skilled in the art from the teachings herein.

The enzymes and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or enzyme present in a living animal is not isolated, but the same polynucleotide or enzyme, separated from some or all of the coexisting materials in the natural system, is isolated. Such polynucleotides could be part of a vector and/or such polynucleotides or enzymes could be part of a composition, and still be isolated in that such vector or composition is not part of its natural environment.

The enzymes of the present invention include the enzymes of SEQ ID NOS: 28-36 (in particular the mature enzyme) as well as enzymes which have at least 70% similarity (preferably at least 70% identity) to the enzymes of SEQ ID NOS: 28-36 and more preferably at least 90% similarity (more preferably at least 90% identity) to the enzymes of SEQ ID NOS: 28-36 and still more preferably at least 95% similarity (still more preferably at least 95% identity) to the enzymes of SEQ ID NOS: 28-36 and also include portions of such enzymes with such portion of the enzyme generally containing at least 30 amino acids and more preferably at least 50 amino acids and most preferably at least up to 150 amino acids.

As known in the art "similarity" between two enzymes is determined by comparing the amino acid sequence and its conserved amino acid substitutes of one enzyme to the sequence of a second enzyme. The definition of 70% similarity would include a 70 amino acid sequence fragment of a 100 amino acid sequence, for example, or a 70 amino acid sequence obtained by sequentially or randomly deleting 30 amino acids from the 100 amino acid sequence.

A variant, *i.e.* a "fragment", "analog" or "derivative" polypeptide, and reference polypeptide may differ in amino acid sequence by one or more substitutions, additions, deletions, fusions and truncations, which may be present in any combination.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Most highly preferred are variants which retain the same biological function and activity as the reference polypeptide from which it varies.

Fragments or portions of the enzymes of the present invention may be employed for producing the corresponding full-length enzyme by peptide synthesis; therefore, the fragments may be employed as intermediates for producing the full-length enzymes. Fragments or portions of the polynucleotides of the present invention may be used to synthesize full-length polynucleotides of the present invention.

The present invention also relates to vectors which include polynucleotides of the present invention, host cells which are genetically engineered with vectors of the invention and the production of enzymes of the invention by recombinant techniques.

Host cells are genetically engineered (transduced or transformed or transfected) with the vectors of this invention which may be, for example, a cloning vector such as an expression vector. The vector may be, for example, in the form of a plasmid, a phage, *etc*. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing enzymes by recombinant techniques. Thus, for example, the polynucleotide may be included in any one of a variety of expression vectors for expressing an enzyme. Such vectors include chromosomal, nonchromosomal and synthetic DNA sequences, *e.g.*, derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the *E*. *coli*. *lac* or *trp*, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E*. *coli.*

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as *E. coli*, *Streptomyces, Bacillus subtilis*; fungal cells, such as yeast; insect cells such as *Drosophila S2* and *Spodoptera Sf9*; animal cells such as CHO, COS or Bowes melanoma; adenoviruses; plant cells, *etc*. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQE70, pQE60, pQE-9 (Qiagen), pBluescript II KS, ptrc99a, pKK223-3, pDR540, pRIT2T (Pharmacia); Eukaryotic: pXT1, pSG5 (Stratagene) pSVK3, pBPV, pMSG, pSVL SV40 (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., *Basic Methods in Molecular Biology,* (1986)).

The constructs in host cells can be used in a conventional manner to produce the gene product encoded by the recombinant sequence. Alternatively, the enzymes of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook *et al., Molecular Cloning: A Laboratory Manual, Second Edition,* Cold Spring Harbor, N.Y., (1989), the disclosure of which is hereby incorporated by reference.

Transcription of the DNA encoding the enzymes of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, *e*.*g*., the ampicillin resistance gene of *E*. *coli* and *S*. *cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated enzyme. Optionally, the heterologous sequence can encode a fusion enzyme including an N-terminal identification peptide imparting desired characteristics,- *e.g.*, stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E*. *coli, Bacillus subtilis, Salmonella typhimurium* and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and pGEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (*e.g.*, temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Various mammalian cell culture systems can also be employed to express recombinant protein. Examples of mammalian expression systems include the COS-7 lines of monkey kidney fibroblasts, described by Gluzman, *Cell*, *23*:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The enzyme can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The enzymes of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the enzymes of the present invention may be glycosylated or may be non-glycosylated. Enzymes of the invention may or may not also include an initial methionine amino acid residue.

Phosphatases are a group of key enzymes in the removal of phosphate groups from organophosphate ester compounds. There are numerous phosphatases, including alkaline phosphatases, phosphodiesterases and phytases.

The general application and definitions of such compounds are discussed above under the background of the invention section.

The present invention provides novel phosphatase enzymes having enhanced thermostability. Such phosphatases are beneficial in enzyme labeling processes and in certain recombinant DNA techniques, such as in the dephosphorylation of vector DNA prior to insert DNA ligation. The recombinant phosphatase enzymes provide the proteins in a format amenable to efficient production of pure enzyme, which can be utilized in a variety of applications as described herein.
Antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate-antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, *Nature*, *256*:495-497, 1975), the trioma technique, the human B-cell hybridoma technique (Kozbor *et al*., *Immunology Today 4*:72, 1983), and the EBV-hybridoma technique to produce human monoclonal antibodies (Cole *et al*., in *Monoclonal Antibodies and Cancer Therapy,* Alan R. Liss, Inc., pp. 77-96, 1985).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

Antibodies generated against an enzyme of the present invention may be used in screening for similar enzymes from other organisms and samples. Such screening techniques are known in the art, for example, one such screening assay is described in Sambrook and Maniatis, Molecular Cloning: A Laboratory Manual (2d Ed.), vol. 2:Section 8.49, Cold Spring Harbor Laboratory, 1989, which is hereby incorporated by reference in its entirety.

The present invention will be further described with reference to the following examples; however, it is to be understood that the present invention is not limited to such examples. All parts or amounts, unless otherwise specified,- are by weight.

In order to facilitate understanding of the following examples certain frequently occurring methods and/or terms will be described.

"Plasmids" are designated by a lower case "p" preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel *et al*., *Nucleic Acids Res., 8*:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., *et al., Id.*, p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in Sambrook and Maniatis, Molecular Cloning: A Laboratory Manual, Cold Spring Harbor Laboratory, 1989.

One means for isolating the nucleic acid molecules encoding the enzymes of the present invention is to probe a gene library with a natural or artificially designed probe using art recognized procedures (see, for example: Current Protocols in Molecular Biology, Ausubel F.M. *et al.* (EDS.) Green Publishing Company Assoc. and John Wiley Interscience, New York, 1989, 1992). It is appreciated to one skilled in the art that the polynucleotides of SEQ ID NOS:1-16, or fragments thereof (comprising at least 10 or 12 contiguous nucleotides), are particularly useful probes. Other particularly useful probes for this purpose are fragments hybridizable fragments to the sequences of SEQ ID NOS:19-27 (*i.e.*, comprising at least 10 or 12 contiguous nucleotides).

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at Tm -10°C for the oligonucleotide probe. The membrane is then exposed to autoradiographic film for detection of hybridization signals.

Stringent conditions means hybridization will occur only if there is at least 90% identity, preferably 95% identity and most preferably at least 97% identity between the sequences. *See* J. Sambrook *et al.*, Molecular Cloning, A Laboratory Manual (2d Ed. 1989) (Cold Spring Harbor Laboratory) which is hereby incorporated by reference in its entirety.

"Identity" as the term is used herein, refers to a polynucleotide sequence which comprises a percentage of the same bases as a reference polynucleotide (SEQ ID NOS:1-16). For example, a polynucleotide which is at least 90% identical to a reference polynucleotide, has polynucleotide bases which are identical in 90% of the bases which make up the reference polynucleotide and may have different bases in 10% of the bases which comprise that polynucleotide sequence.

The present invention relates to polynucleotides which differ from the reference polynucleotide such that the differences are silent changes, for example, the amino acid sequence encoded by both polynucleotides is the same. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

The polynucleotides of this invention were recovered from genomic gene libraries from the organisms listed in Table 1. Gene libraries were generated in the Lambda ZAP II cloning vector (Stratagene Cloning Systems). Mass excisions were performed on these libraries to generate libraries in the pBluescript phagemid. Libraries were generated and excisions were performed according to the protocols/methods hereinafter described.

The excision libraries were introduced into the *E*. *coli* strain BW14893 F'kanlA. Expression clones were then identified using a high temperature filter assay using phosphatase buffer containing 1 mg/ml BCIP (5-Bromo-4-chloro-3-indolyl phosphate). Expression clones encoding BCIPases were identified and repurified from the following organisms: *Ammonifex degensii* KC4, *Methanococcus igneus* KoL5, *Thermococcus alcaliphilus* AED112RA, *Thermococcus celer, Thermococcus* GU5L5, OC9a, M11TL, *Thermococcus* CL-2 and *Aquifex* VF-5.

Expression clones were identified by use of a high temperature filter assay with either acid phosphatase buffer or alkaline phosphatase buffer containing BCIP. Metcalf, *et al*., Evidence for two phosphonate degradative pathways in Enterobacter Aerogenes, J. Bacteriol., 174:2501-2510 (1992)).

BCIPase activity was tested as follows: An excision library was introduced into the *E. Coli* strain BW14893 F'kan, a pho⁻pnh⁻lac⁻ strain. After growth on 100 mm LB plates containing 100 µg/ml ampicillin, 80 µg/ml methicillin and 1mM IPTG, colony lifts were performed using Millipore HATF membrane filters. The colonies transferred to the filters were lysed with chloroform vapor in 150 mm glass petri dishes. The filters were transferred to 100 mm glass petri dishes containing a piece of Whatman 3MM filter paper saturated with either acid phosphatase buffer (see recipe below) or alkaline phosphatase buffer (see recipe below) containing no BCIP. The dish was placed in the oven at 80-85°C for 30-45 minutes to heat inactivate endogenous *E*. *coli* phosphatases. The filter bearing lysed colonies were then transferred to a 100 mm glass petri dish containing 3MM paper saturated with either acid phosphatase buffer or alkaline phosphatase buffer containing 1 mg/ml BCIP. The dish was placed in the oven at 80-85°C.

Alkaline Phosphatase Buffer (referenced in Sambrook, J. *et al.* (1989) *Molecular Cloning, A Laboratory Manual,* p. 1874) includes 100 mM NaCl, 5 mM MgCl₂ and 100 mM Tris-HCl (pH 9.5). Clones expressing phosphatase activity (when the alkaline phosphatase buffer was used) were derived from libraries derived from the organism identified above.

Acid Phosphatase Buffer includes 100 mM NaCl, 5 mM MgCl₂ and 100 mM Tris-HCL (pH 6.8). Clones expressing phosphatase activity (when the acid phosphatase buffer was used) were derived from the library derived from M11TL.

'Positives' were observed as blue spots on the filter membranes. The following filter rescue technique was used to retrieve plasmid from lysed positive colony.

Filter Rescue Technique: A pasteur pipette (or glass capillary tube) was used to core blue spots on the filter membrane. The small filter disk was placed in an Eppendorf tube containing 20 ul of deionized water. The Eppendorf tube was incubated at 75°C for 5 minutes followed by vortexing to elute plasmid DNA off the filter. Plasmid DNA containing DNA inserts from *Thermococcus alcaliphilus* AEDII12RA was used to transform electrocompetent *E. coli* DH10B cells. Electrocompetent BW14893 F'kan1A *E. coli* cells were used for transformation of plasmid DNA containing inserts from *Ammonifex degensii* KC4, *Methanococcus igneus* KOL5, and *Thermococcus* GU5L5. The filter-lift assay was repeated on transformation plates to identify 'positives.' The transformation plates were returned to 37°C incubator to regenerate colonies. 3 ml of LBamp liquid was inoculated with repurified positives and incubated at 37°C overnight. Plasmid DNA was isolated from these cultures and plasmid insert were sequenced.

In some instances where the plates used for the initial colony lifts contained non-confluent colonies, a specific colony corresponding to a blue spot on the filter could be identified on a regenerated plate and repurified directly, instead of using the filter rescue technique. This "repurification" protocol was used for plasmid DNA containing inserts from the following: *Ammonifex degensii* KC4, *Thermococcus celer*, M11TL, and *Aquifex* VF-5.

The filter rescue technique was used for DNA from the following organisms: *Ammonifex degensii* KC4, *Methanococcus igneus* KOL5, *Thermococcus alcaliphilus* AED1112RA, *Thermococcus* CL-2, and OC9a.

Phosphatases are a group of key enzymes that remove phosphate groups from organophosphate ester compounds. The most important phosphatases for commercial purposes are alkaline phosphatases, phosphodiesterases, and phytases.

Alkaline phosphatases have several commercial applications, including their use in analytical applications as an enzyme label in ELISA immunoassays and enzyme-linked gene probes, and their use in research applications for removing 5' phosphates in polynucleotides prior to end-labeling and for dephosphorylating vectors prior to insert ligation (see also Current Protocols in Molecular Biology, (John Wiley & Sons) (1995), chapter 3, section 10).

Alkaline phosphatase hydrolyzes monophosphate esters, releasing inorganic phosphate and the cognate alcohol compound. It is non-specific with respect to the alcohol moiety, a feature which accounts for the many uses of this enzyme. The enzyme has a pH optimum between 9 and 10, however, it can also work at neutral pH. (From a study of the enzyme industry conducted by Business Communications, Co., Inc., 25 Van Zant Street, Norwalk, CT 06855, 1995.)

Two sources of alkaline phosphatase dominate and compete in the market: animal, from bovine and calf intestinal mucosa, and bacterial, from *E. coli*. Due to the high turnover number of calf intestinal phosphatase, it is often selected as the label in many enzyme immunoassays. The usefulness of calf alkaline phosphatase is limited by its inherently low thermal stability, which is even-further compromised during the chemical preparation of enzyme: antibody conjugates. Bacterial alkaline phosphatase could be an attractive alternative to calf alkaline phosphatase due to bacterial alkaline phosphatase's extreme thermotolerance at temperatures as high as 95°C. (Tomazic-Allen S.J., Recombinant bacterial alkaline phosphatase as an immunodiagnostic enzyme, *Annales de Biologie Clinique*, 1991, 49 (5) :287-90) .

Antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

Antibodies, as described above, may be employed as a probe to screen a library to identify the above-described activities or cross-reactive activities in gene libraries generated from the organisms described above or other organisms.

### Example 1

### Bacterial Expression and Purification of Alkaline Phosphatase Enzymes

DNA encoding the enzymes of the present invention, SEQ ID NOS:1 through 16, were initially amplified from a pBluescript vector containing the DNA by the PCR technique using the primers noted herein. The amplified sequences were then inserted into the respective pQE vector listed beneath the primer sequences, and the enzyme was expressed according to the protocols set forth herein. The 5' and 3' oligonucleotide primer sequences used for subcloning and vectors for the respective genes are as follows:
*Ammonifex degensii* KC4 - 3A1A 
5′ CCGA GAA TTC ATT AAA GAG GAG AAA TTA ACT ATG GGG GCA GGT CCG AAA AGG 3′
5′ CCGA GGA TCC TCA CCG CCC CCT GCG GGT GCG 3′
 Vector: pQET3
*Methanococcus igneus* Ko15 - 9A1A 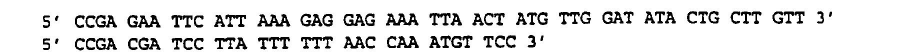 Vector: pQET3
*Thermococcus Alcaliphilus* AEDII12RA -18A 
5' CCGA CAA TTG ATT AAA GAG GAG AAA TTA ACT ATG ATG ATG GAA TTC ACT CGC 3'
5' CGGA GGA TCC CTA CAG TTC TAA AAG TCT TTT A 3'
 Vector: pQET3
*Thermococcus Celer* 25A1A (incorporating Mfe1 restriction site) 
5' CCGA CAA TTG ATT AAA GAG GAG AAA TTA ACT ATG AGA ACC CTG ACA ATA AAC 3'
5' CCGA GGA TCC TTA CAC CCA CAG AAC CCT TAC 3'
 Vector pQET3
*Thermococcus* GU5L5 - 26A1A 
5' CCGA GAA TTC ATT AAA GAG GAG AAA TTA ACT ATG AAA GGA AAG TCT CTT GTT 3'
5' CCGA GGA TCC TCA AGC TTC CTG GAG AAT CAA 3'
 Vector pQET3
*OC9a* - 27A3A 
5' CCGA GAA TTC ATT AAA GAG GAG AAA TTA ACT ATG CCA AGA AAT ATC GCC GCT 3'
5' CCGA GGA TCC TTA AGG CTT CTC GAG GTG GGG GTT 3'
 Vector pQET3
*M11 TL* - 29A1A (incorporating Mfe1 restriction site) 
5' CCGA CAA TTG ATT AAA GAG GAG AAA TTA ACT ATG TAT AAA TGG ATT ATT GAG GG 3'
5' CCGA GGA CTA AAC ATA GTC TAA GTA ATT AGC 3'
 Vector pQET3
*Thermococcus* CL-2 - 30A1A 
5' CCGA GAA TTC ATT AAA GAG GAG AAA TTA ACT ATG AGA ATC CTC CTC ACC AAC 3'
5' CCGA GGA TCC TCA CAG GCT CAG AAG CCT TTG 3'
 Vector pQET3
*Aquifex* VF-5 - 34A1A 
5' CCGA GAA TTC ATT AAA GAG GAG AAA TTA ACT ATG GAA AAC TTA AAA AAG TAC CT 3'
5' CCGA GGA TCC TCA CCG CCC CCT GCG GGT GCG 3'
 Vector pQET3

The restriction enzyme sites indicated correspond to the restriction enzyme sites on the bacterial expression vector indicated for the respective gene (Qiagen, Inc. Chatsworth, CA). The pQE vector encodes antibiotic resistance (Amp^{r}), a bacterial origin of replication (ori), an IPTG-regulatable promoter operator (P/O), a ribosome binding site (RBS), a 6-His tag and restriction enzyme sites.

The pQE vector was digested with the restriction enzymes indicated. The amplified sequences were ligated into the respective pQE vector and inserted in frame with the sequence encoding for the RBS. The native stop codon was incorporated so the genes were not fused to the His tag of the vector. The ligation mixture was then used to transform the E. coli strain M15/pREP4 (Qiagen, Inc.) by electroporation. M15/pREP4 contains multiple copies of the plasmid pREP4, which expresses the lacI repressor and also confers kanamycin resistance (Kan^{r}). Transformants were identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture was used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation.

The primer sequences set out above may also be employed to isolate the target gene from the deposited material by hybridization techniques described above.

### Example 2

### Isolation of A Selected Clone From the Deposited Genomic Clones

A clone is isolated directly by screening the deposited material using the oligonucleotide primers set forth in Example 1 for the particular gene desired to be isolated. The specific oligonucleotides are synthesized using an Applied Biosystems DNA synthesizer.

The two oligonucleotide primers corresponding to the gene of interest are used to amplify the gene from the deposited material. A polymerase chain reaction is carried out in 25 µl of reaction mixture with 0.1 ug of the DNA of the gene of interest. The reaction mixture is 1.5-5 mM MgCl₂, 0.01% (w/v) gelatin, 20 µM each of dATP, dCTP, dGTP,-dTTP, 25 pmol of each primer and 1.25 Unit of Taq polymerase. Thirty cycles of PCR (denaturation at 94°C for 1 min; annealing at 55°C for 1 min; elongation at 72°C for 1 min) are performed with the Perkin-Elmer Cetus 9600 thermal cycler. The amplified product is analyzed by agarose gel electrophoresis and the DNA band with expected molecular weight is excised and purified. The PCR product is verified to be the gene of interest by subcloning and sequencing the DNA product. The ends of the newly purified genes are nucleotide sequenced to identify full length sequences. Complete sequencing of full length genes is then performed by Exonuclease III digestion or primer walking.

Numerous modifications and variations of the present invention are possible in light of the above teachings and, therefore, within the scope of the appended claims, the invention may be practiced otherwise than as particularly described.

The present invention is further characterized by the following items:
1. An isolated polynucleotide selected from the group consisting of:
   (a) a polynucleotide encoding an enzyme comprising an amino acid sequence selected from the group of amino acid sequences set forth in SEQ ID NOS:28-36;
   (b) a polynucleotide which is complementary to the polynucleotide of (a); and
   (c) a polynucleotide comprising at least 15 bases of the polynucleotide of (a) or (b).
2. An isolated polynucleotide selected from the group consisting of:
   (a) SEQ ID NOS:19-27, 37-41, 43, 45, 47, 49, 51, or 53;
   (b) SEQ ID NOS:19-27, 37-41, 43, 45, 47, 49, 51, or 53, where T can also be U; and
   (c) fragments of a)or b)that are at least 15 bases in length and that will hybridize to DNA which encodes the amino acid sequence of any of SEQ ID Nos:28-36, 42, 44, 46, 48, 50, 52, or 54.
3. The polynucleotide of item 1 wherein the polynucleotide is DNA.
4. The polynucleotide of item 1 wherein the polynucleotide is RNA.
5. An isolated polynucleotide comprising a polynucleotide having at least 70% identity to a member selected from the group consisting of:
   (a) a polynucleotide encoding an enzyme encoded by the DNA contained in ATCC Deposit No. 97379, wherein said enzyme is selected from the group consisting of Ammonifex degensii KC4, Aquifex VF-5, M11TL, Methanococcus igneus KOL5, Thermococcus AED112RA, and Thermococcus celer, Thermococcus CL-2, and Thermococcus GU5L5.
   (b) a polynucleotide complementary to the polynucleotide of (a); and
   (c) a polynucleotide comprising at least 15 bases of the polynucleotide of (a) and (b).
6. A vector comprising the DNA of item 1 or Claim 2.
7. A host cell comprising the vector of item 6.
8. A process for producing a polypeptide comprising: expressing from the host cell of item 7 a polypeptide encoded by said DNA and isolating the polypeptide.
9. A process for producing a recombinant cell comprising: transforming or transfecting the cell with the vector of item 6 such that the cell expresses the polypeptide encoded by the DNA contained in the vector.
10. An enzyme of which at least a portion is coded for by a polynucleotide of claim 1, and which is selected from the group consisting of:
   (a) an enzyme comprising an amino acid sequence which is at least 70% identical to an amino acid sequence selected from the group of amino acid sequences set forth in SEQ ID NOS:28-36; and
   (b) an enzyme which comprises at least 30 amino acid residues to the enzyme of (a).
11. An enzyme of which at least a portion is coded for by a polynucleotide of item 1, and which is selected from the group consisting of:
   (a) an enzyme comprising an amino acid sequence selected from the group of amino acid sequences set forth in in SEQ ID NOS:28-36, 42, 44, 46, 48, 50, 52, or 54; and
   (b) an enzyme which comprises at least 30 amino acid residues to the enzyme of (a).
12. A method for hydrolyzing phosphate bonds comprising:
   administering an effective amount of an enyzme selected from the group consisting of an enzyme having the amino acid sequence selected from the group of amino acid sequences set forth in SEQ ID NOS:28-36, 42, 44, 46, 48, 50, 52, or 54.

(1) GENERAL INFORMATION:
(i) APPLICANT:
RECOMBINANT BIOCATALYSIS, INC.

(ii) TITLE OF INVENTION:
THERMOSTABLE PHOSPHATASES

(iii) NUMBER OF SEQUENCES: 54

(iv) CORRESPONDENCE ADDRESS:

(A) ADDRESSEE:FISH & RICHARDSON
(B) STREET: 4225 EXECUTIVE SQUARE, STE. 1400
(C) CITY: LA JOLLA
(D) STATE: CA
(E) COUNTRY: USA
(F) ZIP: 92037

(v) COMPUTER READABLE FORM:
(A) MEDIUM TYPE: 3.5 INCH DISKETTE
(B) COMPUTER: IBM PS/2
(C) OPERATING SYSTEM: MS-DOS
(D) SOFTWARE: WORD PERFECT 6.0

(vi) CURRENT APPLICATION DATA:
(A) APPLICATION NUMBER: Unassigned
(B) FILING DATE: June 19, 1997
(C) CLASSIFICATION: Unassigned

(vii) PRIOR APPLICATION DATA:
(A) APPLICATION NUMBER:
(B) FILING DATE:
(C) CLASSIFICATION:

(viii) ATTORNEY/AGENT INFORMATION:
(A) NAME: Haile, Lisa A.
(B) REGISTRATION NUMBER: 38,347
(C) REFERENCE/DOCKET NUMBER: 09010/015WO1

(ix) TELECOMMUNICATION INFORMATION:
(A) TELEPHONE: 619-678-5070
(B) TELEFAX: 619-678-5099

(2)    INFORMATION FOR SEQ ID NO:1:

(i)        SEQUENCE CHARACTERISTICS
           (A) LENGTH:   52 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi)       SEQUENCE DESCRIPTION:  SEQ ID NO:1:

CCGAGAATTC ATTAAAGAGG AGAAATTAAC TATGGGGGCA GGTCCGAAAA GG    52

(2)    INFORMATION FOR SEQ ID NO:2:

(i)        SEQUENCE CHARACTERISTICS
           (A) LENGTH:  31 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi)       SEQUENCE DESCRIPTION:  SEQ ID NO:2:

CCGAGGATCC TCACCGCCCC CTGCGGGTGC G    31

(2)    INFORMATION FOR SEQ ID NO:3:

(i)        SEQUENCE CHARACTERISTICS
           (A) LENGTH:  52  NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi)       SEQUENCE DESCRIPTION:  SEQ ID NO:3:

CCGAGAATTC ATTAAAGAGG AGAAATTAAC TATGTTGGAT ATACTGCTTG TT    52

(2)    INFORMATION FOR SEQ ID NO:4:

(i)        SEQUENCE CHARACTERISTICS
           (A) LENGTH:  32 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi)       SEQUENCE DESCRIPTION:  SEQ ID NO:4:

CCGAGGATCC TTATTTTTTA ACCAAATTTC CC    32

(2) INFORMATION FOR SEQ ID NO:5:

(i) SEQUENCE CHARACTERISTICS
(A) LENGTH: 52 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:5:

CCGACAATTG ATTAAAGAGG AGAAATTAAC TATGATGATG GAATTCACTC GC 52

(2) INFORMATION FOR SEQ ID NO:6:

(i) SEQUENCE CHARACTERISTICS
(A) LENGTH: 32 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:6:

CGGAGGATCC CTACAGTTCT AAAAGTCTTT TA 32

(2) INFORMATION FOR SEQ ID NO:7:

(i) SEQUENCE CHARACTERISTICS
(A) LENGTH: 52 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:7:

CCGACAATTG ATTAAAGAGG AGAAATTAAC TATGAGAACC CTGACAATAA AC 52

(2) INFORMATION FOR SEQ ID NO:8:

(i) SEQUENCE CHARACTERISTICS
(A) LENGTH: 31 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:8:

CCGAGGATCC TTACACCCAC AGAACCCTTA C 31

(2)    INFORMATION FOR SEQ ID NO:9:

(i)        SEQUENCE CHARACTERISTICS
           (A) LENGTH:  52 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi)       SEQUENCE DESCRIPTION:  SEQ ID NO:9:

CCGAGAATTC ATTAAAGAGG AGAAATTAAC TATGAAAGGA AAGTCTCTTG TT                52

(2)    INFORMATION FOR SEQ ID NO:10:

(i)        SEQUENCE CHARACTERISTICS
           (A) LENGTH:  31 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi) SEQUENCE DESCRIPTION:  SEQ ID NO:10:

CCGAGGATCC TCAAGCTTCC TGGAGAATCA A                31

(2)    INFORMATION FOR SEQ ID NO:11:

(i)        SEQUENCE CHARACTERISTICS
           (A) LENGTH:  52 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi) SEQUENCE DESCRIPTION:  SEQ ID NO:11:

CCGAGAATTC ATTAAAGAGG AGAAATTAAC TATGCCAAGA AATATCGCCG CT                52

(2)    INFORMATION FOR SEQ ID NO:12:

(i)        SEQUENCE CHARACTERISTICS
           (A) LENGTH:  34 NUCLEOTIDES
           (B) TYPE:  NUCLEIC ACID
           (C) STRANDEDNESS:  SINGLE
           (D) TOPOLOGY:  LINEAR

(ii)       MOLECULE TYPE:  cDNA

(xi) SEQUENCE DESCRIPTION:  SEQ ID NO:12:

CGGAGGATCC TTAAGGCTTC TCGAGGTGGG GGTT                34

(2) INFORMATION FOR SEQ ID NO:13:

(i) SEQUENCE CHARACTERISTICS
(A) LENGTH: 52 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:13:

CCGACAATTG ATTAAAGAGG AGAAATTAAC TATGTATAAA TGGATTATTG AGGG 54

(2) INFORMATION FOR SEQ ID NO:14:

(i) SEQUENCE CHARACTERISTICS
(A) LENGTH: 34 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:14:

CCGAGGATCC CTAAACATAG TCTAAGTAAT TAGC 34

(2) INFORMATION FOR SEQ ID NO:15:

(i) SEQUENCE CHARACTERISTICS
(A) LENGTH: 52 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:15:

CCGAGAATTC ATTAAAGAGG AGAAATTAAC TATGAGAATC CTCCTCACCA AC 32

(2) INFORMATION FOR SEQ ID NO:16:

(i) SEQUENCE CHARACTERISTICS
(A) LENGTH: 31 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:16:

CCGAGGATCC TCACAGGCTC AGAAGCCTTT G 31

(2) INFORMATION FOR SEQ ID NO:17:

(i) SEQUENCE CHARACTERISTICS
(A) LENGTH: 54 NUCLEOTIDES
(B) TYPE: NUCLEIC ACID
(C) STRANDEDNESS: SINGLE
(D) TOPOLOGY: LINEAR

(ii) MOLECULE TYPE: GENOMIC DNA

(xi) SEQUENCE DESCRIPTION: SEQ ID NO:17:

CCGAGAATTC ATTAAAGAGG AGAAATTAAC TATGGAAAAC TTAAAAAAGT ACCT 54

(2) INFORMATION FOR SEQ ID NO:18:

(i) SEQUENCE CHARACTERISTICS
(A) LENGTH: 31 NUCLEOTIDES
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:18:

CGGAAGATCT TCACACCGCC ACTTCCATAT A 31

(2) INFORMATION FOR SEQ ID NO:19:

(i) SEQUENCE CHARACTERISTICS
(A) LENGTH: 783 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:19:

ATG AGG GGG AGC GGA GTG CGG ATA CTT CTC ACC AAC GAT GAC GGC ATC 48

TTT GCC GAG GGT CTG GGG GCT CTG CGC AAG ATG CTG GAG CCC GTG GCT 96

ACC CTT TAC GTG GTG GCT CCG GAC CGA GAG CGT AGC GCG GCC AGC CAT 144

GCT ATC ACC GTT CAC CGC CCC CTG CGG GTG CGG GAG GCG GGT TTT CGC 192

AGC CCC AGG CTT AAA GGC TGG GTA GTG GAC GGT ACC CCG GCC GAC TGC 240

GTC AAG CTG GGC CTG GAG GTA CTT TTG CCC GAA CGT CCA GAT TTC CTG 288

GTT TCG GGC ATA AAC TAC GGG CCC AAC CTG GGT ACC GAC GTA CTT TAC 336

TCC GGC ACC GTC TCG GCG GCC ATA GAA GGG GTA ATT AAC GGC ATT CCC 384

TCG GTG GCC GTA TCT TTG GCC ACG CGG CGG GAG CCG GAC TAT ACC TGG 432

GCG GCC CGG TTC GTC CTG GTC CTG CTG GAG GAA CTG CGA AAA CAC CAA 480

CTG CCC CCA GGA ACC CTG CTC AAC GTC AAC GTG CCC GAC GGG GTG CCC 528

CGC GGG GTC AAG GTG ACC AAA CTG GGA AGC GTA CGC TAC GTC AAC GTG 576
GTA GAC TGC CGC ACC GAC CCT CGG GGG AAG GCT TAC TAC TGG ATG GCG 624
GGA GAA CCA TTG GAG CTG GAC GGC AAC GAC TCC GAA ACC GAC GTC TGG 672
GCG GTG CGA GAA GGC TAT ATT TCC GTA ACA CCG GTC CAG ATC GAC CTT 720
ACT AAC TAC GGC TTC CTG GAA GAA CTC AAA AAA TGG CGT TTC AAG GAT 768
ATC TTT TCT TCT TAA 783

(2) INFORMATION FOR SEQ ID NO:20:

(i) SEQUENCE CHARACTERISTICS
(A) LENGTH: 765 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:20:

ATG TTG GAT ATA CTG CTT GTT AAT GAT GAT GGC ATT TAT TCA AAT GGA 48
TTA ATA GCT TTG AAG GAT GCA TTA TTG GAA AAA TTT AAT GCG AGG ATT 96
ACT ATT GTA GCC CCA ACA AAT CAG CAG AGT GGT ATT GGT AGG GCA ATA 144
AGT TTA TTC GAG CCG TTA AGG ATA ACT AAA ACC AAA TTA GCA GAT GGT 192
TCT TGG GGA TAT GCA GTT TCA GGA ACC CCA ACA GAT TGC GTT ATA TTG 240
GGC ATT TAT GAG ATA TTA AAG AAG GTA CCT GAT GTA GTT ATA TCA GGA 288
ATA AAC ATT GGA GAA AAC CTT GGG ACT GAA ATA ACA ACT TCT GGA ACG 336
TTG GGG GCT GCG TTT GAA GGG GCC CAT CAT GGG GCT AAG GCA TTA GCA 384
TCA TCA CTC CAA GTT ACC TCT GAC CAT CTA AAG TTT AAA GAG GGG GAG 432
ACC CCA ATA GAC TTC ACA GTC CCA GCA AGA ATT ACT GCA AAT GTT GTT 480
GAG AAG ATG TTG GAT TAT GAT TTC CCA TGT GAT GTC GTC AAC TTA AAC 528
ATT CCA GAA GGA GCA ACA GAA AAG ACA CCG ATT GAA ATC ACA AGG TTG 576
GCA AGG AAA ATG TAT ACA ACA CAC GTT GAG GAA AGA ATA GAT CCA AGA 624
GGG AGG AGT TAT TAT TGG ATT GAT GGG TAT CCT ATT TTA GAG GAA GAG 672
GAA GAC ACT GAT GTC TAT GTT GTT AGA AGA AAG GGA CAT ATT TCT CTA 720
ACC CCA TTA ACA TTA GAC ACA ACA ATT AAA AAT TTA GAG GAA TTT AAG 768
AAA AAA TAT GAG AGA ATA TTA AAT GAA TGA 798

(2) INFORMATION FOR SEQ ID NO:21:

(i) SEQUENCE CHARACTERISTICS
(A) LENGTH: 765 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:21:

ATG ATG ATG GAA TTC ACT CGC GAG GGA ATA AAA GCT GCT GTA GAG GCA 48

CTT CAA GGG TTA GGA GAG ATC TAC GTA GTT GCC CCA ATG TTT CAA AGG 96

AGC GCA AGT GGA AGG GCA ATG ACC ATC CAC AGA CCT CTA AGG GCT AAA 144

AGA ATA AGT ATG AAC GGT GCA AAA GCA GCC TAT GCT TTG GAT GGA ATG 192

CCC GTT GAT TGC GTT ATC TTT GCC ATG GCC AGA TTT GGA GAT TTC GAC 240

CTT GCA ATA AGT GGT GTA AAC TTG GGA GAA AAC ATG AGC ACC GAG ATA 288

ACG GTT TCC GGG ACT GCA AGC GCT GCA ATA GAG GCT GCA ACC CAA GAG 336

ATC CCA AGC ATT CCC ATA AGC CTG GAA GTT AAT AGA GAA AAA CAC AAA 384

TTT GGT GAG GGC GAA GAG ATT GAC TTC TCA GCT GCC AAG TAT TTC CTA 432

AGA AAA ATC GCA ACG GCG GTT TTA AAG AGA GGC CTC CCC AAA GGA GTC 480

GAT ATG CTG AAC GTC AAC GTC CCT TAT GAT GCA AAT GAA AGG ACA GAG 528

ATA GCT TTT ACT CGC CTG GCA AGA AGG ATG TAT AGG CCT TCT ATT GAA 576

GAG CGC ATA GAC CCA AAG GGG AAT CCC TAC TAC TGG ATA GTT GGA ACT 624

CAG TGC CCT AAG GAG GCA TTA GAG CCG GGA ACG GAT ATG TAT GTA GTT 672

AAA GTT GAG AGA AAA GTT AGC GTG ACT CCA ATA AAC ATT GAT ATG ACA 720

GCA AGA GTG AAT TTA GAC GAG ATT AAA AGA CTT TTA GAA CTG TAG 765

(2) INFORMATION FOR SEQ ID NO:22:

(i) SEQUENCE CHARACTERISTICS
(A) LENGTH: 816 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:22:

ATG AGA ACC CTG ACA ATA AAC ACT GAC GCG GAG GGG TTC GTT TTG AGG 48

ATT CTC CTG ACG AAC GAC GAT GGA ATC TAC TCC AAC GGA CTG CGC GCC 96

GCT GTG AAA GCC CTG AGT GAG CTC GGC GAA GTT TAC GTC GTT GCC CCC 144

CTC TTC CAG AGG AGC GCG AGC GGC AGG GCC ATG ACG CTC CAC AGG CCG 192

ATA AGG GCC AAG CGC GTT GAC GTT CCC GGC GCA AAG ATA GCC TAC GGA 240

ATA GAT GGA ACT CCT ACT GAC TGC GTG ATT TTC GCC ATA GCC CGC TTC 288

GGG AGC TTT GGT TTA GCC GTG AGC GGG ATT AAC CTC GGC GAG AAC CTG 336

AGC ACC GAG ATA ACA GTC TCA GGG ACG GCC TCC GCT GCC ATA GAG GCC 384

TCA ACT CAT GGA ATT CCG AGC ATA GCG ATT AGC CTT GAG GTG GAG TGG 432

AAG AAG ACC CTC GGC GAG GGT GAG GGG GTT GAC TTC TCG GTC TCG ACT 480

CAC TTC CTC AAG AGA ATC GCG GGA GCC CTC TTG GAG AGA GGT CTT CCT 528

GAG GGC GTT GAC ATG CTC AAC GTC AAC GTT CCG AGC GAC GCG ACG GAG 576

GAA ACG GAG ATA GCA ATC ACC CGC TTA GCC CGG AAG CGC TAC TCC CCA 624

ACG GTC GAG GAG AGG ATT GAC CCC AAG GGC AAC CCC TAC TAC TGG ATT 672

GTC GGC AAA CTT GTC CAA GAC TTC GAG CCA GGG ACA GAT GCC TAC GCC 720

CTG AAG GTC GAG AGG AAG GTC AGC GTC ACG CCG ATA AAC ATA GAT ATG 768

ACT GCG AGG GTG GAC TTT GAG GAG CTT GTA AGG GTT CTG TGG GTG TAA 816

(2) INFORMATION FOR SEQ ID NO:23:

(i) SEQUENCE CHARACTERISTICS
(A) LENGTH: 1494 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:23:

ATG AAA GGA AAG TCT CTT GTT AGC GGT CTG TTG TTG GGT CTT TTA ATT 48

TTG AGC CTG ATT TCA TTC CAG CCA AGC TTT GCA TAC TCC CCA CAC GGC 96

GGT GTC AAA AAC ATC ATA ATC CTG GTT GGA GAC GGC ATG GGT CTT GGG 144

CAT GTA GAA ATT ACA AAG CTC GTT TAT GGA CAC TTA AAC ATG GAA AAC 192

TTT CCA GTT ACT GGA TTT GAG CTT ACT GAT TCC CTA AGT GGT GAA GTT 240

ACA GAT TCT GCT GCG GCA GGA ACT GCA ATA TCC ACT GGA GCT AAA ACG 288

TAT AAT GGT ATG ATT TCA GTA ACC AAC ATA ACC GGA AAG ATA GTT AAC 336

TTA ACA ACC CTA CTT GAA GTG GCT CAA GAG CTT GGG AAG TCA ACA GGG 384

CTG GTC ACC ACA ACA AGG ATT ACC CAT GCA ACT CCA GCA GTT TTT GCG 432

TCC CAT GTC CCA GAT AGG GAT ATG GAG GGG GAG ATA CCC AAG CAA CTC 480
ATA ATG CAC AAA GTT AAC GTC TTG TTG GGT GGT GGA AGG GAG AAA TTC 528
GAT GAG AAA AAT TTG GAG CTG GCC AAA AAG CAG GGA TAC AAA GTA GTT 576
TTC ACG AAG GAA GAG CTT GAA AAA GTT GAA GGA GAT TAT GTC CTA GGA 624
CTC TTT GCA GAA AGT CAC ATC CCT TAC GTA TTG GAT AGA AAA CCC GAT 672
GAT GTT GGA CTT TTA GAA ATG GCC AAA AAG GCA ATT TCA ATA CTC GAG 720
AAG AAC CCG AGC GGA TTC TTT CTC ATG GTT GAG GGC GGA AGG ATT GAC 768
CAT GCA GCC CAT GGA AAC GAT GTC GCA TCG GTT GTT GCA GAA ACT AAG 816
GAG TTT GAC GAT GTT GTC AGA TAC GTG CTG GAA TAT CCG AAG AAG AGG 864
GGA GAT ACC TTG GTA ATA GTG CTT GCC GAT CAC GAA ACT GGA GGT CTT 912
GCA ATA GGT CTA ACG TAT GGA AAT GCA ATC GAT GAA GAT GCC ATA AGA 960
AAA ATA AAA GCA AGC ACG TTG AGG ATG CCC AAA GAG GTT AAG GCA GGG 1008
AGT AGT GTA AAA GAG TCC TCA AAG GTA TGC CGG ATT TGT CCC AAC AGA 1056
GGA AGA AGT CAG TAT ATT GAG AAT GCG CTG CAC TCG ACA AAC AAG TAT 1104
GCC CTC TCA AAT GCA GTA GCC GAT GTT ATA AAC AGG CGT ATT GGT GTT 1152
GGA TTC ACC TCC TAT GAG CAT ACA GGA GTT CCA GTT CCG CTC TTA GCT 1200
TAC GGT CCC GGG GCA GAG AAC TTC AGA GGT TTC TTA CAC CAT GTG GAT 1248
ACA GCA AGA TTA GTT GCA AAG TTA ATG CTC TTT GGA AGG AGG AAT ATT 1296
CCA GTT ACC ATT TCA AGC GTG AGC AGT GTT AAG GGA GAC ATA ACC GGT 1344
GAT TAC AGG GTT GAT GAG AAG GAT GCC TAC GTT ACG CTC ATG ATG TTT 1392
CTC GGA GAA AAA GTG GAT AAT GAA ATT GAA AAG AGA GTC GAT ATA GAC 1440
AAC AAC GGC ATG GTT GAC TTA AAT GAC GTC ATG TTG ATT CTC CAG GAA 1488
GCT TGA 1494

(2) INFORMATION FOR SEQ ID NO:24:

(i) SEQUENCE CHARACTERISTICS
(A) LENGTH: 1755 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:24:

ATG CCA AGA AAT ATC GCC GCT GTA TGC GCC CTG GCC GCT TTG TTA GGG 48
TCG GCC TGG GCG GCC AAA GTT GCC GTC TAC CCC TAC GAC GGA GCC GCT 96
TTG CTG GCG GGG CAG CGC TTC GAT TTG CGC ATA GAA GCC TCC GAG CTG 144

AAA GGC AAT TTA AAG GCT TAC CGC ATC ACC CTG GAC GGC CAG CCT CTG 192
GCG GGC CTC GAG CAA ACC GCG CAG GGG GCC GGG CAG GCC GAG TGG ACC 240
CTG CGC GGT GCC TTC CTG CGC CCT GGA AGC CAC ACC CTC GAG GTC AGC 288
CTC ACC GAC GAC GCT GGG GAG AGC AGG AAG AGC GTA CGT TGG GAG GCT 336
CGG CAG AAC CTT CGC TTG CCC CGA GCG GCC AAG AAT GTG ATT CTC TTC 384
ATT GGC GAC GGG ATG GGC TGG AAC ACC CTC AAC GCC GCC CGC ATC ATC 432
GCC AAA GGC TTT AAC CCC GAA AAC GGT ATG CCC AAC GGA AAC CTC GAG 480
ATC GAG AGT GGT TAC GGT GGG ATG GCT ACC GTC ACT ACC GGC AGC TTT 528
GAT AGC TTC ATC GCC GAC TCA GCT AAC TCG GCT TCT TCC ATC ATG ACC 576
GGG CAG AAG GTG CAG GTG AAT GCC CTC AAC GTT TAC CCA TCA AAC CTC 624
AAA GAT ACC CTG GCC TAC CCC CGG ATC GAA ACC CTA GCG GAG ATG CTC 672
AAG CGG GTA CGC GGG GCC AGC ATT GGG GTA GTG ACC ACC ACC TTC GGC 720
ACC GAC GCT ACC CCG GCT TCA CTC AAC GCC CAT ACC CGC CGC CGC GGT 768
GAT TAC CAG GCT ATC GCC GAC ATG TAC TTT GGT AGA GGC GGG TTC GGT 816
GTT CCC TTG GAT GTG ATG CTC TTC GGT GGT TCA CGC GAC TTC ATC CCC 864
CAG AGC ACC CCT GGC TCG CGG CGC AAG GAT AGC ACG GAC TGG ATT GCC 912
GAA TCC CAG AAG CTG GGC TAC ACC TTT GTC AGC ACC CGC AGC GAG CTG 960
CTG GCG GCC AAA CCC ACC GAT AAG CTG TTT GGG CTG TTC AAC ATT GAC 1008
AAC TTC CCC AGC TAC CTA GAC CGC GCA GTG TGG AAG CGG CCC GAG ATG 1056
CTG GGA AGC TTT ACC GAT ATG CCC TAC CTC TGG GAG ATG ACC CAG AAA 1104
GCC GTG GAG GCT CTC TCC AGA AAC GAC AAA GGC TTT TTC TTG ATG GTT 1152
GAG GGG GGA ATG GTG GAT AAG TAC GAG CAC CCC TTG GAC TGG CCC CGC 1200
GCA CTT TGG GAT GTA CTC GAG CTG GAC CGC GCG GTG GCT TGG GCC AAG 1248
GGC TAT GCG GCC TCC CAC CCC GAT ACC CTG GTG ATT GTC ACC GCC GAC 1296
CAC GCT CAC TCG ATC TCG GTG TTT GGC GGT TAC GAC TAC TCC AAG CAG 1344
GGC CGG GAG GGG GTG GGG GTT TAT GAG GCC GCC AAG TTC CCC ACC TAC 1392
GGC GAC AAA AAA GAC GCC AAC GGC TTT CCC TTG CCC GAC ACC ACT CGG 1440
GGA ATC GCG GTA GGC TTC GGG GCC ACG CCG GAT TAC TGT GAA ACC TAC 1488
CGG GGC CGC GAG GTC TAC AAA GAC CCC ACC ATC TCC GAC GGC AAA GGT 1536
GGT TAC GTG GCC AAC CCT GAG GTC TGC AAG GAG CCG GGC CTT CCA ACG 1584
TAC CGG CAA CTC CCA GTA GAT AGC GCC CAG GGC GTG CAC ACG GCT GAT 1632
CCC ATG CCG CTG TTT GCC TTT GGC GTG GGG TCT CAG TTC TTC AAT GGC 1680

CTC ATC GAC CAG ACC GAG ATC TTC TTC CGC ATG GCC CAG GCC CTA GGG 1728
TTC AAC CCC CAC CTC GAG AAG CCT TAA 1755

(2) INFORMATION FOR SEQ ID NO:25:

(i) SEQUENCE CHARACTERISTICS
(A) LENGTH: 912 NUCLEOTIDES
(B) TYPE: NUCLEIC ACID
(C) STRANDEDNESS: SINGLE
(D) TOPOLOGY: LINEAR

(ii) MOLECULE TYPE: genomic DNA

(xi) SEQUENCE DESCRIPTION: SEQ ID NO:25:

ATG TAT AAA TGG ATT ATT GAG GGT AAG CTT GCC CAA GCA CCT TTT CCA 48
AGC CTA GGT GAA CTA GCC GAT CTC AAA AGA CTT TTC GAC GCC ATT ATT 96
GTT CTT ACA ATG CCG CAT GAA CAA CCG CTT AAT GAG AAA TAT ATC GAG 144
ATA TTA GAG AGC CAT GGA TTC CAA GTC CTC CAT GTC CCC ACG CTC GAC 192
TTT CAT CCT TTA GAA CTC TTC GAC CTT TTG AAA ACA AGC ATA TTC ATT 240
GAT GAA AAC CTG GAG AGA TCC CAC AGA GTG CTT GTC CAC TGC ATG GGA 288
GGC ATA GGC CGG AGC GGG CTT GTA ACT GCT GCG TAC TTA ATA TTC AAA 336
GGT TAT GAT ATT TAC GAC GCG GTA AAG CAT GTG AGA ACG GTA GTG CCT 384
GGT GCT ATT GAA AAC AGA GGG CAA GCG TTA ATG CTT GAG AAC TAC TAT 432
ACC CTG GTC AAA AGT TTC AAC AGA GAG TTG CTG AGA GAC TAC GGG AAG 480
AAA ATT TTC ACG CTC GGT GAC CCG AAG GCG GTT CTC CAC GCT TCT AAG 528
ACG ACT CAG TTC ACG ATT GAA CTC TTA AGC AAC TTA CAC GTC AAC GAG 576
GCG TTT TCA ATC AGT GCG ATG GCT CAA TCA CTG CTC CAC TTT CAC GAC 624
GTA AAA GTC CGC TCT AAA CTG AAA GAA GTA TTC GAA AAC ATG GAA TTC 672
TCA TCC GCC TCA GAG GAG GTT CTG TCA TTT ATT CAC CTA CTC GAT TTC 720
TAT CAG GAT GGC AGG GTT GTT TTA ACC ATT TAC GAT TAT CTC CCC GAT 768
AGG GTG GAT TTG ATT TTA TTG TGT AAG TGG GGT TGT GAT AAA ATA GTT 816
GAA GTC TCG TCT TCA GCG AAG AAA ACC GTT GAG AAG CTT GTA GGA AGA 864
AAG GTT TCC CTA TCC TGG GCT AAT TAC TTA GAC TAT GTT TAG 912

(2) INFORMATION FOR SEQ ID NO:26:

(i) SEQUENCE CHARACTERISTICS
(A) LENGTH: 774 NUCLEOTIDES
(B) TYPE: NUCLEIC ACID
(C) STRANDEDNESS: SINGLE
(D) TOPOLOGY: LINEAR

(ii)      MOLECULE TYPE:  genomic DNA

(xi)      SEQUENCE DESCRIPTION:  SEQ ID NO:26:

ATG AGA ATC CTC CTC ACC AAC GAC GAC GGC ATC TAT TCC AAC GGT CTG      48

CGC GCG GCG GTG AAG GGC CTG AGC GAG CTC GGC GAG GTC TAC GTC GTC      96

GCC CCG CTC TTC CAG AGG AGC GCG AGC GGT CGG GCG ATG ACC CTA CAC     144

AGG CCG ATA AGG GCA AAG AGG GTT GAC GTT CCC GGC GCG AAG ATA GCG     192

TAT GGC ATA GAC GGA ACG CCG ACC GAC TGC GTG ATT TTT GCC ATC GCC     240

CGC TTC GGC GAC TTT GAT CTG GCG GTC AGC GGG ATA AAC CTA GGC GAG     288

AAC CTG AGC ACG GAG ATA ACC GTC TCC GGA ACG GCC TCG GCG GCG ATA     336

GAG GCT TCC ACC CAC GGG ATT CCA AGT GTA GCT ATA AGC CTC GAG GTC     384

GAG TGG AAG AAG ACC CTC GGC GAG GGG GAG GGT ATT GAC TTC TCG GTT     432

TCA GCA CAC TTC CTG AGA AGG ATA GCG ACG GCT GTC CTT AAG AAG GGC     480

CTG CCT GAA GGG GTG GAC ATG CTC AAC GTG AAC GTC CCT AGC GAC GCC     528

AGC GAG GGG ACT GAG ATC GCC ATA ACG CGC CTC GCG AGG AAG CGC TAT     576

TCT CCG ACG ATA GAG GAG AGG ATA GAC CCC AAG GGC AAC CCC TAC TAC     624

TGG ATC GTT GGC AGG CTC GTC CAG GAG TTC GAG CCG GGC ACG GAC GCC     672

TAC GCT CTG AAA GTC GAG AGA AAG GTC AGC GTC ACG CCC ATA AAC ATC     720

GAC ATG ACT GCG AGG GTT GAC TTT GAG AAC CTT CAA AGG CTT CTG AGC     768

CTG TGA                                                             774

(2)    INFORMATION FOR SEQ ID NO:27:

(i)       SEQUENCE CHARACTERISTICS
          (A) LENGTH:  795 NUCLEOTIDES
          (B) TYPE:  NUCLEIC ACID
          (C) STRANDEDNESS:  SINGLE
          (D) TOPOLOGY:  LINEAR

(ii)      MOLECULE TYPE:  genomic DNA

(xi) SEQUENCE DESCRIPTION:  SEQ ID NO:27:

ATG GAA AAC TTA AAA AAG TAC CTA GAA GTT GCA AAA ATA GCC GCG CTC      48

GCG GGT GGG CAG GTT CTG AAA GAA AAC TTC GGA AAG GTA AAA AAG GAA      96

AAC ATA GAG GAA AAA GGG GAA AAG GAC TTT GTA AGT TAC GTG GAT AAA     144

ACT TCA GAG GAA AGG ATA AAG GAG GTG ATA CTC AAG TTC TTT CCC GAT     192

CAC GAG GTC GTA GGG GAA GAG ATG GGT GCG GAG GGA AGC GGA AGC GAA     240

TAC AGG TGG TTC ATA GAC CCC CTT GAC GGC ACA AAG AAC TAC ATA AAC     288

GGT TTT CCC ATC TTT GCC GTA TCA GTG GGA CTT GTT AAG GGA GAA GAG 336

GCA ATT GTG GGT GCG GTT TAC CTT CCT TAC TTT GAC AAG CTT TAC TGG 384

GGT GCT AAA GGT CTC GGG GCT TAC GTA AAC GGA AAG AGG ATA AAG GTA 432

AAG GAC AAT GAG AGT TTA AAG CAC GCC GGA GTG GTT TAC GGA TTT CCC 480

TCT AGG AGC AGG AGG GAC ATA TCT ATC TAC TTG AAC ATA TTC AAG GAT 528

GTC TTT TAC GAA GTT GGC TCT ATG AGG AGA CCC GGG GCT GCT GCG GTT 576

GAC CTC TGC ATG GTG GCG GAA GGG ATA TTT GAC GGG ATG ATG GAG TTT 624

GAA ATG AAG CCG TGG GAC ATA ACC GCA GGG CTT GTA ATA CTG AAG GAA 672

GCC GGG GGC GTT TAC ACA CTT GTG GGA GAA CCC TTC GGA GTT TCG GAC 720

ATA ATT GCG GGC AAC AAA GCC CTC CAC GAC TTT ATA CTT CAG GTA GCC 768

AAA AAG TAT ATG GAA GTG GCG GTG TGA 795

(2) INFORMATION FOR SEQ ID NO:28:

(i) SEQUENCE CHARACTERISTICS
(A) LENGTH: 260 AMINO ACIDS
(B) TYPE: AMINO ACID
(D) TOPOLOGY: LINEAR

(ii) MOLECULE TYPE: PROTEIN

(xi) SEQUENCE DESCRIPTION: SEQ ID NO:28:

Met Arg Gly Ser Gly Val Arg Ile Leu Leu Thr Asn Asp Asp Gly Ile
                5                   10                  15

Phe Ala Glu Gly Leu Gly Ala Leu Arg Lys Met Leu Glu Pro Val Ala
            20                  25                  30

Thr Leu Tyr Val Val Ala Pro Asp Arg Glu Arg Ser Ala Ala Ser His
        35                  40                  45

Ala Ile Thr Val His Arg Pro Leu Arg Val Arg Glu Ala Gly Phe Arg
    50                  55                  60

Ser Pro Arg Leu Lys Gly Trp Val Val Asp Gly Thr Pro Ala Asp Cys
65                  70                  75                  80

Val Lys Leu Gly Leu Glu Val Leu Leu Pro Glu Arg Pro Asp Phe Leu
                85                  90                  95

Val Ser Gly Ile Asn Tyr Gly Pro Asn Leu Gly Thr Asp Val Leu Tyr
            100                 105                 110

Ser Gly Thr Val Ser Ala Ala Ile Glu Gly Val Ile Asn Gly Ile Pro
        115                 120                 125

Ser Val Ala Val Ser Leu Ala Thr Arg Arg Glu Pro Asp Tyr Thr Trp
    130                 135                 140

Ala Ala Arg Phe Val Leu Val Leu Leu Glu Glu Leu Arg Lys His Gln
145                 150                 155                 160

Leu Pro Pro Gly Thr Leu Leu Asn Val Asn Val Pro Asp Gly Val Pro
                165                 170                 175

Arg Gly Val Lys Val Thr Lys Leu Gly Ser Val Arg Tyr Val Asn Val
            180                 185                 190

Val Asp Cys Arg Thr Asp Pro Arg Gly Lys Ala Tyr Tyr Trp Met Ala
        195                 200                 205

Gly Glu Pro Leu Glu Leu Asp Gly Asn Asp Ser Glu Thr Asp Val Trp
    210                 215                 220

Ala Val Arg Glu Gly Tyr Ile Ser Val Thr Pro Val Gln Ile Asp Leu
225                 230                 235                 240

Thr Asn Tyr Gly Phe Leu Glu Glu Leu Lys Lys Trp Arg Phe Lys Asp
                245                 250                 255

Ile Phe Ser Ser
            260

(2) INFORMATION FOR SEQ ID NO:29:

(i) SEQUENCE CHARACTERISTICS
(A) LENGTH: 265 AMINO ACIDS
(B) TYPE: AMINO ACID
(D) TOPOLOGY: LINEAR

(ii) MOLECULE TYPE: PROTEIN

(xi) SEQUENCE DESCRIPTION: SEQ ID NO:29:

Met Leu Asp Ile Leu Leu Val Asn Asp Asp Gly Ile Tyr Ser Asn Gly
                5                   10                  15

Leu Ile Ala Leu Lys Asp Ala Leu Leu Glu Lys Phe Asn Ala Arg Ile
            20                  25                  30

Thr Ile Val Ala Pro Thr Asn Gln Gln Ser Gly Ile Gly Arg Ala Ile
        35                  40                  45

Ser Leu Phe Glu Pro Leu Arg Ile Thr Lys Thr Lys Leu Ala Asp Gly
    50                  55                  60

Ser Trp Gly Tyr Ala Val Ser Gly Thr Pro Thr Asp Cys Val Ile Leu
65                  70                  75                  80

Gly Ile Tyr Glu Ile Leu Lys Lys Val Pro Asp Val Val Ile Ser Gly
                85                  90                  95

Ile Asn Ile Gly Glu Asn Leu Gly Thr Glu Ile Thr Thr Ser Gly Thr
            100                 105                 110

Leu Gly Ala Ala Phe Glu Gly Ala His His Gly Ala Lys Ala Leu Ala
        115                 120                 125

Ser Ser Leu Gln Val Thr Ser Asp His Leu Lys Phe Lys Glu Gly Glu
    130                 135                 140

Thr Pro Ile Asp Phe Thr Val Pro Ala Arg Ile Thr Ala Asn Val Val
145                 150                 155                 160

Glu Lys Met Leu Asp Tyr Asp Phe Pro Cys Asp Val Val Asn Leu Asn
                165                 170                 175

Ile Pro Glu Gly Ala Thr Glu Lys Thr Pro Ile Glu Ile Thr Arg Leu
            180                 185                 190

Ala Arg Lys Met Tyr Thr Thr His Val Glu Glu Arg Ile Asp Pro Arg
        195                 200                 205

Gly Arg Ser Tyr Tyr Trp Ile Asp Gly Tyr Pro Ile Leu Glu Glu Glu
    210                 215                 220

Glu Asp Thr Asp Val Tyr Val Val Arg Arg Lys Gly His Ile Ser Leu
225                 230                 235                 240

Thr Pro Leu Thr Leu Asp Thr Thr Ile Lys Asn Leu Glu Glu Phe Lys
                245                 250                 255

Lys Lys Tyr Glu Arg Ile Leu Asn Glu
            260                 265

(2)   INFORMATION FOR SEQ ID NO:30:

      (i)       SEQUENCE CHARACTERISTICS
                (A) LENGTH:  254 AMINO ACIDS
                (B) TYPE:  AMINO ACID
                (D) TOPOLOGY:  LINEAR

      (ii)      MOLECULE TYPE:  PROTEIN

      (xi) SEQUENCE DESCRIPTION:  SEQ ID NO:30:

Met Met Met Glu Phe Thr Arg Glu Gly Ile Lys Ala Ala Val Glu Ala
                5                   10                  15

Leu Gln Gly Leu Gly Glu Ile Tyr Val Val Ala Pro Met Phe Gln Arg
            20                  25                  30

Ser Ala Ser Gly Arg Ala Met Thr Ile His Arg Pro Leu Arg Ala Lys
        35                  40                  45

Arg Ile Ser Met Asn Gly Ala Lys Ala Ala Tyr Ala Leu Asp Gly Met
    50                  55                  60

Pro Val Asp Cys Val Ile Phe Ala Met Ala Arg Phe Gly Asp Phe Asp
65                  70                  75                  80

Leu Ala Ile Ser Gly Val Asn Leu Gly Glu Asn Met Ser Thr Glu Ile
                85                  90                  95

Thr Val Ser Gly Thr Ala Ser Ala Ala Ile Glu Ala Ala Thr Gln Glu
            100                 105                 110

Ile Pro Ser Ile Pro Ile Ser Leu Glu Val Asn Arg Glu Lys His Lys
        115                 120                 125

Phe Gly Glu Gly Glu Glu Ile Asp Phe Ser Ala Ala Lys Tyr Phe Leu
    130                 135                 140

Arg Lys Ile Ala Thr Ala Val Leu Lys Arg Gly Leu Pro Lys Gly Val
145                 150                 155                 160

Asp Met Leu Asn Val Asn Val Pro Tyr Asp Ala Asn Glu Arg Thr Glu
                165                 170                 175

Ile Ala Phe Thr Arg Leu Ala Arg Arg Met Tyr Arg Pro Ser Ile Glu
            180                 185                 190

Glu Arg Ile Asp Pro Lys Gly Asn Pro Tyr Tyr Trp Ile Val Gly Thr
        195                 200                 205

Gln Cys Pro Lys Glu Ala Leu Glu Pro Gly Thr Asp Met Tyr Val Val
    210                 215                 220

Lys Val Glu Arg Lys Val Ser Val Thr Pro Ile Asn Ile Asp Met Thr
225                 230                 235                 240

Ala Arg Val Asn Leu Asp Glu Ile Lys Arg Leu Leu Glu Leu
                245                 250

(2)    INFORMATION FOR SEQ ID NO:31:

       (i)       SEQUENCE CHARACTERISTICS
                 (A) LENGTH: 271 AMINO ACIDS
                 (B) TYPE:  AMINO ACID
                 (D) TOPOLOGY:  LINEAR

       (ii)      MOLECULE TYPE:  PROTEIN

       (xi) SEQUENCE DESCRIPTION:  SEQ ID NO:31:

Met Arg Thr Leu Thr Ile Asn Thr Asp Ala Glu Gly Phe Val Leu Arg
                5                   10                  15

Ile Leu Leu Thr Asn Asp Asp Gly Ile Tyr Ser Asn Gly Leu Arg Ala
            20                  25                  30

Ala Val Lys Ala Leu Ser Glu Leu Gly Glu Val Tyr Val Val Ala Pro
        35                  40                  45

Leu Phe Gln Arg Ser Ala Ser Gly Arg Ala Met Thr Leu His Arg Pro
    50                  55                  60

Ile Arg Ala Lys Arg Val Asp Val Pro Gly Ala Lys Ile Ala Tyr Gly
65                  70                  75                  80

Ile Asp Gly Thr Pro Thr Asp Cys Val Ile Phe Ala Ile Ala Arg Phe
                85                  90                  95

Gly Ser Phe Gly Leu Ala Val Ser Gly Ile Asn Leu Gly Glu Asn Leu
            100                 105                 110

Ser Thr Glu Ile Thr Val Ser Gly Thr Ala Ser Ala Ala Ile Glu Ala
        115                 120                 125

Ser Thr His Gly Ile Pro Ser Ile Ala Ile Ser Leu Glu Val Glu Trp
    130                 135                 140

Lys Lys Thr Leu Gly Glu Gly Glu Gly Val Asp Phe Ser Val Ser Thr
145                 150                 155                 160

His Phe Leu Lys Arg Ile Ala Gly Ala Leu Leu Glu Arg Gly Leu Pro
                165                 170                 175

Glu Gly Val Asp Met Leu Asn Val Asn Val Pro Ser Asp Ala Thr Glu
            180                     185                     190

Glu Thr Glu Ile Ala Ile Thr Arg Leu Ala Arg Lys Arg Tyr Ser Pro
        195                     200                     205

Thr Val Glu Glu Arg Ile Asp Pro Lys Gly Asn Pro Tyr Tyr Trp Ile
    210                     215                     220

Val Gly Lys Leu Val Gln Asp Phe Glu Pro Gly Thr Asp Ala Tyr Ala
225                     230                     235                     240

Leu Lys Val Glu Arg Lys Val Ser Val Thr Pro Ile Asn Ile Asp Met
                245                     250                     255

Thr Ala Arg Val Asp Phe Glu Glu Leu Val Arg Val Leu Trp Val
            260                     265                     270

(2)    INFORMATION FOR SEQ ID NO:32:

       (i)       SEQUENCE CHARACTERISTICS
                 (A) LENGTH: 497 AMINO ACIDS
                 (B) TYPE:  AMINO ACID
                 (D) TOPOLOGY:  LINEAR

       (ii)      MOLECULE TYPE:  PROTEIN

       (xi) SEQUENCE DESCRIPTION:  SEQ ID NO:32:

Met Lys Gly Lys Ser Leu Val Ser Gly Leu Leu Leu Gly Leu Leu Ile
                5                       10                      15

Leu Ser Leu Ile Ser Phe Gln Pro Ser Phe Ala Tyr Ser Pro His Gly
            20                      25                      30

Gly Val Lys Asn Ile Ile Ile Leu Val Gly Asp Gly Met Gly Leu Gly
        35                      40                      45

His Val Glu Ile Thr Lys Leu Val Tyr Gly His Leu Asn Met Glu Asn
    50                      55                      60

Phe Pro Val Thr Gly Phe Glu Leu Thr Asp Ser Leu Ser Gly Glu Val
65                      70                      75                      80

Thr Asp Ser Ala Ala Ala Gly Thr Ala Ile Ser Thr Gly Ala Lys Thr
                85                      90                      95

Tyr Asn Gly Met Ile Ser Val Thr Asn Ile Thr Gly Lys Ile Val Asn
            100                     105                     110

Leu Thr Thr Leu Leu Glu Val Ala Gln Glu Leu Gly Lys Ser Thr Gly
        115                     120                     125

Leu Val Thr Thr Thr Arg Ile Thr His Ala Thr Pro Ala Val Phe Ala
    130                     135                     140

Ser His Val Pro Asp Arg Asp Met Glu Gly Glu Ile Pro Lys Gln Leu
145                     150                     155                     160

Ile Met His Lys Val Asn Val Leu Leu Gly Gly Gly Arg Glu Lys Phe
                165                 170                 175

Asp Glu Lys Asn Leu Glu Leu Ala Lys Lys Gln Gly Tyr Lys Val Val
            180                 185                 190

Phe Thr Lys Glu Glu Leu Glu Lys Val Glu Gly Asp Tyr Val Leu Gly
        195                 200                 205

Leu Phe Ala Glu Ser His Ile Pro Tyr Val Leu Asp Arg Lys Pro Asp
    210                 215                 220

Asp Val Gly Leu Leu Glu Met Ala Lys Lys Ala Ile Ser Ile Leu Glu
225                 230                 235                 240

Lys Asn Pro Ser Gly Phe Phe Leu Met Val Glu Gly Gly Arg Ile Asp
                245                 250                 255

His Ala Ala His Gly Asn Asp Val Ala Ser Val Val Ala Glu Thr Lys
            260                 265                 270

Glu Phe Asp Asp Val Val Arg Tyr Val Leu Glu Tyr Pro Lys Lys Arg
        275                 280                 285

Gly Asp Thr Leu Val Ile Val Leu Ala Asp His Glu Thr Gly Gly Leu
    290                 295                 300

Ala Ile Gly Leu Thr Tyr Gly Asn Ala Ile Asp Glu Asp Ala Ile Arg
305                 310                 315                 320

Lys Ile Lys Ala Ser Thr Leu Arg Met Pro Lys Glu Val Lys Ala Gly
                325                 330                 335

Ser Ser Val Lys Glu Ser Ser Lys Val Cys Arg Ile Cys Pro Asn Arg
            340                 345                 350

Gly Arg Ser Gln Tyr Ile Glu Asn Ala Leu His Ser Thr Asn Lys Tyr
        355                 360                 365

Ala Leu Ser Asn Ala Val Ala Asp Val Ile Asn Arg Arg Ile Gly Val
    370                 375                 380

Gly Phe Thr Ser Tyr Glu His Thr Gly Val Pro Val Pro Leu Leu Ala
385                 390                 395                 400

Tyr Gly Pro Gly Ala Glu Asn Phe Arg Gly Phe Leu His His Val Asp
                405                 410                 415

Thr Ala Arg Leu Val Ala Lys Leu Met Leu Phe Gly Arg Arg Asn Ile
            420                 425                 430

Pro Val Thr Ile Ser Ser Val Ser Ser Val Lys Gly Asp Ile Thr Gly
        435                 440                 445

Asp Tyr Arg Val Asp Glu Lys Asp Ala Tyr Val Thr Leu Met Met Phe
    450                 455                 460

Leu Gly Glu Lys Val Asp Asn Glu Ile Glu Lys Arg Val Asp Ile Asp
465                 470                 475                 480

Asn Asn Gly Met Val Asp Leu Asn Asp Val Met Leu Ile Leu Gln Glu
                485                 490                 495

Ala
497

(2) INFORMATION FOR SEQ ID NO:33:

(i) SEQUENCE CHARACTERISTICS
(A) LENGTH: 584 AMINO ACIDS
(B) TYPE: AMINO ACID
(D) TOPOLOGY: LINEAR

(ii) MOLECULE TYPE: PROTEIN

(xi) SEQUENCE DESCRIPTION: SEQ ID NO:33:

Met Pro Arg Asn Ile Ala Ala Val Cys Ala Leu Ala Ala Leu Leu Gly
                5                   10                  15

Ser Ala Trp Ala Ala Lys Val Ala Val Tyr Pro Tyr Asp Gly Ala Ala
            20                  25                  30

Leu Leu Ala Gly Gln Arg Phe Asp Leu Arg Ile Glu Ala Ser Glu Leu
        35                  40                  45

Lys Gly Asn Leu Lys Ala Tyr Arg Ile Thr Leu Asp Gly Gln Pro Leu
    50                  55                  60

Ala Gly Leu Glu Gln Thr Ala Gln Gly Ala Gly Gln Ala Glu Trp Thr
65                  70                  75                  80

Leu Arg Gly Ala Phe Leu Arg Pro Gly Ser His Thr Leu Glu Val Ser
                85                  90                  95

Leu Thr Asp Asp Ala Gly Glu Ser Arg Lys Ser Val Arg Trp Glu Ala
            100                 105                 110

Arg Gln Asn Leu Arg Leu Pro Arg Ala Ala Lys Asn Val Ile Leu Phe
        115                 120                 125

Ile Gly Asp Gly Met Gly Trp Asn Thr Leu Asn Ala Ala Arg Ile Ile
    130                 135                 140

Ala Lys Gly Phe Asn Pro Glu Asn Gly Met Pro Asn Gly Asn Leu Glu
145                 150                 155                 160

Ile Glu Ser Gly Tyr Gly Gly Met Ala Thr Val Thr Thr Gly Ser Phe
                165                 170                 175

Asp Ser Phe Ile Ala Asp Ser Ala Asn Ser Ala Ser Ser Ile Met Thr
            180                 185                 190

Gly Gln Lys Val Gln Val Asn Ala Leu Asn Val Tyr Pro Ser Asn Leu
        195                 200                 205

Lys Asp Thr Leu Ala Tyr Pro Arg Ile Glu Thr Leu Ala Glu Met Leu
    210                 215                 220

Lys Arg Val Arg Gly Ala Ser Ile Gly Val Val Thr Thr Thr Phe Gly
225                 230                 235                 240

Thr Asp Ala Thr Pro Ala Ser Leu Asn Ala His Thr Arg Arg Arg Gly
                245                 250                 255

Asp Tyr Gln Ala Ile Ala Asp Met Tyr Phe Gly Arg Gly Gly Phe Gly
            260                 265                 270

Val Pro Leu Asp Val Met Leu Phe Gly Gly Ser Arg Asp Phe Ile Pro
        275                 280                 285

Gln Ser Thr Pro Gly Ser Arg Arg Lys Asp Ser Thr Asp Trp Ile Ala
    290                 295                 300

Glu Ser Gln Lys Leu Gly Tyr Thr Phe Val Ser Thr Arg Ser Glu Leu
305                 310                 315                 320

Leu Ala Ala Lys Pro Thr Asp Lys Leu Phe Gly Leu Phe Asn Ile Asp
                325                 330                 335

Asn Phe Pro Ser Tyr Leu Asp Arg Ala Val Trp Lys Arg Pro Glu Met
            340                 345                 350

Leu Gly Ser Phe Thr Asp Met Pro Tyr Leu Trp Glu Met Thr Gln Lys
        355                 360                 365

Ala Val Glu Ala Leu Ser Arg Asn Asp Lys Gly Phe Phe Leu Met Val
    370                 375                 380

Glu Gly Gly Met Val Asp Lys Tyr Glu His Pro Leu Asp Trp Pro Arg
385                 390                 395                 400

Ala Leu Trp Asp Val Leu Glu Leu Asp Arg Ala Val Ala Trp Ala Lys
                405                 410                 415

Gly Tyr Ala Ala Ser His Pro Asp Thr Leu Val Ile Val Thr Ala Asp
            420                 425                 430

His Ala His Ser Ile Ser Val Phe Gly Gly Tyr Asp Tyr Ser Lys Gln
        435                 440                 445

Gly Arg Glu Gly Val Gly Val Tyr Glu Ala Ala Lys Phe Pro Thr Tyr
    450                 455                 460

Gly Asp Lys Lys Asp Ala Asn Gly Phe Pro Leu Pro Asp Thr Thr Arg
465                 470                 475                 480

Gly Ile Ala Val Gly Phe Gly Ala Thr Pro Asp Tyr Cys Glu Thr Tyr
                485                 490                 495

Arg Gly Arg Glu Val Tyr Lys Asp Pro Thr Ile Ser Asp Gly Lys Gly
            500                 505                 510

Gly Tyr Val Ala Asn Pro Glu Val Cys Lys Glu Pro Gly Leu Pro Thr
        515                 520                 525

Tyr Arg Gln Leu Pro Val Asp Ser Ala Gln Gly Val His Thr Ala Asp
    530                 535                 540

Pro Met Pro Leu Phe Ala Phe Gly Val Gly Ser Gln Phe Phe Asn Gly
545                 550                 555                 560

Leu Ile Asp Gln Thr Glu Ile Phe Phe Arg Met Ala Gln Ala Leu Gly
                565                 570                 575

Phe Asn Pro His Leu Glu Lys Pro
            580

(2) INFORMATION FOR SEQ ID NO:34:

(i) SEQUENCE CHARACTERISTICS
(A) LENGTH: 301 AMINO ACIDS
(B) TYPE: AMINO ACID
(D) TOPOLOGY: LINEAR

(ii) MOLECULE TYPE: PROTEIN

(xi) SEQUENCE DESCRIPTION: SEQ ID NO:34:

Met Tyr Lys Trp Ile Ile Glu Gly Lys Leu Ala Gln Ala Pro Phe Pro
                5                   10                  15

Ser Leu Gly Glu Leu Ala Asp Leu Lys Arg Leu Phe Asp Ala Ile Ile
            20                  25                  30

Val Leu Thr Met Pro His Glu Gln Pro Leu Asn Glu Lys Tyr Ile Glu
        35                  40                  45

Ile Leu Glu Ser His Gly Phe Gln Val Leu His Val Pro Thr Leu Asp
    50                  55                  60

Phe His Pro Leu Glu Leu Phe Asp Leu Leu Lys Thr Ser Ile Phe Ile
65                  70                  75                  80

Asp Glu Asn Leu Glu Arg Ser His Arg Val Leu Val His Cys Met Gly
                85                  90                  95

Gly Ile Gly Arg Ser Gly Leu Val Thr Ala Ala Tyr Leu Ile Phe Lys
            100                 105                 110

Gly Tyr Asp Ile Tyr Asp Ala Val Lys His Val Arg Thr Val Val Pro
        115                 120                 125

Gly Ala Ile Glu Asn Arg Gly Gln Ala Leu Met Leu Glu Asn Tyr Tyr
    130                 135                 140

Thr Leu Val Lys Ser Phe Asn Arg Glu Leu Leu Arg Asp Tyr Gly Lys
145                 150                 155                 160

Lys Ile Phe Thr Leu Gly Asp Pro Lys Ala Val Leu His Ala Ser Lys
                165                 170                 175

Thr Thr Gln Phe Thr Ile Glu Leu Leu Ser Asn Leu His Val Asn Glu
            180                 185                 190

Ala Phe Ser Ile Ser Ala Met Ala Gln Ser Leu Leu His Phe His Asp
        195                 200                 205

Val Lys Val Arg Ser Lys Leu Lys Glu Val Phe Glu Asn Met Glu Phe
    210                 215                 220

Ser Ser Ala Ser Glu Glu Val Leu Ser Phe Ile His Leu Leu Asp Phe
225                 230                 235                 240

Tyr Gln Asp Gly Arg Val Val Leu Thr Ile Tyr Asp Tyr Leu Pro Asp
                245                 250                 255

Arg Val Asp Leu Ile Leu Leu Cys Lys Trp Gly Cys Asp Lys Ile Val
            260                 265                 270

Glu Val Ser Ser Ser Ala Lys Lys Thr Val Glu Lys Leu Val Gly Arg

275                    280                    285

Lys Val Ser Leu Ser Trp Ala Asn Tyr Leu Asp Tyr Val
    290                    295                    300

(2)      INFORMATION FOR SEQ ID NO:35:

(i)      SEQUENCE CHARACTERISTICS
         (A) LENGTH: 257 AMINO ACIDS
         (B) TYPE:  AMINO ACID
         (D) TOPOLOGY:  LINEAR

(ii)     MOLECULE TYPE:  PROTEIN

(xi)     SEQUENCE DESCRIPTION:  SEQ ID NO:35:

Met Arg Ile Leu Leu Thr Asn Asp Asp Gly Ile Tyr Ser Asn Gly Leu
                5                   10                  15

Arg Ala Ala Val Lys Gly Leu Ser Glu Leu Gly Glu Val Tyr Val Val
            20                  25                  30

Ala Pro Leu Phe Gln Arg Ser Ala Ser Gly Arg Ala Met Thr Leu His
        35                  40                  45

Arg Pro Ile Arg Ala Lys Arg Val Asp Val Pro Gly Ala Lys Ile Ala
    50                  55                  60

Tyr Gly Ile Asp Gly Thr Pro Thr Asp Cys Val Ile Phe Ala Ile Ala
65                  70                  75                  80

Arg Phe Gly Asp Phe Asp Leu Ala Val Ser Gly Ile Asn Leu Gly Glu
                85                  90                  95

Asn Leu Ser Thr Glu Ile Thr Val Ser Gly Thr Ala Ser Ala Ala Ile
            100                 105                 110

Glu Ala Ser Thr His Gly Ile Pro Ser Val Ala Ile Ser Leu Glu Val
        115                 120                 125

Glu Trp Lys Lys Thr Leu Gly Glu Gly Glu Gly Ile Asp Phe Ser Val
    130                 135                 140

Ser Ala His Phe Leu Arg Arg Ile Ala Thr Ala Val Leu Lys Lys Gly
145                 150                 155                 160

Leu Pro Glu Gly Val Asp Met Leu Asn Val Asn Val Pro Ser Asp Ala
                165                 170                 175

Ser Glu Gly Thr Glu Ile Ala Ile Thr Arg Leu Ala Arg Lys Arg Tyr
            180                 185                 190

Ser Pro Thr Ile Glu Glu Arg Ile Asp Pro Lys Gly Asn Pro Tyr Tyr
        195                 200                 205

Trp Ile Val Gly Arg Leu Val Gln Glu Phe Glu Pro Gly Thr Asp Ala
    210                 215                 220

Tyr Ala Leu Lys Val Glu Arg Lys Val Ser Val Thr Pro Ile Asn Ile

225                 230                 235                 240

Asp Met Thr Ala Arg Val Asp Phe Glu Asn Leu Gln Arg Leu Leu Ser
                245                 250                 255

Leu

(2)   INFORMATION FOR SEQ ID NO:36:

      (i)      SEQUENCE CHARACTERISTICS
               (A) LENGTH: 264 AMINO ACIDS
               (B) TYPE:  AMINO ACID
               (D) TOPOLOGY:  LINEAR

      (ii)     MOLECULE TYPE:  PROTEIN

      (xi)     SEQUENCE DESCRIPTION:  SEQ ID NO:36:

Met Glu Asn Leu Lys Lys Tyr Leu Glu Val Ala Lys Ile Ala Ala Leu
                5                   10                  15

Ala Gly Gly Gln Val Leu Lys Glu Asn Phe Gly Lys Val Lys Lys Glu
            20                  25                  30

Asn Ile Glu Glu Lys Gly Glu Lys Asp Phe Val Ser Tyr Val Asp Lys
        35                  40                  45

Thr Ser Glu Glu Arg Ile Lys Glu Val Ile Leu Lys Phe Phe Pro Asp
    50                  55                  60

His Glu Val Val Gly Glu Glu Met Gly Ala Glu Gly Ser Gly Ser Glu
65                  70                  75                  80

Tyr Arg Trp Phe Ile Asp Pro Leu Asp Gly Thr Lys Asn Tyr Ile Asn
                85                  90                  95

Gly Phe Pro Ile Phe Ala Val Ser Val Gly Leu Val Lys Gly Glu Glu
            100                 105                 110

Pro Ile Val Gly Ala Val Tyr Leu Pro Tyr Phe Asp Lys Leu Tyr Trp
        115                 120                 125

Gly Ala Lys Gly Leu Gly Ala Tyr Val Asn Gly Lys Arg Ile Lys Val
    130                 135                 140

Lys Asp Asn Glu Ser Leu Lys His Ala Gly Val Val Tyr Gly Phe Pro
145                 150                 155                 160

Ser Arg Ser Arg Arg Asp Ile Ser Ile Tyr Leu Asn Ile Phe Lys Asp
                165                 170                 175

Val Phe Tyr Glu Val Gly Ser Met Arg Arg Pro Gly Ala Ala Ala Val
            180                 185                 190

Asp Leu Cys Met Val Ala Glu Gly Ile Phe Asp Gly Met Met Glu Phe
        195                 200                 205

Glu Met Lys Pro Trp Asp Ile Thr Ala Gly Leu Val Ile Leu Lys Glu
    210                 215                 220

Ala Gly Gly Val Tyr Thr Leu Val Gly Glu Pro Phe Gly Val Ser Asp

225                 230                 235                 240
Ile Ile Ala Gly Asn Lys Ala Leu His Asp Phe Ile Leu Gln Val Ala
                245                 250                 255
Lys Lys Tyr Met Glu Val Ala Val
            260

Pyrolobus fumarius 1A (1ph7)
SEQ ID NO:37

1 TGC CCG AGC GTG TTG CCA AGA TGC TTG AAA GAA TGC TAT CCA AGG CGG AAT CTA TGC TCG
60

61 GCG ACG CCC AGA GGC TTA TCG AGG AGG GTA AGG CCG TTG AGG CTA AGA AGC TGT TAG CGG
120

121 CTG CTC ATA GGC TAG TAG ATC GCC TAG AGG ATG CTC TCG ACC ACG CCC TCA ACC ATA TAG
180

181 AGC ATC ACA AGG AAC ATC ATG AGG AGC ACC ACA AGG AGC ACG ACT AAC AAC ACT CTT AGA
240

241 ATC TCG AGA CGA GCT TGC TTC CCG TGT CTC TCG CGC CTA GCC AGT TTT TAA TAG CCT AAG
300

301 CCG AGA CCC ACA TTC CAA CAT TAC TCC GTT TGT CAC TAT CAT GTT CTA ATT GTC ACA CGC
360

361 CCC GTA TAA ATT GGG GGA CCT GGA GGA AGC GTT GCC GGT GAC CCC GCG TGG CCA AGA AGG
420

421 CTG TCT GCC CAA TAT GCG GTG GCG ATG TTG AAC TAC CCG ATA ACG TAA TGG ATG GCG AGA
480

481 TCG TGG AGC ACG ACT GTG GGG CAA TGC TAG TCG TGA GGA TCC GGG ATG GCA ATG TTG TTC
540

541 TAG AGC AGT TGG AGC GCG TTG AGG AGG ACT GGG GAG AGT AGA GGC TAT GCG CAT AGC AAT
600

601 CGT TTA TGA CCA TCC GCG TGT TGA GGA GAA GAG GTT AGC TGA GGA AGC GAG GAA GCT TGG
660

661 TCA CGA ACC TGT CCT CTT TAA TAT TGA CTC GTT GCT CTT TCG CCT TGA TAG CCT GGA GCG
720

721 CAT TCT AGG CGA TGT TGA TGT AGT ACT TCA GAG GGC GGT GAG TTA CTT CAA GGC TCT CGA
780

781 GTC TAC AAG GAT ACT CGA GGC TGC CGG CTA CAC TGT CAT CAA CAA TAG TTT AGT GCA GCT
840

841 TAA CTG CGG CGA CAA ACT ATT GAC AAC GAT CTT GCT TGC TAA GCA TGG TGT GCC AAC ACC
900

901 GCG TGC ATA CGC TGC TTT TTC GCG TGA CAC TGC TGT GCG GGC TGC AGA GGA GCT TGG ATA
960

961 CCC CGT TGT TGT CAA GCC CGT CAT TGG TAG TTG GGG TAG GCT TGT GGC TAG GGC TGA TTC
1020

1021 CAG GGA GAG TCT AGA GGC TGT GAT AGA GCA TAG AGA GGT TCT CGG CCC GGC TTA CTA CAA
1080

1081 GGT TCA TTA TGT GCA AGA GTA TGT GCG CAA GCC TCT ACG TGA CAT ACG CGT ATT CGT GAT
1140

1141 TGG TGA TGA GGT TCC CGT GGC GAT ATA CAG GGT TAA CGA GCG TCA TTG GAA GAC TAA CAC
1200

1201 GGC ACT AGG CGC CAA GGC CGA GCC TGC GCC AGT GAC CCC CGA GTT ACG TGA GTT AGC GCT
1260

1261 TCG CGC GGC CAA GGC TGT GGG TGG CGG TGT GCT TGG TAT AGA TGT GTT TGA AGA CCC GGA
1320

1321 GAG AGG CCT CCT CGT GAA CGA GAT TAA CGC GAA CTC GGA CTT CAA GAA CAC TGA GAG GGT
1380

1381 GAC CGG GTT TAA CAT GGC TAG GGC TAT CGT CGA GTA TGC AGT GTC GGT CGC GAA GAG GTG
1440

1441 AAT GGA ATG GAT AGG GTA GAG GTG CTT CTG GAT GAG GCT AGG CGT GGC GCT ATA GAG GGT
1500

1501 GAC GCT CGC CGC GCA TGT GAA GCG GCA TTA AGG CTG GTT GAC GTT GTG CTC CGC GAG GGG
1560

1561 CCT AGG GTT GCA CAG GAG TCT GGG CGT GGG ATT GAA CCC GGT GAT GTA CTA CTA GCT GAG
1620

1621 GCT CTG AGC TTG AGA GCA GAG CAG GTG AAG GAG GAG CCC AAG GCG GAC AAT TGT CTG GAG
1680

1681 CTC GCA AAG GCT GCA TTC CGC CTC TAT AAG CGG CTC CAG GGG ATG GAG TAA AGT TCG CAG
1740

1741 TGT GTT GCC CGT TTT AGC CTC TGC CTT ACT TTC TAC TCG CGT GAG GCG AGT GTC CCT TGA
1800

1801 CAC GTT GCT GGC GCG AGC TGA GAA ACG ACC TCG AGA TGA TAC CCG AGA TCG TCG AGA AGC
1860

1861 AGA TCG AGG AGA CGA TAG TGC CGG AGG GTC TTG GCG AGC AAC GAC TTG TGT TCA TTG GCA
1920

1921 GCG GTG ATT CTT TCG CGG CCG CAC TTG TAG CCG AGC ATG CCG GCA TAG GCG TCG CAC GCG
1980

1981 ATC CTC TTG ATG TGC TAG TGG CTG GCG TTG ATG GGC CTG GCG ACG CTA TAC TCC TAA GCG
2040

2041 TTG GTG GGC GCT CAA AAC GAG TTG TTG ACG CGG CTC GTT TCC TGT CTT CAC GTG GCT TTC
2100

2101 GTA TCA TAG CGG TCA CGG GTA ACG AGA GGA GTC CTC TCG CAC GCA CAG CAC ACG TTA CCG
2160

2161 TGA AGC TCG TCT ATT CTG ACC TCG CCT GTG GCA TGG GCG CCG CAC GCC ATG TCG CTA TGC
2220

2221 TTG CAG CGC TCT CCG CAT TGT TCA ACG CTA GAC CTC GTA TAC CCG AGA AGC TTG TTG AGG
2280

2281 AGC CCC TGC CTT TCG ACC CTC AGG CTG TGT ACG CGG GTG TGG GCG TTG GTG TAG CCT CTG
2340

2341 CCC TGT TCA TGG TGT TGA AGA TCT GCG AGT TGC TCG CAG ACT GCG CCA CCT GGT GGC ATC
2400

2401 TAG AGC AGT TCG CAC ACG CAC CTG TCT ATG GCA CGA GAA GCA ATA TAC TCG TCG TGT ATC
2460

2461 CGA TCC TCG TTG TGA GAG GAG CAC GCT AGA GGA GTA TCT CTC GGC CTT CCG GGA GGC CGG
2520

2521 GTT TGA GGT CAC CAC TGT ACC CGT GTT GAA CGA CCC TTG GTC TAC AGC TAT TCT CCA CGC
2580

2581 TAC GCT GGC CAT CTC CAG TGC TGC AGA GAC CGC CTT CAG TCG CGG CAT TGA GGA GCC GGG
2640

2641 ATA TCG TGC ACA TCC CGC GCT TAG CAG GCT AAC CAG GCT GAT CTA CCT AGA GGA GTA GAA
2700

2701 CCT CTC GAG GAC CGG TAT GTA GTG GTC TAG AGG CTT CCC GTC ATG GTG TAT CGC GAG GCC
2760

2761 TAT TCC TGC TCT CCT CGC GCC TTC CAC GTT GGG CTC ATA ATC ATC TAT GAA TGC TGT TTT
2820

2821 CGC TGG GTC CGC GCG AAG GAG TTG CAT CGC CGC CTC GTA TAT CTT TGT GTG TGG CTT GCA
2880

2881 AAA GCC GAC AAT ATC CCT CGT AAC CAC CGT ATC CAC GAG GTG GGC TAG ATC GTC ACG CTC
2940

2941 TAG AAG TAG ACG TAC GCA TTC GTA GCA CCA GTT GTT CGA GAC TAT GCC GAC CAG TAT CCC
3000

3001 GTT TCT CTT GGC CCA TCT TAG CAG CTC GTA TGT ACC CGG TGC TAC GTA TAC GCC AGA CAG
3060

3061 CAC AGC TGA TTG CAA TAC CCT TGC TAA TGC CTC TGC CCT TGA GGG GGT CGG CGT CAA GCC
3120

3121 GTG TTT TGC GAG GAG CAC GGC AGC CGC ATA CAC TAT ACT TTG TTG CAC GGA GAC ATC CAG
3180

3181 CCT CCA CGT GTC CAT TAC ACG CCT CAC GCT ATC CGG CGT CGC GTC GGC CCC TAG GGC ACG
3240

3241 TAG ATG TCT GGC AGC AGT CTC GTA GAG AGT CTC CTC GTA CCA CTC ATT TGT GAG GTA AAT
3300

3301 GAC GCC ACC TAA ATC CAG CAG GAG TGT AGG GTT ACG CGG CAA GGC GCC TCC TCA TGT ATT
3360

3361 CGA GGA GGC CGC CCG TTG CCA GAA TTT CAG CTA CAA CAC CCC GGA AGG GCG GGA AAC GGT
3420

3421 ACG TCA ACA CCC TAC CAT CCT TCT TGA TGA GCT TCG CTA CAC CCT CGT CAA GGT TTA TCT
3480

3481 CTA TCT CGT CGC CCT CCT CGG CCG CCT CCA CGA GCT CTG GGA GCA CTA TAA CGG GGA GCC
3540

3541 CGT TGT TAA TCG CGT TAC GGT AGA ATA TTC TCG AGA AGC TCT TCG CTA TGA TGG CCT TGA
3600

3601 CGC CTG CAG CCT TGA GAG CTA TCG CGG CTT GCT CCC TGC TAC TAC CCA TAC CAA AGT TCC
3660

3661 TAC CCG CGA CCA GCA CTA CAC CCT TGG ACG CCT TCT TGG GGA ACT CCG GAT CCA GAG GCT
3720

3721 CCA TAG CAT GCT CGG CAA GCT TCT CCG GCT CAG TAT ATA CCA GGT AGC GGG CAG GGA TAA
3780

3781 TCA CGT CGG TGT TGA TGT TAT TGC CGT AAT TGA GCA CAG GGC CCT TCA CGA CAC CCA GGT
3840

3841 TCA AGA GAG GTT CAC CAC AAG TTT GGC CTC GCT ATC CCA GGC TAT AAT CCA GCT GTT TAC
3900

3901 TCG GCC AGC TTC ACC CAC ACA CTT TTC AAC TCC ATT ATC CTT GTA GCG CAA TCT ACC CTT
3960

3961 CTG GGT AGC ACA GCG TTA AGC CCA TAG TGC CAA GGC GCC ACA ATG ATG CCC TCC GGC ACA
4020

4021 TTC TCG TCG GGT ATC AGC CGG AGG CGT ATG GCC CCT CTC TCC GTC TCG AGC CTA GCG TGA
4080

4081 CCG GCG CCA GCC TCC TTA GGG TTG ACT CGT GCG TAT AGC TCG CCG CTC ACA TCT AGC ATC
4140

4141 GCG TTT GTA CAG TAG CTC ACC GGG TCT CTT GCA GTC ACG AGC ACC TTC CTA TCA CCA TCG
4200

4201 GGC ACG ACC GGC TCG ACC GGC GGG TAT AGA CGG ACG CGT ATC CTC GAG ACA CGC CTG GGC
4260

4261 AGG AGG TAC TCG CCT CTC TCC GCA ACC GCC TTG GAG GAA 4299

Thermococcus 9N-2 (31ph1)
SEQ ID NO:38

1 TGG ACT GAT AAA GAA AAA GAA GAG GTT TAA GGG CCT CAA TAT TAA ATT CTA CAC ATT AGA
60

61 TAT CCA AAA TGG AGA ATT ACT TAA TCT AGA GAC TTA CCT TAA GGA GTT ACA TGA GTT CCT
120

121 TAG AGG CCT TAC ATT AAA ACG AAA AGT AGA AGA GGA ACA ATG ACC CCC GAA GAG CTC CTA
180

181 ACC CGC CTC GAA TTC AAA GGA GTA ACC CTC GAA AAG ATG CTC AAT ACT GCG TTA GAG CTC
240

241 TAC ATC GGC GAC GAG CGC GAG AAA GTT CGA GAA AGG CTG AGA GAG CTG ATG CTG AGG TAT
300

301 CTG GGC GAC ATC AAC GTT CAA GCT CTG CTC TTT TCG GCT CTA CTG CTC GAA GAG AAC TTC
360

361 AAG GTT GAG GGC GAC CCC GTG AAC CTT GTG GCC GAC GAG CTC ATC GGC ATG AAC ATC GCC
420

421 GAG CTC ATA GGT GGA AAG ATG GCG CTC TTC AAC TTC TTC TAC TAC GAC ACC AAG AAG CCC
480

481 GGC ATT TTA GCC GAG CTT CCG CCT TTC CTC GAC GAT GCG ATA GGG GGC TTT ATA GCG GGC
540

541 TGT ATG ACA AGG CTG TTC GAG GGG GTG TAC GGT GCG GAA TCT CTT ACC CTT CTT CAC GCG
600

601 GAT TCC GGT CAA AGG CAA CTT CAA AAG GGT TAG AAA TGA GCT CTG GGC ACT TCC CAT TCT
660

661 CGC ACC GGT AAC TTC GGC CCT GGC GAC GCT CGT GGG CTC TGT GCT CGC CGG GGT AAT AAT
720

721 CCT GGG CGG CAA CTA CGC GTT TCA CCC AAC GTC TCG GCA ACC CAC GTG CTG ATA ACC CTC
780

781 ATA GGC TTC GTC GTG GTC TAC AGC ATA CTG TTC TAC ATC TGG CTC CAC TTC GTC AGG AAG
840

841 CTC ATC AGG GAG GGC CCC GAA CCG GTT GAG GGT GAC GTC ACC GCG AAG CCG ACC CCT GCC
900

901 GTT AGC GCC GCG GGA GGT GGT CAG TGA TGG ACT ACG CGA CCG CAT GGT TTT ACT TCT CCG
960

961 CCT TCC TCC TCG GAA TGT ACT TAG CGT TTG ATG GCT TCG ACC TTG GCA TAG GCG CGT TGC
1020

1021 TCG CCC TGA TTA AGG ACC AGA GGG AGC GCG ACA TAC TCG TGA ACA CCA TCG CGC CGG TCT
1080

1081 GGG ACG GCA ACG AGG TCT GGT TCA TCA CCT GGG GTG CCG GCC TCT TCG CGA TGT GGC CGG
1140

1141 CGC TCT ACG CGA CGC TCT TCA GCA CGT TCT ACC TTG CCG TCT GGC TGC TCG CGT TCC TGT
1200

1201 TCA TAT TCA GGG CTG TCG GCT TTG AGT TCA GGA ACA AGA ACA AGG AGC TAT GGG ACA AGC
1260

1261 TCT TCG CTC TCG TCA GCG CGT TAA TCC CGC TCG TCA TCG GCG TCA TAG TCG GCA ACC TCA
1320

1321 TCA TGG GAA TTC CCA TTG ACG CCA AGG GCT TCC ACG GCT CAC TGC TGA CGC TCT TCA GGC
1380

1381 CCT ACC CGC TCA TCG TCG GCC TCT TCA TAC TCT TCG CGG TGA CCT GGC ACG GAG CCA ACT
1440

1441 GGG GCG TCT ACA AAA CCA CAG GAA AGC TCC AGG AGC AGA TGA GGG AGC TCG CCT TCA AGG
1500

1501 CCT GGC TCC TGA CCG TCG TCT TCC TCC TGC TCA CAG TCA TCG GCA TGA AAA TCT GGG CCC
1560

1561 CAC TGA GGT TCG AGA GGG CAC TAA CGC CGC TTG GGC TCC TCC TAA CGG TTG TCA TCC TCG
1620

1621 TGG CAG GAC TGC TCG ACG GAC AGC TCA TCA AGA AAG GGG AGG AGA ATT TGG CCT TCT ACA
1680

1681 TCA GCT GGC TGG CCT TCC CGC TCG TTG TGT TCC TCG TCT ACT ACA CAA TGT ACC CCT ACT
1740

1741 GGG TCA TCT CGA CCA CCG ATC CGA ACT TCA AGC TCA GCA TAC ACG ACC TCG CGG CAT CTC
1800

1801 CGC TGA CCC TCA AGG CCG TCT TGG GAA TCT CGC TGA TCC TGG CGG TCA TCA TCA TGG CCT
1860

1861 ACA CCC TCT ACG TAT ACA GGG CCT TCG GCG GAA AGG TCA CCG AGG CGG AGG GCT ACT ACT
1920

1921 GAG TTC CCC TTT CCT TTT TCG ATA TTC GAA CTT TTT TAG GGA AAA GTT TAT AAT TCG AGT
1980

1981 CAC CTA AGT TCC TTC TGG AAA CCT AAA AAA CGG TGG TCG AAA TGC ACA GAG GCA GAT CTA
2040

2041 CCG GCT GGC CCT ACG ACC GGA AGC CGG TCC TCG TCT TCT GGG AAA CCA CCA AAG CCT GCC
2100

2101 GGC TCA AGT GCA AGC ACT GCA GAG CGG AGG CAA TAC TCC AGG CAC TGC CGG GCG AGC TGA
2160

2161 ACA CGG AGG AGG GAA AGG CCC TCA TCG ATT CCC TCA CCG ACT TCG GAA GGC CCT ACC CGA
2220

2221 TAC TCA TTC TCA CCG GTG GCG ACC CGC TCA TGA GGA AGG ACA TCT TCG AGC TCA TCG AGT
2280

2281 ACG CCG TTG AGA AGG GCA TTC GCG TTG GTC TCG CCC CCG CTG TAA CGC CCC TCC TGA CCG
2340

2341 AGG AAA CAA TCG AGA GAA TCG CGA GGA GCG GAG TTA AGG CGG TAA GCA TAA GCC TCG ACA -
2400

2401 GCC CGT TTC CAG AAG TTC ACG ACG CAA TCA GAG GCA TAG AAG GGA CGT GGG AGA AAA CCG
2460

2461 TCT GGG CCA TCA AGG AGT TCC TGA AAC ACG GCC TAA GCG TTC AGG TGA ACA CGG TTG TGA
2520

2521 TGC GCG AGA CCG TTG AAG GAC TGC CCG AGA TGG TGA AAC TGC TTA AAG ACC TCG GCG TCG
2580

2581 AAA TCT GGG AGG TCT TCT ACC TCG TCC CGA CCG GGA GGG GCA ACT TCG AGA GCG ACC TGA
2640

2641 GGC CGG AGG AGT GGG AGG ACG TCA CAC ACT TCC TCT ACG AGG CCT CGA AGC ACC TCC TCG
2700

2701 TGA GGA CCA CCG AGG GCC CGA TGT TCA GGC GAG TGG CGA TAA TGA GGA AAG CCC TTG AGG
2760

2761 AGA AGG GAT TCG ACC CCG ACG AGG TTC TCA AGC CCG GGG AGC TCT ACT TCC GGC TGA AGA
2820

2821 AAC GGC TCG TTG AGC TTC TCG GCG AGG GGA ACG AGG CGA GGG CCC AAA CTA TGG GAA CGC
2880

2881 GCG ACG GGA AGG GAA TAG TCT TCA TCG CCT ACA ACG GCA ACG TCT ACC CGA GCG GTT TCC
2940

2941 TGC CCT TCA GCG TCG GCA ACG TCC GCG AGA AAA GTT TGG TTG AGA TTT ACA GGG AGA GTG
3000

3001 AAC TTA TGA AAA AGC TCC GCT CGG CCG AGT TCG AGG GGC GCT GCG GGA GGT GCG AGT TCA
3060

3061 GGG AAA TCT GCG GGG GAA GCA GGG CGA GGG CCT ACG CCT ATC GCT TAA ACC CGC TCG CCG
3120

3121 AAG ACC CTG CCT GCC CGT ACG AGC CGG GCT CAT ACC TAA GGC TCG CCA AAA AGT TCA ATC
3180

3181 TTC ACC TTC CGA TTG AGA TTT TTG GAG CCC AAA AGC CGA TTT GAG GTG ATG GAA ATG AGG
3240

3241 TGG AAG GCT GTT TTA CTG ATT GGA ATC CTC CTC GTG TCT GTC CTC GGT GCC GGA TGC GTT
3300

3301 GGC TCG AAT ACC TCA ACT GAA ACC GGC CCA TCC CAG AAG GAA ATA ACC GTG AAG GAC TTC
3360

3361 TCG GGA AGG AAC ATC ACG GCT AAA GTT CCG GTT CAG CGG GCG GTC GTT CTC TCG ACT TCC
3420

3421 GCC CTC GAA ATA ATC CAG CTC CTC AAC GCG AGC GAC CAG GTC GTC GGT ATT CCA AAG GAG
3480

3481 GCC CAG TAC GAC GCT TTA CTG AGC GAA AGC CTG AAG AAC AAG ACC GTC GTT GGC GCG AGG
3540

3541 CTC AAG ATT GAC GAC TGG GAG AAG GTT TTA GCC CTA AAG CCC GAC CTA ATC ATC GAC CTC
3600

3601 GAC CTG AAG AAG TTC TAC AAC GTT GAC GAG CTC CTC AAC CGC TCC GCC AGC TAC GGA ATT
3660

3661 CCG GTC GTC CTG CTG AGG GAG GAT AAC CTT GAG GAC ATA CCG AAG GCG GTT TCG CTC CTC
3720

3721 GGT CAG CTC TTC GGA AGG GAG AAA GAG GCC AAG GCC TTC GAC GAC TAC TTC AAC GAG CAG
3780

3781 GTG AAG GAG GTT AAG GCC ATA GCC TCA AAG ATT CCA GCG GAG GAG AGA AAG AAG GCG ATA
3840

3841 ATG ATA CAG CCG ATA ATG GGC AAG CTC TAC CTC GTC AAC GGC AAC GAC GTC CTT GCT CAG
3900

3901 GCC GTC AGG CTC GTT GGG GCG GAC TAC CTC GTG AAC CTG ACC TTC AAC GGC TAC ACT CCG
3960

3961 GTT AGG GTC CCG ATG GAC GGG GAG AAG ATA ATA GCG AAC TAC CGC GAT GCA GAC GTC GTA
4020

4021 ATC CTC CTG ACG AGC GCC GTA ACG CCT TAC GAC CAG GTC GAG AAG CTC CGG GAG GAG ATG
4080

4081 CTC AGC GAC GAG GCC TGG AGG GGC ATT AAG GCC GTC AGG GAG GGC AAC GTA GTA ATC CTC
4140

4141 AGG GCG GAC ATG GGT AAA GAC TCC TTC CTC CGC TGG AGC CCG CGC TTG GCA GTG GGA ATC
4200

4201 TGG GTC ATT GGA AAG GCA ATC TAC CCG GAC TAC TAT CCT GAC TGG AAC GAC AAG GCC AAG
4260

4261 GAC TTT CTG AAG AGG TTT TAC GGC CTC TCC TGA TTT TTC TTT TGG GGT GGG ACG ATG ATA
4320

4321 GCG GTC TTT CCA GCG AGT CTC GCG GAA ATC GTC AAA CTC GTC GGG AAA GCC GGG GAG ATA
4380

4381 GCC GGA GTG AAC GAG GAA ATC AGG TTC GAC CCC TGC CTG CCG GAG CTG AAG GAT AAG CCT
4440

4441 GTC ATC GGA AAG TAC CTC AAG CGG AGC AAG AGG ACC TAC TGG GAC GTT TTA GAG GAG CTT
4500

4501 AGG CCG GAC CTT ATC CTC GAC TTC GAT GTT GAG AAC CTG CAC TCC GGG GAC GAG CTG AGG
4560

4561 GCC TTT GGG GAG CGT ATA GGG GCA AGG GTC GAG CTG ATT GAC TTC GAG ACC GTT GAA GGC
4620

4621 TTC GTC GAG GCG AGC AGG AGG ATA GCC GAG CTA ACG AGG GGC GAC TTT TCA AAG CTC GGC
4680

4681 GGG TTC TAT GAG AAG CAC CTG ACG AGG CTG GGT GAG ATA ACT GAA GCC ATC GAG GAG AGG
4740

4741 CCT AAA GCC CTG CTC ACC TAC CGG AAC TTC AAC GTC GTA ACG AGG ACC AAC GTT CTG AGC -
4800

4801 GAC GCG GTT AGA AAA GCA GGG GCG ATG AAC CTC GGC GAG AGG ATA CGG ACA AAG CGG AAG
4860

4861 GTC TAT CCG GTA AAG AAG GAG CGC TTC TTC AGG TCC TTC GGC GAT GCG GAG CAC CTC TTC
4920

4921 CTG CTC ACG AGC ATA ATG ACG GAC AGG GAG AAA ATG GAG GGG ATA AGG GAT GAA ATC CTT
4980

4981 GAC TCG GCC GAG TGG AGG GCA ATG GAA GCC GTT CAG CTC GGA AAC GTG CAC ATA GTT GGC
5040

5041 TCG GCC CTC GAC CTT GAG AGC TTC ATG CGC TGG AGT CCC CGC ATA ATC CCG GGA ATC TAC
5100

5101 CAG CTT GGA AGG TTT ATA CAC GGA ACA AAT CAC CCA CGA ATC TCG TGG AAA TCA CTG CAA
5160

5161 AAG TTT AAA ATC CCC CTC CCA CCC CTC GAA GAA CAA AAA CGC ATC GTC GCC TAC CTC GAC
5220

5221 TCG ATA CAC GAG CGC GCC CAA AAG CTG GTA AAG CTC TAC GAG GAG CGG GAG AAG GAG CTT
5280

5281 GAG AAG CTT TTC CCC GCG GTG CTT GAT AGG GCG TTT AGG GGT GAG CTG TGA TTC CGG GAA
5340

5341 TGG AAT ACG GCT TTG AGA GGG CAA TCT TTG AGA TAG TCA GCG GCT TTG TTC TCT CCC TCG
5400

5401 TAG TCA GGG CTT TCG CTT ACA GTT TTG GTC TTC CAT GGG TAT CCT TTT TGT TCA ACG TTC
5460

5461 TTT CGA TAC TTC TGA CAA TAG GCC TGA TTG ACA AAA TGC CCT TCT GGT CCA TGT CAT ATC
5520

OC1/4V (33ph1)
SEQ ID NO:39

1 AGC TTG GAT ATC GAA TTC CTT ATA TGA AAA ATT CAT CGA ATT GGT AAA AAA CCA CGA TCT
60

61 TCA TGT GGA AAC TGG AAT ATT TGC TGC GCA TAT GCT TGT GGA AAT ACA TAA CGA TGG TCC
120

121 GGT GAC TTT GTT ACT TGA TTC AAG AAA AGG TAT TTT GAA GTC ATC TTT GCT GTC TCT AGG
180

181 AGG ACT ATA TGC CTG AAT ACT CGC ATA GCA ATA AAA ACA ACT TTT TTG CCG AAA ACG ATG
240

241 TGA AGA ATT GTC ATC TAC TGC ATG TAT GTT GTG CAC CCG ATT TGG CAA TTT CTT ATT TGT
300

301 CCG GTG CAC GTG GTG ATA TTT TCT TTT ACA ATC CTA ACA TAC ATC CAA AAG CTG AAT ACG
360

361 AGA AAC GAC ACG CCG AAG TGA TTA AAA TTG CTG CAC TCT TTA AAA TGA ATG TTC TGA AAG
420

421 TTC CTT ATA ATC CTG ACC TGT TCT TCA AGC TTA CTA AAG GAT TAA AAA ATG AAC CTG AAG
480

481 GCG GGA CAA GGT GCG AGA TTT GTA TAA GAA TGC GAC TAG AAA AAA CAA TGG AAT ACG CGA
540

541 AAG AAA ATG GCT ACA AGA GTG TTT CCA CAA CGC TAA CAG CCT CTC CAA AGA AAA ATG TAG
600

601 CGA TGA TTG TGA AGA TAG GAA AAG AAC TGG AAA AAA AAT ACG GTG TGG AAT TTT TGC CTA
660

661 ATG TGT ACC GCA AAA GTC CGC TTT ACA ACG ATG CGC AAA AGC TTA TAA CGA AAA TGG GTT
720

721 ATT TAC AGA CAA AAC TAC TGT GGT TGT ATT TTC TCA ATA AGA ACT TCC GTT ATA GTA GCC
780

781 ACT CAA GAA ACT AAA ACC GTA AAA AGT GGG GTC GAA GTA TGA AAA TAT ACC ACA AAT TAG
840

841 AAG AAG TTG AAG AAC ATA AGC GGT CGT ATG CAT CAA TTG CTT TTT CAT CGA AAG TCA GGG
900

901 TTG AAT ATG AAC ATG CTG GCG AAA AAC TTG CCC TCA TCC CTG TAA CTA TTG GAG ACC TTA
960

961 CGG TGG TTA TCG AAA TTG ACG ATG ATA GAG AAG TAT TCA ATA CTT TGT TGA ACG AGC ACA
1020

1021 TCA AAA ACT CTA TCC TGA AAC AGT TTC CGT ATC CGG AAG AGA TTA GAG GGT TAG CCA GAC
1080

1081 ATT TTC GCA CAG AAT TGA AGA ATT TCA GAA TCT TGG TTG TAA AAT ACA ATA GTG TCG AAG
1140

1141 AAA AGG AAT TCT CAA GGT ATT CAC TGT CTA ATA TAA CAT TCG GTG TGG TGT CAT ACA ATA
1200

1201 AAT TTG ATG TCC ATT TGT TAC CAA GTA ATG TAA AAG TCA GAC CGA AGC CAG GAT ACT GTC
1260

1261 TTT CAC ATG TTG TCC AAA AGC CTG AAG AAG GTA TCA GGC AAG CAT TCT TGT TAG CCC GGT
1320

1321 GGT TTG GTG GTG GAA GCT ACG ACC AAC TGC CCA AAT TAG CGC TTG AAA GCA CTG ACA TTG
1380

1381 ACC TTG GAA AGT GGA CAA ATA TAG TCA AAT ACA TCG TTC TGT CAG ATT TTG AAA AGA GGT
1440

1441 ATT TTT CTG GTA TAA TAA AAA AGC TAA ACG AAT TTA GAA GCG AGA CAT ATT TTG ACC CAT
1500

1501 TTG CTA GGC TTG AAA TGA TAT CAC TTG GCA TAA TAC TCG CCA AGT CAG AGG GAG GAG GTA
1560

1561 ACT TTG AAC CAG ACA GTT ACG ATA TCA TTT AGA GCA CTT ACT GAA AAT ATA AAA TTA GCA
1620

1621 CGA GTT GTT ATA CAT ACT TTT CTA ACA TTC CGA GGA GTG TTC GAT AAA GAT ATA TTC GAT
1680

1681 ACG GAA TTG GCT GTA AAC GAA GCG ATT GCA AAC ATT ATT CAG CAT ACA TAC AAA GGT GAA
1740

1741 CCA AAC TAC GTT GTG ATG ACG CTC AAT TGG ATA GAA CCA GAT ACA CTC GAA GTG TTA CTC
1800

1801 CGC GAT TTT GGT CCA AAA GTG GAC CCA ACG AAA ATC AAA CCA CGA GAT TTA GAT GAT ATC
1860

1861 AGA CCA GGA GGA CTC GGA GTT TAT ATA ATT CAA CGC ATC TTC GAC ATT ATG GAA TTC CGA
1920

1921 AAC GTG AGT CAT GGA AAT TTA CTT TAT CTA AAA CGC TCC TTC TTA ATA CCT CCT AAA AAG
1980

1981 CAG GAG CTT GGG AAT TTA AAT AAT GAA CCC TAT CGA GAA TAT TGA AAA AAC CGT CAA AAC
2040

2041 GGG GGA AAG AAG ACA AAT GGG CTT GCT CAC AGG TTT GAC AAA AAA TCC ATC TTT CAT GTC
2100

2101 TGC ATT TTT TGG CTT TTT GGC AGC ACA ATT TTT GAA AGT GGT GAT ATA CAA AGA TTT CCG
2160

2161 CGT ATT TGG TAG ATA CGG TGG TAT GCC CAG TGC TCA TGT TGC AAC AAC CTC AGC ATT AGC
2220

2221 TTG GGC TGT TGG TTA CAC TAC AGG TTT TGA TTC ACC GCT TAC AGC CAT CGC TGC AAT TTT
2280

2281 CCT TGC TAT TAC AAC AGC TGA TGC TGT TGG TTT ACG AAG AAA TGT CGA CCC CAA TAA AGG
2340

2341 ACA TAC ACT AAT GGA AGC TAT CTA TGG CTT CTT ACT TGG GTG GAT AGT CGC TCT GCT TAC
2400

2401 GGT TAA GTT GTA TCG ATA ATT TTG AAT GAG TTG TAG TGA AAT AGC CCA AGT CTT TTT TCG
2460

2461 CAA TTA CAT CAT AAT GCC AGG AGG GTA ATT TAC AAT GTT TTT TAG ATT ACC ATT TAA AGT
2520

2521 TTT TGT TTT TGC AGT TTT GTT GCT TGC CAT CTC GTT AAC AAG TGT TGT TAG TTT TGG ACA
2580

2581 AGA TGA TGA GCA GAT AAA AAC ACC AAA TTG GTT TAG AAG TGC GGT GAT TAA GAA AAG AGC
2640

2641 TGG TAT GAA TCT AAA GAC CGC CCC AGA GTT TGT AGA TGA CCT ATG GAA TGC GAT ATA CAC
2700

2701 TAT AGG CAC AAA ATA CAA CGT TCC CCC AAC GCT TAT AGC CGC TGT CAT TTC TGT AGA AAG
2760

2761 CAA CTT CGC CAA CGT GAA AGG TGC TGG AGA CGT GGT AGG AAT GAT GCA AAT TTC TAT CTC
2820

2821 CAC AGC CAA AAA TAT ATC GAA ACT CCT CGG CCT CGA ACA ACC AAA AAA CGG TTG GGA TGA
2880

2881 GCT CCT CAC AAA TTA TTG GTT GAA TAT AAC TTA CGG TAC CGC ATA CAT CGC TTA TCT TTA
2940

2941 CAA AAA GCA TGG AAC TTT ACA GAA AGC GCT CGA AGA ATA CAA CAA CGG AAA AAA TAA AAC
3000

3001 TAA ATA CGC CCA GCT GAT ACT ACA ACA ATA CAA CCT ATA CGA GAG CCT CCA TTC TGC TGA
3060

3061 AAT AAG AAA TAA CCA GCA ATT GGA TAC AGA TAA TTC TTC GAC ATC TTC TGA AGC AAC AGA
3120

3121 TAC TTT GAA TAC AAC CAG TGC AAC AAA TTC ACA ACC AAC ATC AGA TGC ATC AAA TAC ATC
3180

3181 AGT TAA CAC TTC AGA AAT CAA GTT CCC GCC TCT TTT CGG AGT TGC AGG TTA TTA AGA TAT
3240

3241 TTG TTC GGT AGT TAC TTA GGA ATG TGG GGT GTA TAG TTT GGA AGA TGA AAA AAT GAA ACC
3300

3301 TGA AAC GAT AGT AAA AAT TGA ACA TTT ATC TTT TTC TTA CCC GAG TTT CAG TCT CAA AGA
3360

3361 TGT AAG TTT TGA GGT TCG GAA GGG AAG TTT CTT CGG CAT TAT TGG ACC AAA TGG TTC GGG
3420

3421 AAA AAC CAC GCT ACT CTC ACT CAT TAT GAA ATT CCA AAA GCC AAA AAG TGG GAA AAT AAC
3480

3481 AGT TGA TGG GAA CGA TGT GCT CAG GCT ATC TCA CAA AAA ACT TGC ACA ACT TAT AGC ATA
3540

3541 CAT CGC TCA AGA CTT TAA CCC TAC ATA CGA TTT CAC AGT TGA AGA ATT GGT CGA AAT GGG
3600

3601 AGG AAT CCC CCG CTC ACC ACA TTT TTT CGA AAC ACC TGT TTA CGA GGA AGA ATT AGA AAA
3660

3661 TGC ACT CAA AAC TGT TGA TTT GCT TGA ATA CCG AAA AAG AAT ATT CTC CAC TCT TAG TGG
3720

3721 AGG ACA ACA GCG CAG GGT CTT GAT TGC ACG CGC AAT CTA TCA AAA CAC ACC TAT CAT CAT
3780

3781 TGC TGA TGA ATT GGT TAA TCA CTT GGA TTT AGG GCA AGC AAT TAA AGT GTT AGA TTA TCT
3840

3841 AAA ACA ACT TAC CGA ATG TGG AAA GAC GAT AAT TGG ACA TTC CAC CTG CAG CCC GG 3896

Archaeoglobus lithotrophicus TF2 (5ph1)
SEQ ID NO:40

1 ATG TGC TGC AAG GCG ATT AAG TTG GTA ACG CCA GGT TTT CCC AGT CAC GAC GTT GTA AAA
60

61 CGA CGG CCA GTG AAT TGT AAT ACG ACT CAC TAT AGG GCG AAT TGG GTA CCG GGC CCC CCC
120

121 TCG AGG TCG ACG GTA TCG ATA AGC TTG ATA TCG AAT TCC GTA CGA AAT GCG GGA AAG AGA
180

181 GAA GGA AAA GGA AAG AGA GCA CAG ATT TGG AAA TGA GAC AGA ACA CGA GGA AGA GCA TGG
240

241 TAT GGC AGA GCG TGA AAG AGC ACA TGA GAA CGA GTC TGA AGA AAT GGG CAA GGG CGT TGG
300

301 CAT GGG CGC CCA TGG AAT GAA GAT GGG CAA AGA AGC TCG CGA AAT GGT GAA GGA AGA ATA
360

361 CAA GGA AGC AAA GGA GAG ATA CAA GAA GGC TAG AGA AGA GTT TGA AAG AGC AAA GAA GAT
420

421 GGG ATT GGA CAT CAG AGA GGA GCG CGG ATT CAA GAT GGC CAA GGG ATT CAT GGT AGC TGG
480

481 ACT AGA CGT TGC TGA GAT GTG GCT GGA GAG ACT GAA GGT ACA GGT CAT GAA TAT GGG TGA
540

541 AGA GGC CAA GAT CAC AGA GGA GAC CAA ACT GGA GCT GCT CGC AAA GAT CGA CGA GAA GCT
600

601 TGC AGA AAT CAA AGA GCT GAA GAA CGA AAT CAA TGA GAC CTC CTC ACC TGA AGA GCT GAT
660

661 AGA AAC TGT CAA GAA AAT CAG AAA GGA GTG GAG AGA AAT CAG AGA TGA AAT GAG GGC TCT
720

721 TAC TGG CTA TGT CGC CGT TGC CAA GGT GGA AAA GCT TGT TGA AAA GGC CAA GCA GGT AGA
780

781 GCT AAT GCT TGA GGC AAA GAT CGA GGA GCT CGA TGC TGC AGG AGT TGA TAC AAC CAA ACT
840

841 CGA GGC AAC ACT CGA GGA CTT CTC GGC AAA GGT TAA TGA AGC AGA AGA TTT GAT TGA CAA
900

901 GGC TGA AAA TCT GTT CGA GGA AGG CAA CAT TGC TGA AGG ACA CAT GAC TCT CAA GGA AGC
960

961 CAT AAA GAC TCT CAA GGA AGC CTT CAA GGA TGT CAA GGA AGT TGT CAG CGA GAT GAA GGA
1020

1021 AAT GAA CCA GTA TAG AGT TAG GGA GGG CAA GAT CTT CTA CGG AAA CGA GAC TGG AGA AGT
1080

1081 CTG GGT GGA TGG TAA TGG TAC TGC TGA GTT TAA CGG TAC CGG TAT CGT TGT GAT CAG AGG
1140

1141 AAA CGC AAC ACT TGA GGT CGC ACC AGA AGA TGC GAT CGT GAC ACT GGT CGG CTT CGG CGT
1200

1201 GAA GAG CGT TGA GGG TGG CGT TTC AAG AGT CAG CGG AGA AGG TAA GGC AGT AAT CAG AGG
1260

1261 AGA AAA CCT CAC CGT CAA GGT GGA AGG TGA CGA CTT CAA GCT CAT AGT GAA GGG CTA CGG
1320

1321 TAC ACT CAA ACT CGA TGG TGA GGG TGA ATA CAG GGT AAA GAA GAG CCC ACA GGA AGA GAT
1380

1381 GAC ATT TAA ACT CTT TCT TCA ACT CTA GCA GTT TGA GCA TTG CAT TTC CAA GAT TTT TGC
1440

1441 TGT TAG CTT CGG GAC AAC TTT GAA AAT ACG TCG AGA CAG GCT CAA ATG TTG TCC CAG CAT
1500

1501 TGC AGC TTT CGG CAA AGC GAA CGA GAT TTG CGT TCC GCT CCC CAG CCC AAC ATG GCT TCT
1560

1561 GTA ATC TGA AAA AAC TTC AAG TTC AAC AGC TTT CCC AAA AAC ATC CAA AAG CTT TTC CGC
1620

1621 AAC ACT TCT AAA TCT TTC GAG ATT TAT TGC ATT TCC TTT CAC CGA AAT GCT ATC GGA TTC
1680

1681 TCT TCC CAC AAC CTC GAT ATG CGG CTC TTC CAG AGC AAT ACC CAC TCC ACC GTC AAT CCT
1740

1741 TCC AAC CTG GCC GTT CAA ATC AAT GAG CGT GAT ATG AAT TCT CGA CGG AGT TTT AAC CTT
1800

1801 AAC ATA CAT CTA TAG AAT TTA AAC GGT AAT TAC TTA AGA AGT TTT GGT TTT GCG AAA AAG
1860

1861 AGT TCA AAA TTC ATT CTT TTA ACT GCA CTA CAG CTC ATC TGT GCC TTT TCT CCT TAA TTC
1920

1921 GAT TTT TCT GAG ATA GTT CTG GTA TCT CGT ATC AAC TAT GTA AGC CTC GGG AGC TAT TAC
1980

1981 AGG CAG ATG ATA ACC GGT GAA TAT CCT TAT TAT CTC TCC AGC CTG AAC CGA GCA TGT CAG
2040

2041 TGC ATA TGA TAT CGG ATC GTG ATC GAT GTG AGG ATA CTC CAC CTC GAA GAA AGA CAC ACC
2100

2101 ATC AGG CAG GAA AGT AGT AAT TAT ATC GGG AAT AAA TGG AGC TCC GAG CTC TTC AGC AAC
2160

2161 TTT TGC AGC CAT TGA AAT GTG CTT ATG AGC AAC AAC AAC ATC AAT ACC TTT CAA CTG TCT
2220

2221 CCT GAG TTC TTT ATA ATC ATG CGG GAA GGG ATA AGA GAT TAT ACA CGA ATC AGA ACT CAT
2280

2281 AGG ATG CAC AAC ATC ATA ATC GTT TGC CTC AAG TGG CTT TAT GCT GGC ATC AAG CCT CAC
2340

2341 ATC CAT TGG TGT AAC TAC ATC TCC AAT ATA CCG AAT GCA ACC AAC ACC ACT TCT CCA GAG -
2400

2401 CAA TTC CAT GAG CAT TCT GCT TCC GAT GAC AGC GAC ACT AAA GTT CCT GAG ATA ATC TAT
2460

2461 CTT TTC TTC ATC TGC CAT CCC ATA CCA GGA AAT TTT TCT CAT GGC AAT AGC CCC GCA TCC
2520

2521 ATT AAA TGG TAT TAA TTT TTT GCC GTA TTT TGA GGA GGT AGA TAT TAA CCA ATT ATT TTC
2580

2581 AAA CCA TTT AAG GGC ATC GAT GAA ACA TCC CAA AAC CAG TTC AGC AAA AAA TTA AAT CAC
2640

2641 TGC CAC ACA TTG AGG ACC CCA AAA TGG TGT GAG AAA TGG ACG AAC TGG GAG GAG TTA TTT
2700

2701 TTG ATC TGA TAG AAG AGG AGC CCG AAG TTG AGG AGG ACG ACG AGA TTA AGC TCG CAG AGA
2760

2761 TAT ACA GGC TTG CTA CAA AAC TTA TAA AGT TAC TCG AAG ATC TCA AAA GCC ATG AGC TTA
2820

2821 AAG AGT CAG CAT CTC TTA TGC TCA TAA AGG AAA TTA TCG GTG AAG ACA GAG TTC TGG TTG
2880

2881 GTT TAG CAT CAA AAA TGC TCC AGG ATA TGA GTC TCG GGT TCG AAG AGG ACG AAA AGT ACG
2940

2941 TTT CTT GAT TTT TGA ACT GTA TTT TCT ACA TGC TCT TTT CCC AAC CAC ATT CAG TTG CAT
3000

3001 GCC ATA CGA AAA TTC CAA TGC CCA AAT CCT GGT AAA TGT ACT TTT TCA TAG TAA ATG CTG
3060

3061 CCA AAC CCA GAT TAA ACT CAA TTT CAT CAA CAG GAA AAA GAA AGA ACG AAA AAA AGA CCT
3120

3121 ACA ACA GTC CTA TAA TTG ACC AAA CTT GAT AGA TTA CAA ACA CCA CAG TTG GAA TCA AAG
3180

3181 CAC AGA TGA AAG CTT TCC GGA TTC CTG CAG CC 3212

Methanococcus thermolithoautotrophicus SN1 (14ph1)

Nucleic acid-SEQ ID NO:41
Amino acid-SEQ ID NO:42

1 ATG GAA ATA ATA AAC AAA TTT CTA AAA AAA ATT GGA TAT AAG AAA GAT GGA GAA GAA AAA
60
1 Met Glu Ile Ile Asn Lys Phe Leu Lys Lys Ile Gly Tyr Lys Lys Asp Gly Glu Glu Lys
20

61 AAG GAC AAA TCT AAA ACC AAA ATA AAA ATT GAA GAA GAA AAA ACC ATG GAT ATC GAA ATT
120
21 Lys Asp Lys Ser Lys Thr Lys Ile Lys Ile Glu Glu Glu Lys Thr Met Asp Ile Glu Ile
40

121 CCA AAA ATT GAA CCT ACT GAA AAT TTT AAT CGT GAT GAA ATT GTT TTT GAG GAA GAT AAT
180
41 Pro Lys Ile Glu Pro Thr Glu Asn Phe Asn Arg Asp Glu Ile Val Phe Glu Glu Asp Asn
60

181 GCC TAC GGT ATA TCC CAC AAA GGA AAT AGA ACA AAC AAC GAA GAC AAT ATT TTA ATT AGA
240
61 Ala Tyr Gly Ile Ser His Lys Gly Asn Arg Thr Asn Asn Glu Asp Asn Ile Leu Ile Arg
80

241 AAA ATA AAA GAT ACC TAC ATA TTA GCA GTT GCA GAT GGT GTC GGA GGG CAC AGC TCA GGA
300
81 Lys Ile Lys Asp Thr Tyr Ile Leu Ala Val Ala Asp Gly Val Gly Gly His Ser Ser Gly
100

301 GAT GTT GCA TCA AAG ATG GCA GTG GAT ATT TTA GAA AAC ATT ATC ATG GAA AAA TAC AAT
360
101 Asp Val Ala Ser Lys Met Ala Val Asp Ile Leu Glu Asn Ile Ile Met Glu-Lys Tyr Asn
120

361 GAA AAC CTA TCA ATT GAA GAG ATA AAA GAA CTT TTA AAA GAT GCA TAC ATT ACG GCA CAC
420
121 Glu Asn Leu Ser Ile Glu Glu Ile Lys Glu Leu Leu Lys Asp Ala Tyr Ile Thr Ala His
140

421 AAC AAA ATA AAA GAA AAC GCT ATT GGA GAT AAA GAG GGA ATG GGA ACA ACA CTA ACA ACT
480
141 Asn Lys Ile Lys Glu Asn Ala Ile Gly Asp Lys Glu Gly Met Gly Thr Thr Leu Thr Thr
160

481 GCA ATA GTT AAA GGG GAT AAA TGC GTT ATA GCA AAC TGC GGG GAT AGT AGG GCT TAT TTA
540
161 Ala Ile Val Lys Gly Asp Lys Cys Val Ile Ala Asn Cys Gly Asp Ser Arg Ala Tyr Leu
180

541 ATT AGA GAT GGA GAA ATA GTT TTT AGA ACA AAA GAC CAC TCT TTG GTT CAG GTT TTA GTA
600
181 Ile Arg Asp Gly Glu Ile Val Phe Arg Thr Lys Asp His Ser Leu Val Gln Val Leu Val
200

601 GAT GAA GGA CAT ATT TCA GAG GAG GAC GCA AGG CAT CAT CCA ATG AAA AAT ATC ATT ACC
660
201 Asp Glu Gly His Ile Ser Glu Glu Asp Ala Arg His His Pro Met Lys Asn Ile Ile Thr
220

661 TCA GCA TTG GGA TTG GAT GAA TTT AAG GTA GAT GAT TAC GAA TGG GAT TTA ATT GAT GGT
720
221 Ser Ala Leu Gly Leu Asp Glu Phe Lys Val Asp Asp Tyr Glu Trp Asp Leu Ile Asp Gly
240

721 GAT GTA TTA TTG ATG AGC TCC GAT GGG CTT CAT GAT TAT GTC AGT AAG GAA GAT ATT TTA
780
241 Asp Val Leu Leu Met Ser Ser Asp Gly Leu His Asp Tyr Val Ser Lys Glu Asp Ile Leu
260

781 AAA ACT GTA AAA AAT AAT GAT CAC CCA AAA GAT ATT GTA GAT GAA TTA TTC AAT ACT GCA
840
261 Lys Thr Val Lys Asn Asn Asp His Pro Lys Asp Ile Val Asp Glu Leu Phe Asn Thr Ala
280

841 TTA AAA GAG ACA AGG GAC AAT GTG AGT ATT ATT CGT ATA 879
281 Leu Lys Glu Thr Arg Asp Asn Val Ser Ile Ile Arg Ile 293

Pyrolobus fumarius 1A (1ph1)

SEQ ID NO:43 -Nucleic acid

SEQ ID NO:44-amino acid

1 ATG ACT CTG CTA GCC CTG TAT CAG AAT AAA CGT GTT ATC GTC AAG CTT GGC TGG GGG AGC
60
1 Met Thr Leu Leu Ala Leu Tyr Gln Asn Lys Arg Val Ile Val Lys Leu Gly Trp Gly Ser
20

61 GGC ACT AGC CAA ATA ACT AAC GAG GCG CAA GTG CTG AGC GTA TTG CAC GAT ATG CCT ATA
120
21 Gly Thr Ser Gln Ile Thr Asn Glu Ala Gln Val Leu Ser Val Leu His Asp Met Pro Ile
40

121 GTG CCC AGA CTG CAT ACC CGT CTA GAC TTA GAT GAT GTC AAG CTC GTT GCG ATA GAG TAC
180
41 Val Pro Arg Leu His Thr Arg Leu Asp Leu Asp Asp Val Lys Leu Val Ala Ile Glu Tyr
60

181 ATA CCC TAC AAG AGC CTT AAC GCC GTC GGC CGC TTG AAC CCC CTT AAG GCT GTC ACA GCC
240
61 Ile Pro Tyr Lys Ser Leu Asn Ala Val Gly Arg Leu Asn Pro Leu Lys Ala Val Thr Ala
80

241 GTC TTC TAT ACA CTC GCA TCG CTA GTC CAT ATC CAC GGC CGT GGT TTT GCT CAT TGC GAC
300
81 Val Phe Tyr Thr Leu Ala Ser Leu Val His Ile His Gly Arg Gly Phe Ala His Cys Asp
100

301 CTA AAG CCG GGT AAC GTT ATA CCA GTT CCC AAG CGT GGC ATG GTG TTC ATC GAC TTT GGT
360
101 Leu Lys Pro Gly Asn Val Ile Pro Val Pro Lys Arg Gly Met Val Phe Ile Asp Phe Gly
120

361 GTT GCA CGA CCT TTT GAC GCT GCG GGC TTC GCG GCA GGA ACA CCA GGG TAT ACG TGC CCA
420
121 Val Ala Arg Pro Phe Asp Ala Ala Gly Phe Ala Ala Gly Thr Pro Gly Tyr Thr Cys Pro
140

421 GAG GCT CTC GGC GGC GAG ACC CCC GGC TCT GGC TGC GAT CTC TAC AGC CTT GCC GGC ATA
480
141 Glu Ala Leu Gly Gly Glu Thr Pro Gly Ser Gly Cys Asp Leu Tyr Ser Leu Ala Gly Ile
160

481 TAC TAC TAC TTG GTT ACC GGG TTA AGC CCG CCA CGC GAC CCA AAA GAG TTC GCC AAG GCG
540
161 Tyr Tyr Tyr Leu Val Thr Gly Leu Ser Pro Pro Arg Asp Pro Lys Glu Phe Ala Lys Ala
180

541 CTC TCG TTG GCT CCC GCT CCA AGT AGC CTC TTG GAA CTG TTC ACA CAG CTG GTG CTG GAT
600
181 Leu Ser Leu Ala Pro Ala Pro Ser Ser Leu Leu Glu Leu Phe Thr Gln Leu Val Leu Asp
200

601 CCC GAG TAT CGT AAC AGC CTT GAT CCT CTC CAG CTG TTG AAG ATT GTT GCA TCT TTT AAC
660
201 Pro Glu Tyr Arg Asn Ser Leu Asp Pro Leu Gln Leu Leu Lys Ile Val Ala Ser Phe Asn
220

661 CCG CAA CTG CTA GTC CCT CAT ATC GTT ATA GAT GGT GTT TAC AAG CCG CTA GGT TAC GGC
720
221 Pro Gln Leu Leu Val Pro His Ile Val Ile Asp Gly Val Tyr Lys Pro Leu Gly Tyr Gly
240

721 GAG GTA AGC ATA GGC TCT AGA GGC GTT ATA CGT GTT GAT GGA CGA CCA GTG TAC CTC GCG
780
241 Glu Val Ser Ile Gly Ser Arg Gly Val Ile Arg Val Asp Gly Arg Pro Val Tyr Leu Ala
260

781 GTT AAG AGG CAT GTG AGG GGC ACA AGT ATG TAC GCG TAT ACG GAT CTT GTC GTG TTT AGG
840
261 Val Lys Arg His Val Arg Gly Thr Ser Met Tyr Ala Tyr Thr Asp Leu Val Val Phe Arg
280

841 AGA GGC GAG AAA CTC ATA GTG AGA AGC GGT GAG AGT ATA GAC CTA GAG TTT AAC GAC CTG
900
281 Arg Gly Glu Lys Leu Ile Val Arg Ser Gly Glu Ser Ile Asp Leu Glu Phe Asn Asp Leu
300

901 GTG TTG TTC GAC AAC CAC ATA CTA TAC GTA TTT ATC CTT CCG GAA AGG CCC 951
301 Val Leu Phe Asp Asn His Ile Leu Tyr Val Phe Ile Leu Pro Glu Arg Pro 317

Thermococcus celer (25ph2)

SEQ ID NO:45-nucleic acid

SEQ ID NO:46-amino acid

1 ATG GAC ATC AGG GCC GTT GTT TTT GAC CTC GAC GGG ACG CTT GTG GGT GCT GAG AAG ACT
60
1 Met Asp Ile Arg Ala Val Val Phe Asp Leu Asp Gly Thr Leu Val Gly Ala Glu Lys Thr
20

61 TTC AGC GAG ATA AAG TCC GAG CTT AAA GAA CGG CTG ATT TCC TTA GGG ATT CCC AGG GAG
120
21 Phe Ser Glu Ile Lys Ser Glu Leu Lys Glu Arg Leu Ile Ser Leu Gly Ile Pro Arg Glu
40

121 CTC GTT GGA GAG CTA ACG CCG ATG TAT GAG GGC CTT ATC GAG CTG TCC AGA AAA ACG GGC
180
41 Leu Val Gly Glu Leu Thr Pro Met Tyr Glu Gly Leu Ile Glu Leu Ser Arg Lys Thr Gly
60

181 AGA CCT TTC GAA GAG ATG TAC TCA ATT CTC GTC AAT CTT GAA GTT GAA AGG ATA AGG GAC
240
61 Arg Pro Phe Glu Glu Met Tyr Ser Ile Leu Val Asn Leu Glu Val Glu Arg Ile Arg Asp
80

241 AGC TTT CTC TTC GAG GGG GCA AGG GAG CTC CTC GAC TTT CTT GTG GGG GAG GGA ATA AAG
300
81 Ser Phe Leu Phe Glu Gly Ala Arg Glu Leu Leu Asp Phe Leu Val Gly Glu Gly Ile Lys
100

301 CTT GCC CTC ATG ACC CGG AGC TCC AGA ATG GCT GCC CTT GAG GCC CTG GAG CTT CAC GGC
360
101 Leu Ala Leu Met Thr Arg Ser Ser Arg Met Ala Ala Leu Glu Ala Leu Glu Leu His Gly
120

361 ATT AAG GAC TAC TTT GAG ATT ATT TCA ACG AGG GAT GAT GTC CCT CCC GAG GAG CTG AAA
420
121 Ile Lys Asp Tyr Phe Glu Ile Ile Ser Thr Arg Asp Asp Val Pro Pro Glu Glu Leu Lys
140

421 CCG AAT CCT GGC CAG CTG AGG AGA ATC CTC GGT GAG CTC AAC GTT CAA CCA GAG AAA GCC
480
141 Pro Asn Pro Gly Gln Leu Arg Arg Ile Leu Gly Glu Leu Asn Val Gln Pro Glu Lys Ala
160

481 ATC GTC GTT GGA GAC CAC GGC TAC GAT GTC ATC CCT GCC CGG GAG CTC GGC GCT CTG AGC
540
161 Ile Val Val Gly Asp His Gly Tyr Asp Val Ile Pro Ala Arg Glu Leu Gly Ala Leu Ser
180

541 GTC CTT GTG ACC GGC CAC GAG GCT GGC AGA ATG AGC TTT CAG GTT GAA GCC GAG CCA AAC
600
181 Val Leu Val Thr Gly His Glu Ala Gly Arg Met Ser Phe Gln Val Glu Ala Glu Pro Asn
200

601 TTT GAG GTC GAG AAC CTC ATT CAC CTC AGG AAG CTC TTC GAG AGG CTC CTG TCG AGC TAC
660
201 Phe Glu Val Glu Asn Leu Ile His Leu Arg Lys Leu Phe Glu Arg Leu Leu Ser Ser Tyr
220

661 GTT GTT GTT CCC GCT TAC AAC GAG GAG AAG ACC ATC AAG GGG GTA ATA GAG AAT CTT CTC
720
221 Val Val Val Pro Ala Tyr Asn Glu Glu Lys Thr Ile Lys Gly Val Ile Glu Asn Leu Leu
240

721 AGG TAT TTC AAA AAG GAC GAG ATA ATC GTC GTG AAC GAC GGC TCC AGG GAT AGA ACG GAG
780
241 Arg Tyr Phe Lys Lys Asp Glu Ile Ile Val Val Asn Asp Gly Ser Arg Asp Arg Thr Glu
260

781 GAG ATA GCT CGT TCT TAC GGA GTC CAC GTT CTT ACG CAT CTC GTC AAC AGG GGG CTT GGT
840
261 Glu Ile Ala Arg Ser Tyr Gly Val His Val Leu Thr His Leu Val Asn Arg Gly Leu Gly
280

841 GGG GCC CTC GGA ACG GGC TTT GCC TAT GCC ATC AGA AAA AAC GCC AAA CTT GTC CTC ACA
900
281 Gly Ala Leu Gly Thr Gly Phe Ala Tyr Ala Ile Arg Lys Asn Ala Lys Leu Val Leu Thr
300

901 TTT GAT GCC GAC GGC CAG CAC CTT ATA AGC GAC GCC CTC CGC GTC ATG AGG CCA GTT GCG
960
301 Phe Asp Ala Asp Gly Gln His Leu Ile Ser Asp Ala Leu Arg Val Met Arg Pro Val Ala
320

961 GAG GGC AGG GCG GAC TTT GCG GTC GGC TCA AGG CTC AAA GGT GAC ACG AGC CAG ATG CCC
1020
321 Glu Gly Arg Ala Asp Phe Ala Val Gly Ser Arg Leu Lys Gly Asp Thr Ser Gln Met Pro
340

1021 CTC GTG AAG AAG TTC GGC AAC TTC GTT CTA GAT GCC GTG ACC GCG GTT TTT GCT GGT AAA
1080
341 Leu Val Lys Lys Phe Gly Asn Phe Val Leu Asp Ala Val Thr Ala Val Phe Ala Gly Lys
360

1081 TAC GTC AGC GAC AGT CAG AGC GGG TTA AGG TGT CTA AGC GGC GAC TGC CTG AGG AAA ATC
1140
361 Tyr Val Ser Asp Ser Gln Ser Gly Leu Arg Cys Leu Ser Gly Asp Cys Leu Arg Lys Ile
380

1141 AGG ATA ACC TGC GAC CGC TAT GCC GTG TCG AGT GAG ATT ATA ATA GAG GCC TCC AAA GCG
1200
381 Arg Ile Thr Cys Asp Arg Tyr Ala Val Ser Ser Glu Ile Ile Ile Glu Ala Ser Lys Ala
400

1201 GGC TGT AGA ATT GTC GAA GTT CCT ATC AAG GCT GTT TAC ACT GAG TAC TTT ATG AAG AAG
1260
401 Gly Cys Arg Ile Val Glu Val Pro Ile Lys Ala Val Tyr Thr Glu Tyr Phe Met Lys Lys
420

1261 GGG ACG AAC GTT TTA GAG GGC GTT AAG ATA GCC CTG AAC CTT CTC TTT GAC AAA CTG AGG
1320
421 Gly Thr Asn Val Leu Glu Gly Val Lys Ile Ala Leu Asn Leu Leu Phe Asp Lys Leu Arg
440

Aquifex pyrophilus (28ph1)
SEQ ID NO:47 and 48

1 ATG GAA AAT CTT GAA AAA CTC CTT GAA GTG GCA AAG ATG GCA GCC CTT GCC GGA GGA CAG
60
1 Met Glu Asn Leu Glu Lys Leu Leu Glu Val Ala Lys Met Ala Ala Leu Ala Gly Gly Gln
20

61 GTA TTA AAG GAA AAC TTC GGA AAG ATT AAG CTT GAA AAC ATT GAA GAA AAG GGA GAG AAG
120
21 Val Leu Lys Glu Asn Phe Gly Lys Ile Lys Leu Glu Asn Ile Glu Glu Lys Gly Glu Lys
40

121 GAC TTC GTG AGC TAC GTT GAT AAA ACC TCC GAA GAG AGA ATA AAA GAG CTA ATA CTT AAG
180
41 Asp Phe Val Ser Tyr Val Asp Lys Thr Ser Glu Glu Arg Ile Lys Glu Leu Ile Leu Lys
60

181 TTC TTT CCC GAC CAC GAG GTC GTG GGG GAG GAA AGG GGA AAG GAG GGA AAA GAA AGC CCT
240
61 Phe Phe Pro Asp His Glu Val Val Gly Glu Glu Arg Gly Lys Glu Gly Lys Glu Ser Pro
80

241 TAC AAA TGG TTC ATA GAC CCC CTT GAT GGG ACC AAG AAC TAC ATA AAG GGC TTT CCC ATA
300
81 Tyr Lys Trp Phe Ile Asp Pro Leu Asp Gly Thr Lys Asn Tyr Ile Lys Gly Phe Pro Ile
100

301 TTT GCA GTC TCC GTC GGA CTC GTT AAG GAA AAC GAA CCT ATA GTG GGA GCG GTT TAC CTT
360
101 Phe Ala Val Ser Val Gly Leu Val Lys Glu Asn Glu Pro Ile Val Gly Ala Val Tyr Leu
120

361 CCT TAC TTT GAT ACC CTA TAC TGG GCT TCA AAG GGA AGG GGA GCC TAT AAA AAC GGG GAG
420
121 Pro Tyr Phe Asp Thr Leu Tyr Trp Ala Ser Lys Gly Arg Gly Ala Tyr Lys Asn Gly Glu
140

421 AGG ATA AGC GTA AAG GAA AGG GGG GAG CTC AAG CAC GCG GCG GTT GTT TAC GGA TTT CCA
480
141 Arg Ile Ser Val Lys Glu Arg Gly Glu Leu Lys His Ala Ala Val Val Tyr Gly Phe Pro
160

481 TCA AGA AGC AGG AGG GAT ATA TCT CTT TAC CTG AAT GTG TTT AAA GAG GTC TTT TAC GAA
540
161 Ser Arg Ser Arg Arg Asp Ile Ser Leu Tyr Leu Asn Val Phe Lys Glu Val Phe Tyr Glu
180

541 GTA GGT TCC GTT AGG AGG CCC GGG GCC GCA GCG GTT GAT ATA TGC ATG CTT GCG GAG GGC
600
181 Val Gly Ser Val Arg Arg Pro Gly Ala Ala Ala Val Asp Ile Cys Met Leu Ala Glu Gly
200

601 ATA TTT GAC GGG ATG ATG GAG TTT GAG ATG AAG CCA TGG GAC ATA ACG GCG GGA CTC GTA
660
201 Ile Phe Asp Gly Met Met Glu Phe Glu Met Lys Pro Trp Asp Ile Thr Ala Gly Leu Val
220

661 ATA CTG AAG GAA GCT GGA GGA TTT TAC ACA CTG AAG GGA GAC CCC TTC GGC ATC TCG GAC
720
221 Ile Leu Lys Glu Ala Gly Gly Phe Tyr Thr Leu Lys Gly Asp Pro Phe Gly Ile Ser Asp
240

721 ATA ATA GCG GGA AAC AGG ATG CTC CAC GAC TTC ATT CTC AAG GTT GTG AAT AAA TAC ATG
780
241 Ile Ile Ala Gly Asn Arg Met Leu His Asp Phe Ile Leu Lys Val Val Asn Lys Tyr Met
260

781 AAT AAT GAA AGC ACG 795
261 Asn Asn Glu Ser Thr 265

Bacillus thermoleovorans (68FY5)
SEQ ID NO:49 and 50

1 ATG AGT GAA CAG CCG GTA TTG TCT GTT CAA GGA TTA AGC GGC GGG TAT AGC ATG AAC CGA
60
1 Met Ser Glu Gln Pro Val Leu Ser Val Gln Gly Leu Ser Gly Gly Tyr Ser Met Asn Arg
20

61 CCG GTT CTG CAT GAC GTA ACC TTT CAG GTT GAA CCG GGT GAG ATG GTG GGT TTG ATC GGC
120
21 Pro Val Leu His Asp Val Thr Phe Gln Val Glu Pro Gly Glu Met Val Gly Leu Ile Gly
40

121 CTG AAC GGT GCG GGC AAG AGT ACC ACG ATG AAG CAT ATT CTC GGG CTG ATG AAT CCG CAA
180
41 Leu Asn Gly Ala Gly Lys Ser Thr Thr Met Lys His Ile Leu Gly Leu Met Asn Pro Gln
60

181 AAA GGG AGC ATT CAG GTT CAA GGA AAG AGC CGG ACA GAG CAT TCG GAA GCC TAT CAC GGC
240
61 Lys Gly Ser Ile Gln Val Gln Gly Lys Ser Arg Thr Glu His Ser Glu Ala Tyr His Gly
80

241 GCC TTG GCG TTT GTT CCC GAA TCC CCG CTG CTG TAT GAG GAG ATG ACA GTA CGA GAG CAT
300
81 Ala Leu Ala Phe Val Pro Glu Ser Pro Leu Leu Tyr Glu Glu Met Thr Val Arg Glu His
100

301 CTG GAA TTT ACG GCG CGC TCC TAT GGC GTA TCC CGT GAA GAT TAT GAG GCA CGT TCG GAG
360
101 Leu Glu Phe Thr Ala Arg Ser Tyr Gly Val Ser Arg Glu Asp Tyr Glu Ala Arg Ser Glu
120

361 CAG CTG TCG AAG ATG TTC CGT ATG GAA GAG AAG ATG GAC AGC CTG TCC ACG CAT TTG TCC
420
121 Gln Leu Ser Lys Met Phe Arg Met Glu Glu Lys Met Asp Ser Leu Ser Thr His Leu Ser
140

421 AAA GGG ATG CGC CAA AAA GTG ATG ATC ATG TGC GCA TTC GTA GCC AGA CCG TCC CTG TAC
480
141 Lys Gly Met Arg Gln Lys Val Met Ile Met Cys Ala Phe Val Ala Arg Pro Ser Leu Tyr
160

481 ATC ATT GAC GAG CCC TTT CTT GGG CTT GAT CCG CTT GGG ATA CGC TCG CTG CTT GAC TTC
540
161 Ile Ile Asp Glu Pro Phe Leu Gly Leu Asp Pro Leu Gly Ile Arg Ser Leu Leu Asp Phe
180

541 ATG CTG GAG CTG AAG GCA TCC GGC GCT TCG GTA TTG CTA AGC TCC CAC ATT 591
181 Met Leu Glu Leu Lys Ala Ser Gly Ala Ser Val Leu Leu Ser Ser His Ile 197

Pyrococcus furiosus VC1 (7ph1)
SEQ ID NO:51 and 52

1 ATG AAG AAA ATA ACT ATT AGT AGT TTG CTT CTA CTT TTA CTT ATT TCT ACC AAT TTG AAT
60
1 Met Lys Lys Ile Thr Ile Ser Ser Leu Leu Leu Leu Leu Leu Ile Ser Thr Asn Leu Asn
20

61 CTC GCA TAC GAT TCC CAA GAG AGC GGT ATT AAA AAT ATA ATA ATC CTC ATT GGA GAC GGC
120
21 Leu Ala Tyr Asp Ser Gln Glu Ser Gly Ile Lys Asn Ile Ile Ile Leu Ile Gly Asp Gly
40

121 ATG GGA ATG AGT CAT GTC CAG ATT ACA AAG CTT GTT TAT GGT CAT CTA AAC ATG GAA GAG
180
41 Met Gly Met Ser His Val Gln Ile Thr Lys Leu Val Tyr Gly His Leu Asn Met Glu Glu
60

181 TTC CCA ATT ATT GGA TTC GAA CTT ACT GAG TCA TTA AGT GGG GAA GTT ACG GAC TCC GCT
240
61 Phe Pro Ile Ile Gly Phe Glu Leu Thr Glu Ser Leu Ser Gly Glu Val Thr Asp Ser Ala
80

241 GCA GCA GGA ACT GCA ATA GCA ACT GGA GTC AAA ACA TAT AAT CGA ATG ATT TCA GTT ACT
300
81 Ala Ala Gly Thr Ala Ile Ala Thr Gly Val Lys Thr Tyr Asn Arg Met Ile Ser Val Thr
100

301 AAC ATA ACT GGA AAA GTT ACA AAT CTA ACT ACC TTG CTT GAA ATA GCC CAG GTA CTT GGA
360
101 Asn Ile Thr Gly Lys Val Thr Asn Leu Thr Thr Leu Leu Glu Ile Ala Gln Val Leu Gly
120

361 AAA TCA ACT GGA CTT GTG ACT ACT ACT AGA ATT ACA CAC GCA ACC CCT GCA GTA TTT GCT
420
121 Lys Ser Thr Gly Leu Val Thr Thr Thr Arg Ile Thr His Ala Thr Pro Ala Val Phe Ala
140

421 TCC CAC GTT CCT GAC AGA GAT ATG GAA GAG GAA ATA GCG AGA CAG CTC ATA GCT CAC CGG
480
141 Ser His Val Pro Asp Arg Asp Met Glu Glu Glu Ile Ala Arg Gln Leu Ile Ala His Arg
160

481 GTC AAC GTC CTA TTA GGT GGA GGG AGA AAG AAA TTT GAC GAG AAT ACC CTA AAA ATG GCA
540
161 Val Asn Val Leu Leu Gly Gly Gly Arg Lys Lys Phe Asp Glu Asn Thr Leu Lys Met Ala
180

541 AAA GAA CAG GGA TAT AAT ATA GTC TTC ACG AAA GAA GAG CTC GAG AAA GCA GAG GGT GAG
600
181 Lys Glu Gln Gly Tyr Asn Ile Val Phe Thr Lys Glu Glu Leu Glu Lys Ala Glu Gly Glu
200

601 TTT ATT CTA GGG CTT TTT GCA GAT AGC CAC ATT CCT TAC GTA TTG GAC AGA AAA CCA GAA
660
201 Phe Ile Leu Gly Leu Phe Ala Asp Ser His Ile Pro Tyr Val Leu Asp Arg Lys Pro Glu
220

661 GAT GTT GGA CTT TTG GAA ATG ACT AAA AAA GCA ATT TCA ATA CTA GAG AAA AAT CCA AAT
720
221 Asp Val Gly Leu Leu Glu Met Thr Lys Lys Ala Ile Ser Ile Leu Glu Lys Asn Pro Asn
240

721 GGG TTC TTT CTC ATG ATT GAA GGG GGC AGA ATT GAT CAT GCA GCT CAT GAG AAT GAT ATA
780
241 Gly Phe Phe Leu Met Ile Glu Gly Gly Arg Ile Asp His Ala Ala His Glu Asn Asp Ile
260

781 GCA TCA GTT GTT GCA GAG ACT AAG GAG TTT GAT GAC GTT GTT GGA TAT GTT CTT GAG TAT
840
261 Ala Ser Val Val Ala Glu Thr Lys Glu Phe Asp Asp Val Val Gly Tyr Val Leu Glu Tyr
280

841 GCA AAA AAG AGG GGA GAT ACA CTA GTA ATA GTG CTG GCT GAC CAT GAG ACA GGG GGG CTT
900
281 Ala Lys Lys Arg Gly Asp Thr Leu Val Ile Val Leu Ala Asp His Glu Thr Gly Gly Leu
300

901 GGA TTA GGT CTA ACA TAT GGA GAT GCA ATT AAT GAA GAT GTC ATC AGG AAC ATA AAC GCT
960
301 Gly Leu Gly Leu Thr Tyr Gly Asp Ala Ile Asn Glu Asp Val Ile Arg Asn Ile Asn Ala
320

961 AGT GTG TCG AAA ATT GCT AGT GAA ATA AGG GCA ACG AAT GAC ATA AAG AGA GTT ATC AAA
1020
321 Ser Val Ser Lys Ile Ala Ser Glu Ile Arg Ala Thr Asn Asp Ile Lys Arg Val Ile Lys
340

1021 AAA TAT ACT GGA TTC GAG CTA ACA GAG GAC GAA ATT AAT TAC ATT GAG GAA GCT ATA AAC
1080
341 Lys Tyr Thr Gly Phe Glu Leu Thr Glu Asp Glu Ile Asn Tyr Ile Glu Glu Ala Ile Asn
360

1081 TTA GCA GAC GAA TAT GCG CTT CAA AAT GCA ATA GCT GAT ATT ATA AAC AAA CGC GTT GGT
1140
361 Leu Ala Asp Glu Tyr Ala Leu Gln Asn Ala Ile Ala Asp Ile Ile Asn Lys Arg Val Gly
380

1141 GTA GGT TTT GTA TCC CAC AAA CAT ACA GGA GCT CCT GTT TCA CTT CTA GCC TAC GGC CCA
1200
381 Val Gly Phe Val Ser His Lys His Thr Gly Ala Pro Val Ser Leu Leu Ala Tyr Gly Pro
400

1201 GGT GCA GAG AAT TTT GCA GGC TTT TTA CAC CAT GTA GAT ACG GCA AAG CTA ATT GCC AAG
1260
401 Gly Ala Glu Asn Phe Ala Gly Phe Leu His His Val Asp Thr Ala Lys Leu Ile Ala Lys
420

1261 CTA ATG CTC TTT GGG AAG AAA GAT ATT CCC GTT ACC ATC TTG GGA ATA AGT GGA GTT AAA
1320
421 Leu Met Leu Phe Gly Lys Lys Asp Ile Pro Val Thr Ile Leu Gly Ile Ser Gly Val Lys
440

1321 GGA GAT ATA ACC GGA GAC TTC AAA GTG GAT GAG CAA GAT GCA TAT GTG ACC TTA ATG ATG
1380
441 Gly Asp Ile Thr Gly Asp Phe Lys Val Asp Glu Gln Asp Ala Tyr Val Thr Leu Met Met
460

1381 TTG CTT GGG GAA AGG GTA GAT ACT GAA CTT GAA AGG AAA GTC GAC ATG AAT AAT AAC GGC
1440
461 Leu Leu Gly Glu Arg Val Asp Thr Glu Leu Glu Arg Lys Val Asp Met Asn Asn Asn Gly
480

1441 ATA ATC GAG TTG GGA GAC GTG CTC CTG ATT CTA CAA GAG TCC 1482
481 Ile Ile Glu Leu Gly Asp Val Leu Leu Ile Leu Gln Glu Ser 494

Pyrococcus furiosus VC1 (7ph2)
SEQ ID NO:53 and 54

1 ATG ATT AAC CAA ATA AAC TTC AAA ACC TCT CAT GGA GGA AGC AGA GAA GAA GGC TAC ATA
60
1 Met Ile Asn Gln Ile Asn Phe Lys Thr Ser His Gly Gly Ser Arg Glu Glu Gly Tyr Ile
20

61 AAC TTC TCG GCC TCT GTA AAT CCT TAT CCA CCA GAA TGG ACT GAT GAA ATG TTT GAG AGG
120
21 Asn Phe Ser Ala Ser Val Asn Pro Tyr Pro Pro Glu Trp Thr Asp Glu Met Phe Glu Arg
40

121 GCT AAA AAG ATA AGC ACC TTC TAT CCT TAC TAT GAA AAG CTT GAG GAA GAA CTC TCA GAT
180
41 Ala Lys Lys Ile Ser Thr Phe Tyr Pro Tyr Tyr Glu Lys Leu Glu Glu Glu Leu Ser Asp
60

181 CTA ATT GGG GAG CCA ATA ACT ATA ACT GCA GGA ATA ACA GAG GCA CTT TAC CTG CTT GGA
240
61 Leu Ile Gly Glu Pro Ile Thr Ile Thr Ala Gly Ile Thr Glu Ala Leu Tyr Leu Leu Gly
80

241 GTT TGG ATG AGG GGT CGG AAA GTA ATA ATC CCG AAG CAC ACC TAT GGG GAA TAC GAG AGG
300
81 Val Trp Met Arg Gly Arg Lys Val Ile Ile Pro Lys His Thr Tyr Gly Glu Tyr Glu Arg
100

301 ATC TCA CGC ATG TTC GGA GGT AGG GTG ATC AAA GGT CCC AAT GAC CCA GGA AAG TTA GCA
360
101 Ile Ser Arg Met Phe Gly Gly Arg Val Ile Lys Gly Pro Asn Asp Pro Gly Lys Leu Ala
120

361 GAA TTT GTT GAA AGA AAT TCA TTC GTG TTC TTC TGC AAT CCA AAC AAT CCA GAT GGA AAG
420
121 Glu Phe Val Glu Arg Asn Ser Phe Val Phe Phe Cys Asn Pro Asn Asn Pro Asp Gly Lys
140

421 TTC TAC CGA GAA AAA GAG ATG AAA CCT CTT TTA GAT GCC ATT CAA GAC ACT AAC TCA ATT
480
141 Phe Tyr Arg Glu Lys Glu Met Lys Pro Leu Leu Asp Ala Ile Gln Asp Thr Asn Ser Ile
160

481 TTG ATC TTG GAT GAA GCC TTC ATA GAC TTT GTT AAG AAA CCA GAA AGC CCA GAG GGA GAG
540
161 Leu Ile Leu Asp Glu Ala Phe Ile Asp Phe Val Lys Lys Pro Glu Ser Pro Glu Gly Glu
180

541 AAC ATA ATC AGG CTA AGG ACT TTT ACC AAA AGC TAC GGG CTC CCA GGG GTA AGG GTT GGA
600
181 Asn Ile Ile Arg Leu Arg Thr Phe Thr Lys Ser Tyr Gly Leu Pro Gly Val Arg Val Gly
200

601 TAT GTT ATT GGA TTT GTC GAT GCT TTC AGG AGC GTT AGA ATG CCA TGG TCA ATT GGC TCT
660
201 Tyr Val Ile Gly Phe Val Asp Ala Phe Arg Ser Val Arg Met Pro Trp Ser Ile Gly Ser
220

661 ACT GGG GTG GCC TTC TTA GAG TTC TTA CTC AAA GAT AAC TTC AAA CAC TTA AGA AAA ACC
720
221 Thr Gly Val Ala Phe Leu Glu Phe Leu Leu Lys Asp Asn Phe Lys His Leu Arg Lys Thr
240

721 CTC CCC CTA ATA TGG AAA GAA AAG GAG AGG ATT GAG AAA GAA TTG AAA GTT AAA AGC GAT
780
241 Leu Pro Leu Ile Trp Lys Glu Lys Glu Arg Ile Glu Lys Glu Leu Lys Val Lys Ser Asp
260

781 GCA AAT TTC TTC ATT ATG AAG GTC AGA GAA GGA ATA ATT GAA AAG CTA AAA GAG AAT GGC
840
261 Ala Asn Phe Phe Ile Met Lys Val Arg Glu Gly Ile Ile Glu Lys Leu Lys Glu Asn Gly
280

841 ATC CTT GTA AGG GAT TGC AAG AGC TTT GGA CTC CCT GGG TAC ATA AGG TTT TCA GTT AGA
900
281 Ile Leu Val Arg Asp Cys Lys Ser Phe Gly Leu Pro Gly Tyr Ile Arg Phe Ser Val Arg
300

901 AGG AGA GAA GAG AAT GAC AAA CTC ATA AAC ATC CTT AGA AAA ACA CTT AAT ACT 954
301 Arg Arg Glu Glu Asn Asp Lys Leu Ile Asn Ile Leu Arg Lys Thr Leu Asn Thr 318

## Claims

1. A polynucleotide selected from the group consisting of:
(a) a nucleic acid sequence encoding a polypeptide as set forth in SEQ ID NO: 28, 29, 33, 34, 36, 42, 44, 48, 50, 52 or 54;
(b) a nucleic acid sequence as set forth in SEQ ID NO: 19, 20, 24, 25, 27, 37, 39, 40, 41, 43, 47, 49, 51 or 53;
(c) the nucleic acid sequence of (a) or (b), wherein T can also be a U;
(d) a nucleic acid sequence which is at least 15, 30 or 50 consecutive bases in length of a sequence as set forth in SEQ ID NO: 19, 20, 24, 25, 27, 37, 39, 40, 41, 43, 47, 49, 51 or 53;
(e) a nucleic acid probe which is at least 15, 30 or 50 consecutive bases in length that will selectively hybridize to the nucleic acid sequence of (a) to (d);
(f) a nucleic acid sequence encoding an enzyme having alkaline phosphatase activity having at least 70%, 90%, 95% or 97% sequence identity to SEQ ID NO: 19, 20, 24, 25, 27, 37, 39, 40, 41, 43, 47, 49, 51 or 53; and
(g) a nucleic acid sequence complementary to the nucleic acid sequence of (a) to (f);
optionally including additional coding and/or non-coding sequence.

2. The polynucleotide of claim 1, wherein the polynucleotide is DNA.

3. The polynucleotide of claim 1, wherein the polynucleotide is RNA.

4. A polynucleotide comprising a member selected from the group consisting of:
(a) a polynucleotide encoding an enzyme having alkaline phosphatase activity and having at lest 70% identity to a polynucleotide encoding an enzyme encoded by the DNA contained in ATCC deposit No. 97536, wherein said enzyme is selected from the group consisting of Ammonifex degensii, Methanococcus igneus, Methanococcus thermolithotrophicus, OC9a-27A3A, Desulfurococcus M11 TL, Aquifex pyrophilus, Aquifex VF-5, Pyrolobus fumarius, OC1/4V, Archaeglobus litotrophicus, Bacillus thermoleovorans, and Pyrococcus furiosus;
(b) a polynucleotide complementary to the polynucleotide of (a); and
(c) a polynucleotide comprising at least 15 bases of the polynucleotide of (a) and (b).

5. A vector comprising the polynucleotide of any one of claims 1 to 4.

6. A host cell comprising the vector of claim 5.

7. A process for producing a polypeptide comprising: expressing from the host cell of claim 6 a polypeptide encoded by said DNA and isolating the polypeptide.

8. A process for producing a recombinant cell comprising: transforming or transfecting the cell with the vector of claim 5 such that the cell expresses the polypeptide encoded by the DNA contained in the vector.

9. An enzyme of which at least a portion of which is coded for by a polynucleotide selected from the group consisting of:
(a) an amino acid sequence as set forth in SEQ ID NO: 28, 29, 33, 34, 36, 42, 44, 48, 50, 52 or 54;
(b) an amino acid sequence encoded by the polynucleotide of claim 1 or claim 4;
(c) an amino acid sequence at least 30 consecutive amino acids in length of the sequence as set forth in SEQ ID NO: 28, 29, 33, 34, 36, 42, 44, 48, 50, 52 or 54 or a polypeptide encoded by the polynucleotide of claim 1;
(d) an amino acid sequence having an alkaline phosphatase activity and having at least 70%, 90%, 95% or 97% sequence identity to SEQ ID NO: 28, 29, 33, 34, 36, 42, 44, 48, 50, 52 or 54 or a polypeptide encoded by the polynucleotide of claim 1.

10. A method for hydrolyzing phosphate bonds comprising: administering an effective amount of an enzyme selected from the group consisting of an enzyme having the amino acid sequence selected from the group of amino acid sequences set forth in SEQ ID NO: 28, 29, 33, 34, 36, 42, 44, 48, 50, 52 or 54, an enzyme of claim 9 or an enzyme encoded by a polynucleotide of any one of claims 1 to 4.

11. A thermostable alkaline phosphatase having a sequence as set forth in claim 9 or encoded by the polynucleotide of claim 1(a), (b), (c) or (f).

12. An antibody generated against the enzyme of claim 9 or generated against a fragment of the enzyme which binds to whole native enzymes.
